# EUROPEAN PATENT SPECIFICATION

(11) **EP 2 898 074 B1**
(45) Date of publication and mention of the grant of the patent: **02.01.2019**
(21) Application number: 13792113.6
(22) Date of filing: 11.09.2013
(51) Int. Cl.: C12N 15/63

(54) **BISTABLE GENETIC TOGGLE SWITCH COMPRISING A PAIR OF RECIPROCAL REPRESSORS AND A POSITIVE FEEDBACK LOOP BASED ON DNA-BINDING PROTEINS**
BISTABILE GENETISCHE KIPPSCHALTER MIT EINEM PAAR VON REZIPROKEN REPRESSOREN UND EINER POSITIVEN RÜCKKOPPLUNGSSCHLEIFE AUF BASIS VON DNA-BINDENDEN PROTEINEN
INTERRUPTEUR À BASCULE GÉNÉTIQUE BISTABLE COMPRENANT UNE PAIRE DE RÉPRESSEURS RÉCIPROQUES ET UNE CHAÎNE DE RÉACTION POSITIVE BASÉ SUR DES PROTÉINES DE LIAISON À L'ADN

(30) Priority: 20.09.2012 SI 201200285
(43) Date of publication of application: 29.07.2015
(73) Proprietor: Kemijski Institut, 1000 Ljubljana (SI)
(72) Inventor: JERALA, Roman, 1000 Ljubljana (SI); BENCINA, Mojca, 1000 Ljubljana (SI); MAJERLE, Andreja, 1000 Ljubljana (SI); OBLAK, Alja, 1420 Trbovlje (SI); LEBAR, Tina, 1000 Ljubljana (SI); FORSTNERIC, Vida, 1000 Ljubljana (SI); LONZARIC, Jan, 2000 Maribor (SI); SMOLE, Anze, 1000 Ljubljana (SI); GABER, Rok, 1000 Ljubljana (SI); BEZELJAK, Urban, 5280 Idrija (SI); GOLOB, Anja, 4203 Duplje (SI); KADUNC, Lucija, 4260 Bled (SI); VUCKO, Dusan, 4000 Kranj (SI); STRAZAR, Martin, 6000 Koper (SI); PIRS, Bostjan, 1000 Ljubljana (SI); JERALA, Miha, 1000 Ljubljana (SI); ZUPANCIC, Uros, 8333 Semic (SI); SOMRAK, Maja, 8000 Novo Mesto (SI); LUZNIK, Zala, 2380 Slovenj Gradec (SI)
(74) Representative: Grünecker Patent- und Rechtsanwälte PartG mbB
(86) International application number: PCT/SI2013/000052
(87) International publication number: WO 2014/046626

(56) References cited:
- WO-A1-00/32748
- US-B2- 6 841 376
- KRAMER BEAT P ET AL: "An engineered epigenetic transgene switch in mammalian cells", July 2004 (2004-07), NATURE BIOTECHNOLOGY, VOL. 22, NR. 7, PAGE(S) 867-870, XP002718464, ISSN: 1087-0156 the whole document
- DATABASE MEDLINE [Online] US NATIONAL LIBRARY OF MEDICINE (NLM), BETHESDA, MD, US; 20 July 2012 (2012-07-20), CHEN SHUOBING ET AL: "Automated design of genetic toggle switches with predetermined bistability.", Database accession no. NLM23651251 & CHEN SHUOBING ET AL: "Automated design of genetic toggle switches with predetermined bistability.", ACS SYNTHETIC BIOLOGY 20 JUL 2012, vol. 1, no. 7, 20 July 2012 (2012-07-20), pages 284-290, ISSN: 2161-5063

## Description

### Field of invention

The disclosure refers to a bistable genetic toggle switch comprising a pair of mutual repressors and a positive feedback loop based on DNA-binding proteins. Each repressor and activator pair binds to its corresponding binding site on DNA. This mechanism constitutes switch functionality.

### State of the art

Genetic regulatory networks are hirearchically organised networks of genes that act on each other in order to perform a function of some kind.

Genetic arrangements of this kind can be found in nature, such as a switch in the bacteriophage lambda and a circadian oscillator in cianobacteria (2).

In the context of recent developments in the fields of biotechnology and synthetic biology, there arose a need to construct synthetic genetic regulatory networks with well-defined and complex functions.

Bistable and multistable toggle swithces are the most useful representatives, as they can function as a form of memory in biological systems as well as in the control of expression of endogenous and heterologous genes. The development started with the construction of a bistable switch using endogenous transcription factors in *Escherichia coli (3).* Kramer *et al.* constructed a bistable switch out of similiar elements in mammalian cells (4). This switch consists of two constitutive promoters and their corresponding opposing repressors. A genetic network of this kind can assume two stable states, since the activation of the promoter of the first construct activates expression of the repressor that represses the promoter of the second construct. For a practical application of such a switch, an external signal able to push the switch into the desired state is needed. This kind of change should be possible with the addition of small molecules that act as inducers, notably anhydrotetracyclin (aTc) and isopropyl-beta-D-1-thiogalactopyranoside (IPTG) (3). Each of the inducers binds to its corresponding repressor and inactivates it. For example, inducer 2 binds to repressor 2 and inactivates it. Lack of repression of promotor 2 results in expression of repressor 1, which binds to promotor 1 and blocks the transcription of repressor 2. Similiarly, when inducer 1 is added, it inactivates repressor 1 and promoter 1 activates transciption of repressor 2, which represses promoter 2 and therefore blocks the transcription of repressor 2. It is important that the system exhibits stability -a set state must persists after the removal of the inducer. Kramer *et al.* prepared a bistable switch in chinese hamster ovary (CHO) cells (4). This switch functions very similiarly to the switch constructed in *Escherichia coli.* The switch is made of the same two opposing promoters, followed by binding sites for repressors. Promoter 1 is followed by the repressor 1 binding site, whereas promoter 2 is followed by the repressor 2 binding site. Both promoters and corresponding binding sites are followed by a gene encoding a DNA-binding protein (promoter 1 followed by DNA-binding protein 2 and vice versa for promoter 2). Mammalian repressors are constructed modularly - the repressor and the DNA-binding function are exerted by separate domains of the protein. This provides the oppurtunity to use specific DNA-binding domains, such as zinc-finger domains or TAL (Transcriptional activator like) proteins and combine them with repressor domains (for instance KRAB), or with activator domains (such as VP16), which results in functional transcriptional repressors or activators, respectively. In the case of the switch designed by Kramer *et al.* (4), the DNA-binding domains A and B are fused with the repression domain KRAB which results in two repressors: A and B.

### Drawbacks of current state of the art

Development in the field continued with the design of gene regulatory networks of higher complexity consisting of several bistable switches. Networks with an odd number of repressors were constructed, exhibiting oscillatory behaviour (5,6). In addition, networks of repressors that were capable of boolean logic operations were constructed (7).

Up to now, all of the designed genetic switches were based on natural bacterial DNA-binding proteins as building blocks of repressors. The number of well characterized natural repressor proteins is limited, which limits the number of independent bistable switches able to operate inside a single cell. Several biochemical properties such as stability, oligomeric state and affinity for DNA vary between different natural repressor proteins, contrary to the the desired balance between the regulatory elements which is needed for a bistable switch to function properly and robustly.

The second drawback of bistable switches described to date is the use of only repressors, which results in relatively small differences between the two states. In the study by Kramer *et al.* (4), it was found that the state to state difference in the expression of a reporter gene under the control of a bistable switch was relatively small.

### Literature:

1. Ptashne, M. A Genetic Switch: Phage λ and Higher Organisms (Cell, Cambridge, Massachusetts, 1992).
2. Ishiura, M. et al. Expression of a gene cluster kaiABC as a circadian feedback process in cyanobacteria. Science 281, 1519-1523
3. Gardner, T. S., Cantor, C. R. & Collins, J. J. Construction of a genetic toggle switch in Escherichia coli. Nature 403, 339-42 (2000).
4. Kramer, B. P. et al. An engineered epigenetic transgene switch in mammalian cells. Nature biotechnology 22, 867-70 (2004).
5. Elowitz, M. B. & Leibler, S. A synthetic oscillatory network of transcriptional regulators. Nature 403, 335-8 (2000).
6. Fung, E. et al. A synthetic gene-metabolic oscillator. Nature 435, 118-122 (2005).
7. Tamsir, A., Tabor, J. J. & Voigt, C. a. Robust multicellular computing using genetically encoded NOR gates and chemical "wires."Nature 1-4 (2010). doi:10.1038/nature09565

### Summary of invention.

Artificially designed DNA-binding proteins, e.g. zinc-finger proteins (ZFP) and TAL effectors (US patent application: US20120110685), which can be prepared in any desired numbers, seem to be the best solution to the problem of limited numbers of natural repressor proteins. However, computer simulations and laboratory experiments have shown that a bistable switch based on synthetic DNA-binding proteins is difficult to implement. Cooperativity of repressors is a prerequisite to for the preparation of a functional bistable toggle switch (US patent US6841376) (Figure 2). Synthetic modular DNA-binding proteins (e.g. TAL effectors, ZFP) bind DNA as monomers and therefore lack binding cooperativity.

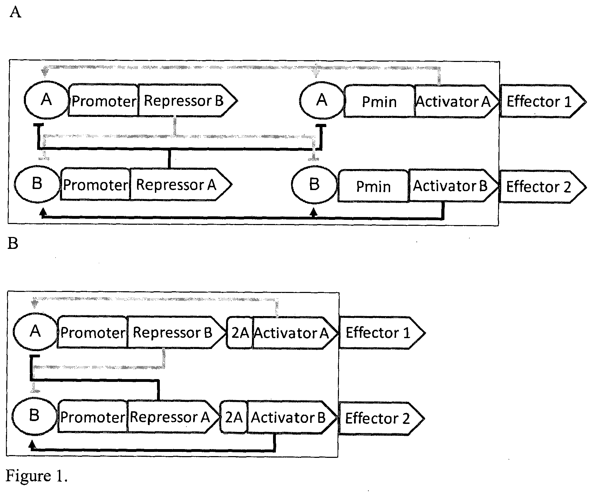

To solve this problem we propose a designed bistable toggle switch composed of two mutual repressors based on DNA-binding protein domains, and an additional positive feedback loop based on the same DNA-binding protein domains fused with an activator domain (Figure 1). This switch is significantly more stable than a switch based on synthetic DNA repressor proteins without an integrated positive feedback loop. Moreover, the difference in reporter expression in both toggle states of this switch is far greater than in the case of a switch without a positive feedback loopand absolute repression is not necessary for bistability.

Figure 1 represents a scheme of the positive feedback loop switch design. Transcription of both the repressors and the activators is controlled by a minimal promoter that ensures very low expression of the downstream gene. The expression of the downstream gene is greatly increased if a DNA-binding protein fused with an activatory domain binds upstream of the minimal promoter. The switch has binding sites for the appropriate artificial transcription factors, which are composed of designed DNA-binding proteins and located upstream of promoters. The binding sites for the artificial DNA-binding proteins A and B are located upstram of the promoters of the first or of the second group of genes, respectively.

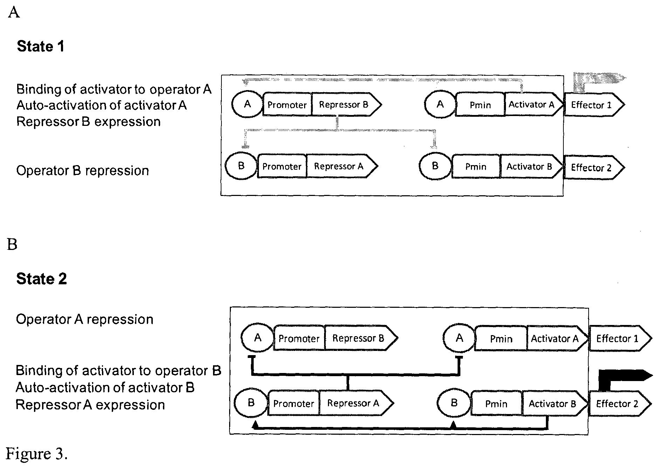

Each state requires transcription of two genes, therefore the switch is composed of four genes. An optional number of genes (effectors or reporters) expressed in either of the two toggle states can be added. (Figure 3).

The following are active in state 1:
- repressor B, which is under the control of operator A located upstream of either a minimal or a constitutive promoter. Repressor B represses the expression of all genes characteristic of state 2 as well as the effectors or the reporters of state 2;
- activator A, which is under the control of operator A. Activator A activates the expression of all genes characteristic of state 1, i.e. the repressor B and the activator A as well as the effectors of state 1. The activator A's operon is auto-activatory and represents a positive feedback loop that can be inhibited by repressor A.

The following are active in state 2:
- repressor A, which is under the control of operator B located upstream of either a minimal or a constitutive promoter. Repressor A represses the expression of all genes characteristic of state 1 as well as the effectors or the reporters of state 1;
- activator B, which is under the control of operator B. Activator B activates the expression of all genes characteristic of state 2, i.e. the repressor A and the activator B as well as the effectors of state 2. The activator B's operon is auto-activatory and represents a positive feedback loop that can be inhibited by repressor B.

The activator consists of a DNA-binding domain that is fused to an activation domain, such as the VP16 or the VP64 domain. The repressor consists of a DNA-binding domain that is fused to a transcriptional repression domain, such as the KRAB domain.

Because the repressor B, activator A and the state 1 effector are under the joint transcriptional control of operator A, we can combine them under the same operator and promoter in single or multiple DNA sequences linked with 2A sequences between structural genes, which enable co-translational cleavage of proteins, e.g. the t2A sequence.

We can combine repressor A, activator B and the state 2 effectors under the joint control of a single operator and promoter in the same manner.

### Figure descriptions

Figure 1: A scheme of a mutual repressor switch with a positive feedback loop based on DNA-binding proteins. A) A multiple-operon implementation allowing toggle control with the same operator for each state. B) A single-operon implementation allowing toggle control with the same operator for each state.
Figure 2: A scheme of a classic bistable toggle switch, as implemented in bacterial and mammalian cells, based on bacterial cooperative DNA-binding domains (Gardner et al. 2000, Kramer et al. 2004).
Figure 3: A scheme of a bistable genetic toggle switch comprising a pair of mutual repressors and a positive feedback loop, based on DNA-binding proteins. A) Activator A is expressed in state 1 and binds to DNA binding element A, activating transcription of the structural genes of repressor B, activator A (itself) and effector 1. The expression of repressor B inhibits the expression of repressor A and of activator B. B) Activator B is expressed in state 2 and it binds to DNA binding element B, activating the transcription of the structural genes of repressor A, activator B (itself) and effector 2. The expression of repressor A inhibits the expression of repressor B and of activator A.
Figure 4: Implementation of a bistable toggle switch with a pair of mutual repressors and a positive feedback loop, based on TAL DNA-binding proteins, applicable for expression in mammalian cells. Legend: the arrows depict the structural genes coding for repressor (TAL:KRAB), activator (TAL:VP16), effectors (fluorescent proteins) and inducer proteins; 2A represents the position of the self-cleaving peptide; a round symbol represents the position of an operator with DNA binding elements, which along with the promoter controls the expression of structural genes.
Figure 5: Implementation of a bistable toggle switch with a pair of mutual repressors and a positive feedback loop, based on TAL DNA-binding proteins, with joint operons for expression in mammalian cells. Legend: the arrows represent structural genes coding for repressor (TAL:KRAB), activator (TAL:VP16), effectors (fluorescent proteins) and inducer proteins; 2A represents the position of the self-cleaving peptide; a round symbol represents the position of the operator with the DNA binding elements, which along with the promoter controls the expression of structural genes.
Figure 6: Detection of bistability in mammalian cells, which have been transfected with plasmids encoding the neccessary genes for the switch.
Figure 7: Detection of the TAL-repressor efficiency.
Figure 8: Detection of the effect of the DNA binding elements in the operon's operator.
Figure 9: Detection of TAL activator efficiency.

### Description of the invention

The present disclosure relates to a bistable switch comprising a pair of mutual repressors and a positive feedback loop based on modular DNA-binding proteins. The bistable switch regulates switching from stable state 1 to stable state 2 and vice-versa. The switching is directed by signals from the environment. The positive feedback loop brings nonlinearity to the system, enabling the switch to function with non-oligomeric and non-cooperative DNA-binding domains.
The bistable switch includes:
a) An operon for maintaining state 1 with a positive feedback loop, which includes the structural gene for activator A, which forms the positive feedback loop. Transcription of the structural gene for activator A is under regulation of an operator, which includes the DNA binding element A and a promoter.
b) An operon for maintaining state 1 with repressor B which inhibits state 2 by repressing the genes under regulation of operator B. Transcription of the structural gene for repressor B is under regulation of an operator, which includes the DNA binding element A and a promoter.
c) An operon representing the activation of state 1, which includes the gene for effector 1. Effector 1 can be any gene or a group of genes e.g. different enzymes, fluorescent proteins, signaling proteins etc. Transcription of the structural gene for effector 1 is under regulation of an operator, which includes the DNA binding element A and a promoter.
d) An operon for maintaining state 2 with a positive feedback loop, which includes the structural gene for activator B, which forms the positive feedback loop. Transcription of the structural gene for activator B is under regulation of an operator, which includes the DNA binding element B and a promoter.
e) An operon for maintaining state 2 with repressor A which inhibits state 1 by repressing the genes under regulation of operator A. Transcription of the structural gene for repressor A is under regulation of an operator, which includes the DNA binding element B and a promoter.
f) An operon representing the activation of state 2, which includes the gene for effector 2. Effector 2 can be any gene or a group of genes e.g. different enzymes, fluorescent proteins, signaling proteins etc. Transcription of the structural gene for effector 2 is under regulation of an operator, which includes the DNA binding element B and a promoter.

State 1 or state 2 are activated with the following switch-ON operons:
a) An operon for switching to state 1, which includes a structural gene for activator A to activate state 1, and a structural gene for repressor B which inhibits state 2. The transcription of these structural genes is under regulation of a promoter and an operator, which is active in the presence of inducer A. The promoter can be constitutive or minimal.
b) An operon for switching to state 2, which includes a structural gene for activator B to activate state 2, and a structural gene for repressor A to inhibit state 2. The transcription of these structural genes is under regulation of a promoter and an operator, which is active in the presence of inducer B. The promoter can be constitutive or minimal.

Switching operons are activated by inducers:
a) Operons of the inducers, which include structural genes for inducer proteins under regulation of a promoter, preferably constitutive or minimal, and optionally an operator.

Operons of the structural genes, which are under regulation of the same operator (activator A, repressor B and effector 1 or activator B, repressor A and effector 2) can be joined in groups of two or more so that they are under regulation of the same operator and are linked with self-cleaving 2A peptides. They are transcribed from structural genes in the form of a single RNA and are translated into polypeptide chains, leading to synthesis of individual proteins in an equal stoichiometric ratio.

The number of DNA binding elements for DNA-binding domains of the activator or repressor ranges from one to several, preferably from one to 20, more preferably from one to 12. The position of the DNA binding elements for transcription factors in regard to the promoter can be upstream or downstream of the promoter, or both. In a preferred embodiment, the DNA binding elements are upstream of the promoter, which is adjacent to transcription initialization site and the structural gene.

The operons composing the bistable switch include a structural gene, which encodes several proteins adjacently linked with a self-cleaving 2A peptide. Optionally, the above-mentioned polycistronic operons may be divided into seperate operons encoding a single protein or two proteins liked via a self-cleaving 2A peptide with the same operators upstream of the structural gene.

### Definitions:

The term »DNA-binding domain« refers to DNA-binding domains of protein families such as TAL effectors, zinc fingers and other transcriptional regulators, their homologues, orthologues and mutants with preserved or enhanced basic functions of DNA-binding proteins.

The term »TAL« refers to synthetic or natural TAL proteins, preferably their central DNA-binding domain with an additional nuclear localization signal. The central domain of a TAL protein is composed of a variable number of TAL repeats. The term »TAL« may also refer to homologues, orthologues and mutants with preserved or enhanced basic function of TAL proteins. The term »TAL« may refer to synthetic TAL domains with any number of TAL repeats in any order, additionally containing a nuclear localization signal.

The term »operator« refers to a DNA sequence containing DNA binding elements located near a promoter. An operator can be located upstream or downstream of a promoter, preferably upstream. For the purposes of the present invention, the operator can contain one or more sequential, either identical or different DNA binding elements. Repeats of different DNA binding elements can either alternate or cluster. The number of DNA binding elements is not limited. The term »DNA binding element« refers to a specific nucleotide sequence on a DNA molecule, which binds to the DNA-binding domain. The nucleotide sequence of the DNA binding element depends on the DNA-binding domain specificity of repressors and activators of the switch. DNA binding elements composing an operator may be separated with a variable number of nucleotides. The number of nucleotides separating the DNA binding elements is between 2 and 100, preferably between 2 and 50.

The term »transcription repression domain« refers to a protein domain, which ensures the inhibition of structural gene transcription, if linked to a DNA-binding domain. The function of a transcription repressor domain, linked to a DNA-binding domain is the inhibition of structural gene transcription by preventing the binding of RNA polymerase to the corresponding promoter. Transcription repressor domains can be chosen from a range of repressors known to persons skilled in the art, preferably from the family of KRAB repressors. The term »KRAB« refers to »Krüppel-associated box« and may also refer to homologues, orthologues and mutants with preserved or enhanced basic function of inhibiting structural gene transcription.

The term »transcription activation domain« refers to a protein domain, which ensures activation of structural gene transcription, if linked to a DNA-binding domain. The function of a transcription activation domain linked to a DNA-binding domain is transcriptional activation of structural genes. Transcription activation domains can be chosen from a range of activators known to persons skilled in the art, preferably from the family of VP16 and VP64 activators. The term »VP16« refers to a transcription activation domain of viral origin. VP16 induces formation of a protein complex, which enhances expression of structural genes. The term »VP64« refers to four tandem repeats of the activation region of the VP16 domain.

The term »repressor« refers to proteins, comprising a DNA-binding domain and a transcription repression domain, preferably KRAB. The term »repressor« refers to proteins with the function of inhibiting structural gene transcription when bound to their respective DNA binding elements.

The term »activator« refers to proteins, comprising a DNA-binding domain and a transcription activation domain, preferably VP16 or VP64. The term »activator« refers to proteins with the function of activating structural gene transcription when bound to their corresponding DNA binding elements (Garg et al. 2012).

The DNA-binding domain and the transcription repression/activation domain are linked together by a linker peptide, which is any polypeptide of any length and any aminoacid sequence. The term »linker peptide« refers to aminoacid sequences with the function of separating individual domains of a chimeric protein. Optional functions of a linker peptide in a chimeric protein can also be cleavage or posttranslational modification site introduction.

The ratio of the DNA-binding domain and the transcriptional repression/activation domain can be 1:1 or 1:2. One or more transcriptional repression/activation domains can be linked to the DNA-binding domain at the N or C terminal end or at the N and C terminal end.

The term »minimal promoter« refers to a DNA sequence of a few nucleotides in length located upstream of a transcription initiation site and is a minimal requirement for the binding of transcription factors and gene transcription. Nucleotide sequences of minimal promoters are known to persons skilled in the art and have been extensively described elsewhere.

The term »constituitive promoter« refers to a DNA sequence, which ensures continuous transcription of structural genes. Its location and sequence is known to persons skilled in the art and has been previously described elsewhere. The term »constituitive promoter« refers to an unregulated promoter, enabling continuous expression of the corresponding gene.

The term »inducer« refers to molecules, able to regulate gene expression by binding to proteins, e.g. repressors or activators. The term »inducer« refers to antibiotics and their analogues, natural compounds and their analogues, metalothionines, steroids and analogues; e.g. tetracyclin, doxycyclin, anhidrotetracyclin, rapamycin and analogues, ecdysone and analogues (e.g. ponasteronA, muristeronA), alolactose (lac operon), arabinose (ara operon), cumermycin and novobiocin, RU486 (mifepriston), estrogens and analogues (e.g. 4-hydroxitamoxifene), streptogramines (e.g. pristinamycin), macrolides (e.g. erythromycin), vanilinic acid, cumate, phloretin, biotin, arginine, metal ions, polymeric substrates (e.g. pectin, xylan, arabinan) or monomeric units of degraded polymers (e.g. arabinose, xylose, metals and metal ion-protein complexes, cAMP, cyanate, CRP, formate, maltose, acetolactate, urea) and other compounds known to persons skilled in the art. The term »inducer« may also refer to temperature, pH or redox potential, if the change in temperature, pH or redox potential effects the activity of repressors or activators.

The term »self-cleaving peptide« refers to aminoacid peptide sequences, that ensure autocatalytic cleavage of the peptide, such as 2A sequences (e.g. t2A, e2A, f2A etc). Self-cleaving peptides enable synthesis of a polycistronic mRNA chain, from which individual proteins are synthesised. The polycistronic chain can contain two or more sequentially linked proteins, separated by the self-cleavable peptide. The described composition ensures synthesis of individual proteins in equal stoichiometric ratio.

The term »signal sequence« or »signal peptide« refers to an aminoacid sequence, important for directing the protein to a certain location in the cell. Signal sequences differ depending on the host organism for protein expression. Aminoacid sequences and functions of signal peptides are known to persons skilled in the art and are available in the literature.

The term »tag peptide« refers to aminoacid sequences, added to a protein for simplified purification, isolation or detection.

The position of signal sequences, linker peptides and tag peptides can be arbitrary, although they should allow functional expression of the protein, while also preserving the function for which these sequences were selected.

The terms »homologue« and »orthologue« refer to polypeptides, originating from the same or different organism. The term »homologous« also refers to mutated protein segments, where the mutations have a minimal effect on the structure or function of the polypeptide. The term »mutant« refers to a polypeptide, differing from the native protein polypetide in at least one aminoacid.

The term »effector« refers to any protein.

The terms »promoter«, »teminator«, »protein«, »DNA« are generally known to persons skilled in the art and are used as expected.

Embodiments can contain one or more switches. The switches can function independently or can be interconnected.

An embodiment of the switch enables controlled expression of state 1 or state 2, maintains a stable state even when the inducer molecule is removed, and is capable of switching between the two states, depending on the presence of a corresponding inducer. States are defined by expression of one or more effectors, e.g. therapeutic molecules, signal molecules, regulators or any other protein molecules.

The switch can be used for state signalization as a reporter system, e.g. reporting the presence of an inducer. Such inducers might include but are not limited to metal ions, pH, glucose and others.

The term »expression vector« refers to circular or linear DNA plasmids or viral DNA, containing operons and the necessary elements for expression in prokaryotic or eukaryotic cells, which are known to persons skilled in the art. Bacterial vectors contain bacterial control elements, a bacterial replication origin and an antibiotic resistance operon for selection of successfully transformed bacteria. Eukaryotic vectors contain, in addition to a bacterial replication origin, appropriate eukaryotic control elements, and appropriate antibiotic resistance operons for selection of successful bacterial transformation and/or successful eukaryotic transfection.

Embodiements of the invention can be used in prokaryotic as well as in eukaryotic organisms and cell lines. The basic difference is the use of transcription and translation ensuring nucleotide sequences in promoters and terminators, known to persons skilled in the art.

The disclosure further includes host cells and organisms, which either transiently or stably incorporate the nucleic acids described herein. The appropriate host cells are known to persons skilled in the art and include bacterial and eukaryotic cells. One skilled in the art will appreciate that a protein can be expressed in mammalians cells of the following origins: human, rodent, bovine, pork, poultry, rabbit and similar. Mammalian host cells include cultivated primary cell lines or immortalized cell lines.

Transfer of DNA into host cells is performed with conventional methods well known to persons skilled in the arts, such as transformation or transfection, including: chemical transfer, electroporation, microinjection, DNA lipofection, cell sonication, gene bombarding, viral DNA transfer etc.

DNA transfer can be either transient or stable. »Transient transfer« refers to transfer of DNA in a vector, that does not undergo cromosomal insertion. »Stable transfer« refers to insertion of DNA into the host genome. DNA transfer to a cell line with a previous stable insertion can be controlled with the presence of markers. »Markers« refer to antibiotic or chemical resistance and can be included in the vector.

Examples of implementations described in detail below are conceived to best describe the invention.

### Exemplification

### Example 1. Preparation of DNA constructs for the switch according to the disclosure

For the preparation of DNA constructs the inventors used methods of molecular biology, such as: chemical transformation of competent *E. coli* cells, DNA plasmid isolation, polymerase chain reaction (PCR), reverse transcription - PCR, PCR ligation, determination of nucleic acid concentration, agarose gel electrophoresis of DNA, isolation of DNA fragments from agarose gels, chemical synthesis of DNA, DNA digestion with restriction enzymes, digestion of plasmid vectors, ligation of DNA fragments, purification of plasmid DNA in larger quantities. The protocols of the experimental techniques and methods are well known to person skilled in the art and are described in the manuals of molecular biology.

All work was performed with sterile techniques, which are well known to persons skilled in the art. All plasmids, completed constructs and partial constructs were transformed into bacteria *E. coli* with chemical transformation. Plasmids and constructs were transfected into cell lines HEK293 and HEK293T using comercially avaliable transfection reagents.

The final gene constructs comprising the operons for the switch as described in the present invention are listed in Table 1 and the proteins transcribed from structural genes are listed in Table 2. All operons have been prepared using techniques and methods known in the art. Operons were inserted into appropriate plasmids suitable for eukaryotic systems. The inventors confirmed adequacy of nucleotide sequences by sequencing and restriction analysis.

The label [A] represents the sequence of the transcription factor-binding DNA element that is the recognition sequence of the DNA-binding protein TALA, which is a TAL effector designed to recognize the chosen DNA sequence. The DNA sequence that is the recognition sequence of the Tt represorrepressor is labeled [TRE] and the DNA sequence that is the recognition sequence of ecdysone is labeled [ECD].

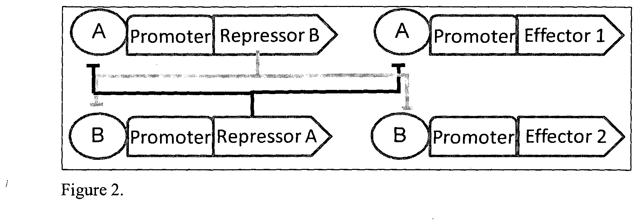

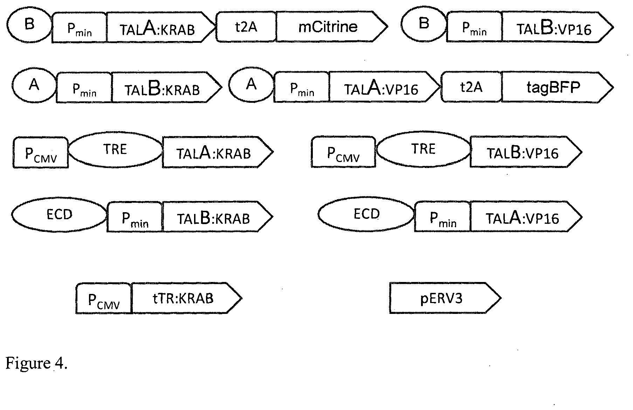

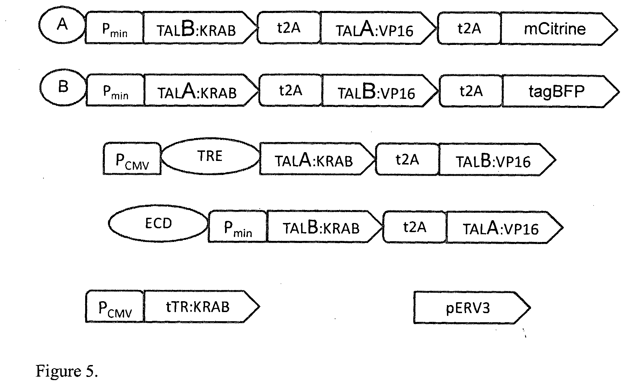

The operon for the maintenance of stable state 1 is comprised of the DNA binding element [A] for the repressor TALA:KRAB or the activator TALA:VP16, a minimal promoter and structural genes of a feedback loop, which in turn is comprised of a repressor for the inhibition of expression of structural genes from the operon formaintenance of stable state 2, specifically it comprises TALB:KRAB. Separately, the operon for the maintenance of stable state 1 comprises also a DNA-binding site [A] for the repressor TALA:KRAB or the activator TALA:VP16, a minimal promoter and structural genes of a positive feedback loop to maintain state 1, specifically TALA:VP16, and any effectors (Figure 1, Figure 3, Figure 4, Figure 5). Both operons can be combined into a single operon in which structural genes are separated by a self-cleaving peptide 2A (Figure 2, Figure 3, Figure 4, Figure 5). The operon for the maintenance of stable state 2 is comprised of the DNA binding site [B] for the repressor TALB:KRAB or the activator TALB:VP16, a minimal promoter and structural genes of a feedback loop, which in turn is comprised of a repressor for the inhibition of expression of structural genes from the operon formaintenance of stable state 1, specifically it comprises TALA:KRAB. Separately, the operon for the maintenance of stable state 2 comprises also a DNA-binding site [B] for the repressor TALB:KRAB or the activator TALB:VP16, a minimal promoter and structural genes of a positive feedback loop to maintain state 2, specifically TALB:VP16, and any effectors (Figure 1, Figure 3, Figure 4, Figure 5). Both operons can be combined into a single operon in which structural genes are separated by a self-cleaving peptide 2A (Figure 1, Figure 3, Figure 4, Figure 5).

The operator contains 10 DNA binding elements for either the DNA-binding protein TALA or TALB.

The operons for switching into stable state 1 are comprised of a constitutive or minimal promoter, DNA binding elements for an inducer- dependent activator or repressor, and structural genes encoding TALA:VP16 for state 1 activation and (on a separate operon) TALB:KRAB for state 2 inhibition. Operons for switching into state 1 can be combined into a single operon, which contains the DNA-binding elements for an inducer-dependent activator or repressor, minimal or constitutive promoter and structural genes separated by a self-cleaving peptide 2A.

The operons for switching into stable state 2 are comprised of a constitutive or minimal promoter, DNA binding elements for an inducer-dependent activator or repressor, and structural genes encoding TALB:VP16 for state 2 activation and (on a separate operon) TALA:KRAB for state 1 inhibition. Operons for switching into state 2 can be combined into a single operon, which contains the DNA-binding elements for an inducer-dependent activator or repressor, minimal or constitutive promoter and structural genes separated by a self-cleaving peptide 2A.

Inducers for state 1 or state 2 induction are different. Inducers are well known to persons skilled in the art and described in detail in the state of the art. Inducers can be arbitrarily selected, provided they specifically activate one of the two/several states.

The TAL DNA-binding domain was obtained from TAL effectors by PCR amplification of the central DNA-binding domain to which a signal sequence for nuclear localization was added. Appropriate DNA binding elements were prepared synthetically so that they match the DNA-binding domains of selected TAL proteins. It is evident from the state of the art that DNA-binding domains based either on TAL or zinc fingers are naturally or synthetically prepared and each have their own recognition binding sites on DNA; the DNA binding elements. The number of DNA binding elements can be varied. It is evident from the state of the art that the effect of a repressor or an activator is improved by increasing the number of its DNA binding elements up to 12. We selected VP16 as the activation domain and KRAB as the repression domain. Both domains alike were fused with a TAL DNA-binding domain.

**Table 1: List of operons, DNA-binding elements and proteins.**

| operon structure | SEQ ID NO. |
|---|---|
| 10x[B]-pₘᵢₙ-TALB:VP16 | 1 |
| 10x[B]-pₘᵢₙ-TALA:KRAB-T2A-mCit | 2 |
| 10x[A]-pₘᵢₙ-TALB:KRAB-T2A-TALA:VP16-T2A-BFP | 3 |
| 10x[A]-pₘᵢₙ-TALB:KRAB | 4 |
| 10x[A]-pₘᵢₙ-TALA:VP16-T2A-BFP | 5 |
| 10x[B]-pₘᵢₙ-TALA:KRAB-T2A-TALB:VP16-T2A-mCit | 6 |
| p_{CMV}-[TRE]-TALB:VP16 | 7 |
| p_{CMV}-[TRE]-TALA:KRAB | 8 |
| [ECD]-pₘᵢₙ-TALB:KRAB | 9 |
| [ECD]-pₘᵢₙ-TALA:VP16 | 10 |
| p_{CMV}-tTR-KRAB | |
| p_{CMV}-ECD-KRAB | |
| [B] | 19 |
| [A] | 20 |
| pCMV-tTR:KRAB | 21 |
| pERV3 | 22 |

| protein | SEQ ID NO. |
|---|---|
| TALA:VP16 | 11 |
| TALB:VP16 | 12 |
| TALA:KRAB | 13 |
| TALB:KRAB | 14 |
| mCit | 15 |
| BFP | 16 |
| TALB:KRAB-T2A-TALA:VP16-T2A-BFP | 17 |
| TALA:KRAB-T2A-TALB:VP16-T2A-mCit | 18 |

### Example 2. Bistable switch in mammalian cells

The methods and techniques of culturing cell cultures are well known to persons skilled in the art, therefore they are only briefly described with the intention of illustrating the implementation example. Cell lines of HEK293 and HEK293T cells were cultured at 37 °C and 5% CO₂. DMEM medium supplemented with 10% FBS, which contains all the necessary nutrients and growth factors was used for cell culturing. Once the cell culture reached an appropriate density, cells were subcultured into a new culture vessel and/or diluted. For the application of cells in experiments the number of cells was determined with a hemocytometer and seeded at 2,5×10⁴ cells per well on a 12 well microtiter plate 24 hours prior to transfection. Seeded plates were incubated at 37 °C and 5% CO₂, until cells were 50-70% confluent for transfection with JetPei transfection reagent (Polyplus transfection). Transfection was performed according to the manufacturer's protocol, modified to a 12 well microtiter plate.

The HEK293 and HEK293T cell lines were transfected with plasmids described in the present disclosure and listed in Table 1.

For determination of effector expression and the system's state the cell medium was changed 2 hours post transfection and again 2 days post transfection, after which cells were incubated an additional 2 days. At the first medium change, an inducer for state 1 or state 2, tetracycline or ecdysone, was added. The expression of the effectors, blue fluorescent protein and yellow fluorescent protein, was monitored on a flow cytometer. A laser with a wavelength of 405 nm (blue fluorescent protein - BFP) and 488 nm (yellow fluorescent protein - mCit) was used. Emission was measured in the 430-480 nm (BFP) and 520-550 nm (mCit) range.

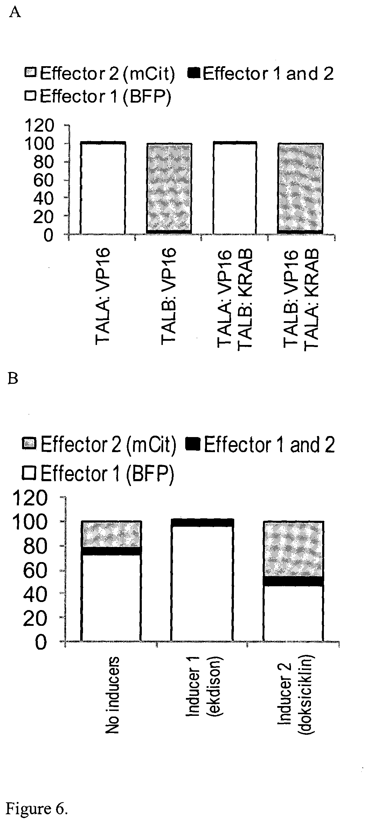

Results shown in figure 6A and 6B demonstrate that the switches function as described in the present disclosure Results shown in figure 6A prove the switch enters state 1 or state 2 accordingly in the presence of plasmids p_{CMV}-TALA:VP16 (column 1), p_{CMV}-TALB:VP16 (column 2), p_{CMV}-TALA:VP16 and p_{CMV}-TALB:KRAB (column 3), p_{CMV}-TALB:VP16 and p_{CMV}-TALA:KRAB (column 4). All cells were, in addition to the plasmids listed earlier, also transfected with plasmids SEQ ID NO. 1, 2, 4, 5 of the switch as described in the present disclosure.

Results shown in figure 6B prove the switch enters state 1 or state 2 accordingly with the addition of inducers for state 1 or 2 and switching of the switch. All cell were in addition to the plasmids listed earlier also transfected with plasmids SEQ ID NO. 1,2,4,5,7,8,9,10 of the switch by the disclosure. Without the addition of an inducer the cells enter either state 1 or state 2. Inducer 1 causes the cells to enter state 1 exclusively, inducer 2 causes the cells to enter state 2 exclusively.

### Example 3. TAL-repressors

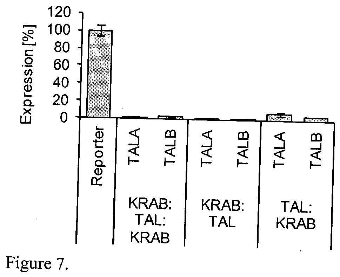

A repressor was prepared by fusing a TAL DNA-binding domain and a KRAB repressor domain, where the KRAB repressor domain was fused to the C- and N-terminal end of the TAL DNA-binding domain. Two different DNA-binding domains were used: TALA and TALB. Operons with operators containing DNA binding elements specific for either TALA or TALB DNA-binding domain were prepared (labeled [A] and [B] respectively). The operator was followed by a CMV constitutive promoter and an effector, which is a luciferase gene in this example. HEK293 cells, whose culturing and transfection are described in implementation example 2, were transfected with repressors TAL:KRAB, KRAB:TAL or KRAB:TAL:KRAB and reporter, either p_{CMV}-[A]-effector (luciferase gene) or p_{CMV}-[B]-effector (luciferase gene). After 72 hours of culturing luciferase expression was assayed. Reduced luciferase activity is a direct proof of TAL-repressor function (figure 7). Results showed that the TAL-repressor function is independent of the KRAB repressor domain position, since the effector (luciferase) expression is sufficiently repressed.

### Example 4. DNA binding elements for TAL DNA-binding domains

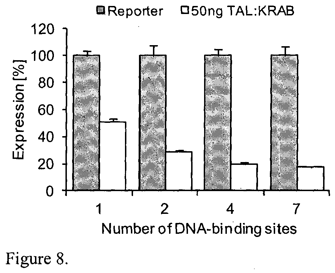

We prepared operons with an effector (luciferase) whose operons contained one, two, four or seven DNA binding elements 1x[D], 2x[D], 4x[D], 7x[D] for the TALD DNA-binding domain fused to a KRAB repressor domain. The operons also contained a CMV constitutive promoter, placed between the operator and structural genes. HEK293 cells, whose culturing and transfection are described in implementation example 2, were transfected with the TALD:KRAB repressor and a reporter, either p_{CMV}-1x[D]-effector (luciferase gene), p_{CMV}-2x[D]-effector (luciferase gene), p_{CMV}-4x[D]-effector (luciferase gene) or p_{CMV}-7x[D]-effector (luciferase gene). After 72 hours of culturing luciferase expression was assayed. Reduced luciferase activity is a direct proof of the TAL-repressor function (figure 8) on reporters with a different number of DNA-binding elements. Results showed that the function of a TAL-repressor is effective and dependent on the number of DNA-binding elements in the operons operator.

### Example 5. TAL-activators

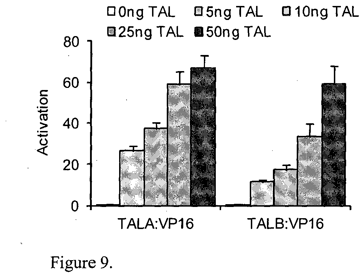

Activators were prepared by fusing a TAL DNA-binding domain and a VP16 activation domain, where the VP16 activation domain was fused to the C-terminal end of a TAL DNA-binding domain. Here we used two different DNA-binding domains TALA and TALB. We prepared operons with operators, which contained DNA binding elements specific for either TALA or TALB DNA-binding domain (labeled [A] and [B] respectively). The operator was followed by CMV or minimal constitutive promoter and an effector, which is a luciferase gene in this example. HEK293 cells, whose culturing and transfection are described in implementation example 2, were transfected with a TAL:VP16 activator (different amounts) and a reporter, either p_{CMV}-[A]-effector (luciferase gene) or p_{CMV}-[B]-effector (luciferase gene). After 72 hours of culturing luciferase expression was assayed. Luciferase activity is a direct proof of the TAL-activator function, which depends on the dose of the activator (figure 9).

## Claims

1. A bistable genetic toggle switch comprising a pair of mutual repressor genes A and B, and a pair of activator genes A and B forming a positive feedback loop based on modular DNA-binding proteins, wherein
(i) the Activator gene A and the Repressor gene B form part of separate expression cassettes each being under the joint control of the same Operator A, wherein the gene coding for Repressor B is activated by Activator A targeting said Operator A, and
(ii) the Activator gene B and Repressor gene A form part of separate expression cassettes each being under the joint control of the same Operator B, wherein the gene coding for Repressor A is activated by Activator B targeting said Operator B;
and wherein the expression of Activator gene B and Repressor gene A is inhibited by Repressor B targeting Operator B, and the expression of Activator gene A and Repressor gene B is inhibited by Repressor A targeting Operator A.

2. The bistable genetic toggle switch according to claim 1, wherein the repressors comprise a DNA-binding domain and a repression domain, and wherein the activators comprise a DNA-binding domain and an activation domain.

3. The bistable genetic toggle switch according to claim 2, wherein the repression domain is the KRAB domain and the activation domain is either the VP16 or the VP64 domain.

4. The bistable genetic toggle switch according to any one of claims 1 to 3, wherein the DNA-binding domain binds DNA as a monomer.

5. The bistable genetic toggle switch according to any one of claims 1 to 4, which can occupy stable states 1 or 2, wherein said states 1 or 2 refer to the two different states of the bistable switch **characterized by** the expression of effector 1 in state 1 or expression of effector 2 in state 2, and which comprises:
a) an operon exhibiting an activation of state 1 with an effector gene 1 that can be any gene or a group of genes e.g. different enzymes, fluorescent proteins, signaling proteins, wherein the operon comprises the activator gene A, the operator A, and the effector gene 1, and wherein transcription of this effector gene 1 is regulated by operator A that comprises the DNA binding element A located near a promoter, and
b) an operon exhibiting an activation of state 2 with an effector gene 2 that can be any gene or a group of genes e.g. different enzymes, fluorescent proteins, signaling proteins etc., wherein the operon comprises the activator gene B, the operator B, and the effector gene 2, and wherein transcription of this effector gene 2 is regulated by operator B that comprises the DNA binding element B located near a promoter,
wherein operator A comprises a DNA-binding element that represents a selected recognition site for the DNA-binding domain that is included in the activator A and the repressor A, whereas operator B comprises a DNA-binding element that represents a selected recognition site for the DNA-binding domain that is included in the activator B and the repressor B.

6. The bistable genetic toggle switch according to any one of claims 1 to 5, wherein two structural genes are regulated by the same operator A or B: activator A, repressor B, effector 1, and activator B, repressor A, effector 2, respectively; joined together so that they are regulated by the same operator A or B, and wherein activator A, repressor B, effector 1, and activator B, repressor A, effector 2, respectively, are linked together by a self-cleaving 2A peptide, wherein the sequences coding for activator A, repressor B, effector 1, and the sequences coding for activator B, repressor A, and effector 2, respectively, are each transcribed from the respective structural gene as a single RNA molecule and are translated into two separate polypeptide chains that each auto-catalytically cleave at the positions of the self-cleaving peptides, leading to synthesis of separate individual proteins of activator A, repressor B, effector 1, and activator B, repressor A, effector 2, respectively, in an equal stoichiometric ratio.

7. The bistable genetic toggle switch according to any one of claims 1 to 6, wherein
a) transcription of the structural gene coding for the activator A for activation of state 1 and the structural gene coding for the repressor B for inhibition of state 2 on separate operons is under control of the same operator A, which activates in the presence of inducer A, and a promoter, which can be either constitutive or minimal, and
b) transcription of the structural gene coding for the activator B for activation of state 2 and the structural gene for the repressor A for inhibition of state 1 on separate operons is under control of the same operator B, which activates in the presence if inducer B, and a promoter, which can be either constitutive or minimal.

8. The bistable genetic toggle switch according to any one of claims 5-7, wherein the operator A or B of the operon includes any number of equal or different type of DNA binding elements, which can be joined into clusters or distributed in alternating order, the operator preferably includes 1 to 20 DNA-binding elements, which are separated with nucleotide sequence of any length, and where operator A of operons for expression of state 1 **characterized by** the expression of effector 1 differ from operator B of operons for expression of state 2 **characterized by** the expression of effector 2, and differ from operator A of operons for switching to state 1 **characterized by** the expression of effector 1 and operator B of operons for switching to state 2 **characterized by** the expression of effector 2, and
a) DNA binding elements in operator A are designed to bind the DNA-binding domain of repressor A and activator A,
b) DNA binding elements in operator B are designed to bind the DNA-binding domain of repressor B and activator B.

9. The bistable genetic toggle switch according to any one of claims 1 to 8, which can be used for controlled synthesis of effector 1 or effector 2, depending on the state of the switch and the presence of inducers for activation of a specific state, wherein the effector 1 or 2 can be any type of protein or a group of proteins.

10. The bistable genetic toggle switch according to any one of claims 1 to 9, which can be used as indicator which through synthesis of effector 1 or effector 2 reports the presence of inducer for activation of a specific state where the effectors can be any type of protein or a group of proteins.

11. The bistable genetic toggle switch according to any one of claims 1 to 10, comprising DNA with the sequence of any one of SEQ ID NOs: 1, 2, 4, 5, 7, 8, 9, and 10.

12. Prokaryotic or eukaryotic host cells that contain a bistable genetic toggle switch according to any one of claims 1 to 11.

13. A method for effector gene expression, that comprises (a) host cells according to claim 12 containing a bistable genetic toggle switch according to any one of claims 1 to 11, and (b) cultivation of the cells in such a way that they express an effector protein when the switch is switched to state 1 **characterized by** the expression of effector 1 in the presence of inducer 1 or when switch is switched to state 2 **characterized by** the expression of effector 2 in the presence of inducer 2.

## Patentansprüche

1. Bistabiler genetischer Kippschalter, umfassend ein Paar wechselseitige Repressorgene A und B und ein Paar Aktivatorgene A und B, die eine positive Feedback-Schleife bilden, beruhend auf modularen DNA-bindenden Proteinen, wobei
(i) das Aktivatorgen A und das Repressorgen B einen Teil getrennter Expressionskassetten bilden, die sich jeweils unter der gemeinsamen Kontrolle des gleichen Operators A befinden, wobei das für Repressor B kodierende Gen durch Aktivator A, der auf den Operator A zielt, aktiviert wird, und
(ii) das Aktivatorgen B und Repressorgen A einen Teil getrennter Expressionskassetten bilden, die sich jeweils unter der gemeinsamen Kontrolle des gleichen Operators B befinden, wobei das für Repressor A kodierende Gen durch Aktivator B, der auf den Operator B zielt, aktiviert wird;
und wobei die Expression von Aktivatorgen B und Repressorgen A durch Repressor B, der auf Operator B zielt, inhibiert wird und die Expression von Aktivatorgen A und Repressorgen B durch Repressor A, der auf Operator A zielt, inhibiert wird.

2. Bistabiler genetischer Kippschalter gemäß Anspruch 1, wobei die Repressoren eine DNA-bindende Domäne und eine Repressionsdomäne umfassen und wobei die Aktivatoren eine DNA-bindende Domäne und eine Aktivierungsdomäne umfassen.

3. Bistabiler genetischer Kippschalter gemäß Anspruch 2, wobei es sich bei der Repressionsdomäne um die KRAB-Domäne handelt und es sich bei der Aktivierungsdomäne entweder um die VP16- oder die VP64-Domäne handelt.

4. Bistabiler genetischer Kippschalter gemäß einem der Ansprüche 1 bis 3, wobei die DNA-bindende Domäne DNA als Monomer bindet.

5. Bistabiler genetischer Kippschalter gemäß einem der Ansprüche 1 bis 4, der stabile Zustände 1 oder 2 besetzen kann, wobei die Zustände 1 oder 2 die beiden unterschiedlichen Zustände des bistabilen Schalters bezeichnen, die durch die Expression von Effektor 1 in Zustand 1 oder Expression von Effektor 2 in Zustand 2 gekennzeichnet sind, und welcher umfasst:
a) ein Operon, das eine Aktivierung von Zustand 1 mit einem Effektorgen 1 vorbringt, bei dem es sich um irgendein Gen oder eine Gruppe von Genen handeln kann, z. B. unterschiedliche Enzyme, fluoreszierende Proteine, Signalproteine, wobei das Operon das Aktivatorgen A, den Operator A und das Effektorgen 1 umfasst und wobei die Transkription dieses Effektorgens 1 durch Operator A reguliert wird, der das in der Nähe eines Promotors lokalisierte DNA-bindende Element A umfasst, und
b) ein Operon, das eine Aktivierung von Zustand 2 mit einem Effektorgen 2 vorbringt, bei dem es sich um irgendein Gen oder eine Gruppe von Genen handeln kann, z. B. unterschiedliche Enzyme, fluoreszierende Proteine, Signalproteine usw., wobei das Operon das Aktivatorgen B, den Operator B und das Effektorgen 2 umfasst und wobei die Transkription dieses Effektorgens 2 durch Operator B reguliert wird, der das in der Nähe eines Promotors lokalisierte DNA-bindende Element B umfasst,
wobei Operator A ein DNA-bindendes Element umfasst, das eine ausgewählte Erkennungsstelle für die DNA-bindende Domäne darstellt, die im Aktivator A und im Repressor A eingeschlossen ist, während Operator B ein DNA-bindendes Element umfasst, das eine ausgewählte Erkennungsstelle für die DNA-bindende Domäne darstellt, die im Aktivator B und im Repressor B eingeschlossen ist.

6. Bistabiler genetischer Kippschalter gemäß einem der Ansprüche 1 bis 5, wobei zwei Strukturgene durch den gleichen Operator A oder B reguliert werden: Aktivator A, Repressor B, Effektor 1 beziehungsweise Aktivator B, Repressor A, Effektor 2; so verknüpft, dass sie durch den gleichen Operator A oder B reguliert werden, und wobei Aktivator A, Repressor B, Effektor 1 beziehungsweise Aktivator B, Repressor A, Effektor 2 durch ein selbstspaltendes 2A-Peptid miteinander verknüpft sind, wobei die für Aktivator A, Repressor B, Effektor 1 kodierenden Sequenzen beziehungsweise die für Aktivator B, Repressor A, Effektor 2 kodierenden Sequenzen jeweils als ein einziges RNA-Molekül von dem jeweiligen Strukturgen transkribiert werden und in zwei getrennte Polypeptidketten translatiert werden, die sich jeweils autokatalytisch an den Positionen der selbstspaltenden Peptide spalten, was zur Synthese getrennter einzelner Proteine von Aktivator A, Repressor B, Effektor 1 beziehungsweise Aktivator B, Repressor A, Effektor 2 in gleichem stöchiometrischem Verhältnis führt.

7. Bistabiler genetischer Kippschalter gemäß einem der Ansprüche 1 bis 6, wobei
a) sich die Transkription des für den Aktivator A zur Aktivierung von Zustand 1 kodierenden Strukturgens und des für den Repressor B zur Inhibierung von Zustand 2 kodierenden Strukturgens auf getrennten Operons unter Kontrolle des gleichen Operators A, der in Anwesenheit von Induktor A aktiviert, und eines Promotors, der entweder konstitutiv oder minimal sein kann, befindet und
b) sich die Transkription des für den Aktivator B zur Aktivierung von Zustand 2 kodierenden Strukturgens und des Strukturgens für den Repressor A zur Inhibierung von Zustand 1 auf getrennten Operons unter Kontrolle des gleichen Operators B, der in Anwesenheit von Induktor B aktiviert, und eines Promotors, der entweder konstitutiv oder minimal sein kann, befindet.

8. Bistabiler genetischer Kippschalter gemäß einem der Ansprüche 5 - 7, wobei der Operator A oder B des Operons eine beliebige Anzahl gleicher oder unterschiedlicher Arten DNA-bindender Elemente einschließt, die in Cluster verknüpft oder in abwechselnder Reihenfolge verteilt sein können, wobei der Operator vorzugsweise 1 bis 20 DNA-bindende Elemente einschließt, die durch Nucleotidsequenz beliebiger Länge getrennt sind, und wobei Operator A der Operons zur Expression von Zustand 1, **gekennzeichnet durch** die Expression von Effektor 1, sich von Operator B der Operons zur Expression von Zustand 2, **gekennzeichnet durch** die Expression von Effektor 2, unterscheidet und sich von Operator A der Operons zum Umschalten auf Zustand 1, **gekennzeichnet durch** die Expression von Effektor 1, und Operator B der Operons zum Umschalten auf Zustand 2, **gekennzeichnet durch** die Expression von Effektor 2, unterscheidet und
a) DNA-bindende Elemente in Operator A so gestaltet sind, dass sie die DNA-bindende Domäne von Repressor A und Aktivator A binden,
b) DNA-bindende Elemente in Operator B so gestaltet sind, dass sie die DNA-bindende Domäne von Repressor B und Aktivator B binden.

9. Bistabiler genetischer Kippschalter gemäß einem der Ansprüche 1 bis 8, welcher zur kontrollierten Synthese von Effektor 1 oder Effektor 2 verwendet werden kann, je nach dem Zustand des Schalters und der Anwesenheit von Induktoren zur Aktivierung eines spezifischen Zustands, wobei es sich bei dem Effektor 1 oder 2 um irgendeine Art von Protein oder eine Gruppe von Proteinen handeln kann.

10. Bistabiler genetischer Kippschalter gemäß einem der Ansprüche 1 bis 9, welcher als Indikator verwendet werden kann, der durch Synthese von Effektor 1 oder Effektor 2 die Anwesenheit eines Induktors zur Aktivierung eines spezifischen Zustands anzeigt, wobei es sich bei den Effektoren um irgendeine Art von Protein oder eine Gruppe von Proteinen handeln kann

11. Bistabiler genetischer Kippschalter gemäß einem der Ansprüche 1 bis 10, umfassend DNA mit der Sequenz irgendeiner der SEQ ID NO: 1, 2, 4, 5, 7, 8, 9 und 10.

12. Prokaryotische oder eukaryotische Wirtszellen, welche einen bistabilen genetischen Kippschalter gemäß einem der Ansprüche 1 bis 11 enthalten.

13. Verfahren zur Effektorgenexpression, welches umfasst (a) Wirtszellen gemäß Anspruch 12, welche einen bistabilen genetischen Kippschalter gemäß einem der Ansprüche 1 bis 11 enthalten, und (b) Züchten der Zellen auf solche Weise, dass sie ein Effektorprotein exprimieren, wenn der Schalter auf Zustand 1, **gekennzeichnet durch** die Expression von Effektor 1 in Anwesenheit von Induktor 1, geschaltet ist, oder wenn der Schalter auf Zustand 2, **gekennzeichnet durch** die Expression von Effektor 2 in Anwesenheit von Induktor 2, geschaltet ist.

## Revendications

1. Interrupteur à bascule génétique bistable comprenant une paire de gènes répresseurs réciproques A et B et une paire de gènes activateurs A et B formant une chaîne de réaction positive basé sur des protéines de liaison à l'ADN modulaires, où
(i) le gène activateur A et le gène répresseur B font partie de cassettes d'expression distinctes chacune sous la commande conjointe du même opérateur A, le gène codant pour le répresseur B étant activé par l'activateur A ciblant ledit opérateur A, et
(ii) le gène activateur B et le gène répresseur A font partie de cassettes d'expression distinctes chacune sous la commande conjointe du même opérateur B, le gène codant pour le répresseur A étant activé par l'activateur B ciblant ledit opérateur B ;
et où l'expression du gène activateur B et du gène répresseur A est inhibée par le répresseur B ciblant l'opérateur B, et l'expression du gène activateur A et du gène répresseur B est inhibée par le répresseur A ciblant l'opérateur A.

2. Interrupteur à bascule génétique bistable selon la revendication 1, où les répresseurs comprennent un domaine de liaison à l'ADN et un domaine de répression, et où les activateurs comprennent un domaine de liaison à l'ADN et un domaine d'activation.

3. Interrupteur à bascule génétique bistable selon la revendication 2, où le domaine de répression est le domaine KRAB et le domaine d'activation est le domaine VP16 ou le domaine VP64.

4. Interrupteur à bascule génétique bistable selon l'une quelconque des revendications 1 à 3, où le domaine de liaison à l'ADN lie l'ADN comme un monomère.

5. Interrupteur à bascule génétique bistable selon l'une quelconque des revendications 1 à 4, qui peut occuper des états stables 1 ou 2, lesdits états stables 1 ou 2 désignant les deux états différents de l'interrupteur bistable **caractérisés par** l'expression de l'effecteur 1 dans l'état 1 ou l'expression de l'effecteur 2 dans l'état 2, et qui comprend :
a) un opéron présentant une activation de l'état 1 avec un gène effecteur 1 qui peut être n'importe quel gène ou un groupe de gènes, par exemple différentes enzymes, protéines fluorescentes, protéines de signalisation, où l'opéron comprend le gène activateur A, l'opérateur A et le gène effecteur 1, la transcription de ce gène effecteur 1 étant régulée par l'opérateur A qui comprend l'élément A de liaison à l'ADN situé à proximité d'un promoteur, et
b) un opéron présentant une activation de l'état 2 avec un gène effecteur 2 qui peut être n'importe quel gène ou un groupe de gènes, par exemple différentes enzymes, protéines fluorescentes, protéines de signalisation, etc., où l'opéron comprend le gène activateur B, l'opérateur B et le gène effecteur 2, la transcription de ce gène effecteur 2 étant régulée par l'opérateur B qui comprend l'élément B de liaison à l'ADN situé à proximité d'un promoteur,
où l'opérateur A comprend un élément de liaison à l'ADN qui représente un site de reconnaissance sélectionné pour le domaine de liaison à l'ADN qui est inclus dans l'activateur A et le répresseur A, tandis que l'opérateur B comprend un élément de liaison à l'ADN qui représente un site de reconnaissance sélectionné pour le domaine de liaison à l'ADN qui est inclus dans l'activateur B et le répresseur B.

6. Interrupteur à bascule génétique bistable selon l'une quelconque des revendications 1 à 5, où deux gènes structuraux sont régulés par le même opérateur A ou B : respectivement l'activateur A, le répresseur B, l'effecteur 1, et l'activateur B, le répresseur A, l'effecteur 2 ; couplés ensemble de façon à être régulés par le même opérateur A ou B, et où l'activateur A, le répresseur B, l'effecteur 1, et l'activateur B, le répresseur A, l'effecteur 2, sont respectivement liés ensemble par un peptide 2A auto-clivant, où les séquences codantes pour l'activateur A, le répresseur B, l'effecteur 1, et les séquences codantes pour l'activateur B, le répresseur A et l'effecteur 2 sont chacune transcrites respectivement à partir du gène structural respectif en tant que molécule d'ARN simple et sont traduites en deux chaînes polypeptidiques distinctes qui se clivent chacune de façon auto-catalytique au niveau des positions des peptides auto-clivants, ce qui conduit à la synthèse de protéines individuelles distinctes respectivement constituées de l'activateur A, du répresseur B, de l'effecteur 1, et de l'activateur B, du répresseur A et de l'effecteur 2 dans un rapport stoechiométrique égal.

7. Interrupteur à bascule génétique bistable selon l'une quelconque des revendications 1 à 6, où
a) la transcription du gène structural codant pour l'activateur A destiné à activer l'état 1 et du gène structural codant pour le répresseur B destiné à inhiber l'état 2 sur des opérons distincts est commandée par le même opérateur A, qui s'active en présence d'un inducteur A, et d'un promoteur, qui peut être constitutif ou minimal, et
b) la transcription du gène structural codant pour l'activateur B destiné à activer l'état 2 et du gène structural codant pour le répresseur A destiné à inhiber l'état 1 sur des opérons distincts est commandée par le même opérateur B, qui s'active en présence d'un inducteur B, et d'un promoteur, qui peut être constitutif ou minimal.

8. Interrupteur à bascule génétique bistable selon l'une quelconque des revendications 5 à 7, où l'opérateur A ou B de l'opéron inclut n'importe quel nombre d'éléments de liaison à l'ADN de type identique ou différent, qui peuvent être couplés dans des clusters ou répartis de façon alternée, l'opérateur incluant de préférence de 1 à 20 éléments de liaison à l'ADN, qui sont séparés par une séquence de nucléotides de n'importe quelle longueur, et où l'opérateur A des opérons destinés à exprimer l'état 1 **caractérisé par** l'expression de l'effecteur 1 diffère de l'opérateur B des opérons destinés à exprimer l'état 2 **caractérisé par** l'expression de l'effecteur 2, et diffère de l'opérateur A des opérons destinés à basculer vers l'état 1 **caractérisé par** l'expression de l'effecteur 1 et de l'opérateur B des opérons destinés à basculer vers l'état 2 **caractérisé par** l'expression de l'effecteur 2, et
a) les éléments de liaison à l'ADN dans l'opérateur A sont conçus pour lier le domaine de liaison à l'ADN du répresseur A et de l'activateur A,
b) les éléments de liaison à l'ADN dans l'opérateur B sont conçus pour lier le domaine de liaison à l'ADN du répresseur B et de l'activateur B.

9. Interrupteur à bascule génétique bistable selon l'une quelconque des revendications 1 à 8, qui peut être utilisé pour commander la synthèse de l'effecteur 1 et de l'effecteur 2, en fonction de l'état de l'interrupteur et de la présence d'inducteurs destinés à activer un état spécifique, l'effecteur 1 ou 2 pouvant être n'importe quel type de protéine ou un groupe de protéines.

10. Interrupteur à bascule génétique bistable selon l'une quelconque des revendications 1 à 9, qui peut être utilisé comme indicateur qui, par le biais de la synthèse de l'effecteur 1 ou de l'effecteur 2, indique la présence d'un inducteur d'activation d'un état spécifique, les effecteurs pouvant être n'importe quel type de protéine ou un groupe de protéines.

11. Interrupteur à bascule génétique bistable selon l'une quelconque des revendications 1 à 10, comprenant un ADN de séquence selon l'une quelconque des SEQ ID NO: 1, 2, 4, 5, 7, 8, 9 et 10.

12. Cellules hôtes procaryotes ou eucaryotes qui contiennent un interrupteur à bascule génétique bistable selon l'une quelconque des revendications 1 à 11.

13. Procédé d'expression d'un gène effecteur, qui comprend (a) des cellules hôtes selon la revendication 12 contenant un interrupteur à bascule génétique bistable selon l'une quelconque des revendications 1 à 11 et (b) la culture des cellules d'une manière telle qu'elles expriment une protéine effectrice quand l'interrupteur est basculé sur l'état 1 **caractérisé par** l'expression de l'effecteur 1 en présence d'un inducteur 1 ou quand l'interrupteur est basculé sur l'état 2 **caractérisé par** l'expression de l'effecteur 2 en présence d'un inducteur 2.


### SEQUENCE LISTING

<110> Kemijski inš̌̌titut, Ljubljana, Slovenia and EN-FIST Center odlič̌ňosti, Ljubljana, Slovenia
<120> Bistable genetic toggle switch comprising a pair of reciprocal Repressors and a positive feedback loop based on DNA-binding Proteins
<130> 301-P31PC/13
<140> -
   <141> 2013-09-11
<150> SI P-201200285
   <151> 2012-09-20
<160> 22
<170> PatentIn version 3.5
<210> 1
   <211> 3465
   <212> DNA
   <213> Unknown
<220>
   <223> Operon composed of operator DNA-binding elements, minimal or constitutive promoter, structural genes
<400> 1
   taaagagcag aaactcatct tccgtttcca catcttgctg gcagccgggg taccgcaggc     60
   aagtcgctaa agagcagaaa ctcatcttcc gtttccacat ctatggaatc acggcggccg    120
   cccgtatctc atcctaaaga gcagaaactc atcttccgtt tccacatcta atgcgtcggc    180
   gtgcggccgc tgccagttgc ataaagagca gaaactcatc ttccgtttcc acatctttgg    240
   ctgtcctagg gtacctggac gctccttgta aagagcagaa actcatcttc cgtttccaca    300
   tcttggctgg tgccgcaccg gtaagcagaa ctagaggtgg caccggtttg attgcacagc    360
   taaagagcag aaactcatct tccgtttcca catcttgctg gcagccgggg taccgcaggc    420
   aagtcgctaa agagcagaaa ctcatcttcc gtttccacat ctatggaatc acggcggccg    480
   cccgtatctc atcctaaaga gcagaaactc atcttccgtt tccacatcta atgcgtcggc    540
   gtgcggccgc tgccagttgc ataaagagca gaaactcatc ttccgtttcc acatctttgg    600
   ctgtcctagg gtacctggac gctccttgta aagagcagaa actcatcttc cgtttccaca    660
   tcttggctgg tgccgcaccg gtaagcagag gatcttagag ggtatataat ggaagctcga    720
   cttccagctc gagggcaatc cggtactgtt ggtaaagcca ccgaattcgc caccatgcac    780
   caccaccacc accacgacta caaagaccat gacggtgatt ataaagatca tgacatcgat    840
   tacaaggatg acgatgacaa gatggccccc aagaagaaga ggaaggtggg cattcaccgc    900
   ggggtaccta tggtggactt gaggacactc ggttattcgc aacagcaaca ggagaaaatc    960
   aagcctaagg tcaggagcac cgtcgcgcaa caccacgagg cgcttgtggg gcatggcttc   1020
   actcatgcgc atattgtcgc gctttcacag caccctgcgg cgcttgggac ggtggctgtc   1080
   aaataccaag atatgattgc ggccctgccc gaagccacgc acgaggcaat tgtaggggtc   1140
   ggtaaacagt ggtcgggagc gcgagcactt gaggcgctgc tgactgtggc gggtgagctt   1200
   agggggcctc cgctccagct cgacaccggg cagctgctga agatcgcgaa gagaggggga   1260
   gtaacagcgg tagaggcagt gcacgcctgg cgcaatgcgc tcaccggggc ccccttgaac    1320
   ctgaccccag accaggtagt cgcaatcgcg tcacatgacg gggaaagcaa agccctggaa    1380
   accgtgcaaa ggttgttgcc ggtcctttgt caagaccacg gccttacacc ggagcaagtc   1440
   gtggccattg caagcaatgg gggtggcaaa caggctcttg agacggttca gagacttctc   1500
   ccagttctct gtcaagccca cgggctgact cccgatcaag ttgtagcgat tgcgtccaac   1560
   ggtggaggga aacaagcatt ggagactgtc caacggctcc ttcccgtgtt gtgtcaagcc   1620
   cacggtttga cgcctgcaca agtggtcgcc atcgccagcc atgatggcgg taagcaggcg   1680
   ctggaaacag tacagcgcct gctgcctgta ctgtgccagg atcatggact gaccccagac   1740
   caggtagtcg caatcgcgtc acatgacggg ggaaagcaag ccctggaaac cgtgcaaagg   1800
   ttgttgccgg tcctttgtca agaccacggc cttacaccgg agcaagtcgt ggccattgca   1860
   aataataacg gtggcaaaca ggctcttgag acggttcaga gacttctccc agttctctgt   1920
   caagcccacg ggctgactcc cgatcaagtt gtagcgattg cgtccaacgg tggagggaaa   1980
   caagcattgg agactgtcca acggctcctt cccgtgttgt gtcaagccca cggtttgacg   2040
   cctgcacaag tggtcgccat cgcctcgaat ggcggcggta agcaggcgct ggaaacagta   2100
   cagcgcctgc tgcctgtact gtgccaggat catggactga ccccagacca ggtagtcgca   2160
   atcgcgtcaa acggaggggg aaagcaagcc ctggaaaccg tgcaaaggtt gttgccggtc     2220
   ctttgtcaag accacggcct tacaccggag caagtcgtgg ccattgcatc ccacgacggt     2280
   ggcaaacagg ctcttgagac ggttcagaga cttctcccag ttctctgtca agcccacggg     2340
   ctgactcccg atcaagttgt agcgattgcg tcgcatgacg gagggaaaca agcattggag     2400
   actgtccaac ggctccttcc cgtgttgtgt caagcccacg gtttgacgcc tgcacaagtg     2460
   gtcgccatcg cctccaatat tggcggtaag caggcgctgg aaacagtaca gcgcctgctg     2520
   cctgtactgt gccaggatca tggactgacc ccagaccagg tagtcgcaat cgcgtcacat     2580
   gacgggggaa agcaagccct ggaaaccgtg caaaggttgt tgccggtcct ttgtcaagac     2640
   cacggcctta caccggagca agtcgtggcc attgcaagca acatcggtgg caaacaggct     2700
   cttgagacgg ttcagagact tctcccagtt ctctgtcaag cccacgggct gactcccgat     2760
   caagttgtag cgattgcgtc caacggtgga gggaaacaag cattggagac tgtccaacgg     2820
   ctccttcccg tgttgtgtca agcccacggt ttgacgcctg cacaagtggt cgccatcgcc     2880
   agccatgatg gcggtaagca ggcgctggaa acagtacagc gcctgctgcc tgtactgtgc     2940
   caggatcatg gactgacacc cgaacaggtg gtcgccattg cttctaatgg gggaggacgg     3000
   ccagccttgg agtccatcgt agcccaattg tccaggcccg atcccgcgtt ggctgcgtta     3060
   acgaatgacc atctggtggc gttggcatgt cttggtggac gacccgcgct cgatgcagtc     3120
   aaaaagggtc tgcctcatgc tcccgcattg atcaaaagaa ccaaccggcg gattcccgag     3180
   agaacttccc atcgagtcgc gggatccgat ccaaaaaaga agagaaaggt agcccccccg     3240
   accgatgtca gcctggggga cgagctccac ttagacggcg aggacgtggc gatggcgcat     3300
   gccgacgcgc tagacgattt cgatctggac atgttggggg acggggattc cccgggtccg     3360
   ggatttaccc cccacgactc cgccccctac ggcgctctgg atatggccga cttcgagttt     3420
   gagcagatgt ttaccgatgc ccttggaatt gacgagtacg gtggg                     3465
<210> 2
   <211> 4395
   <212> DNA
   <213> Unknown
<220>
   <223> Synthetic genetic construct
<400> 2
   taaagagcag aaactcatct tccgtttcca catcttgctg gcagccgggg taccgcaggc       60
   aagtcgctaa agagcagaaa ctcatcttcc gtttccacat ctatggaatc acggcggccg      120
   cccgtatctc atcctaaaga gcagaaactc atcttccgtt tccacatcta atgcgtcggc      180
   gtgcggccgc tgccagttgc ataaagagca gaaactcatc ttccgtttcc acatctttgg      240
   ctgtcctagg gtacctggac gctccttgta aagagcagaa actcatcttc cgtttccaca      300
   tcttggctgg tgccgcaccg gtaagcagaa ctagaggtgg caccggtttg attgcacagc      360
   taaagagcag aaactcatct tccgtttcca catcttgctg gcagccgggg taccgcaggc      420
   aagtcgctaa agagcagaaa ctcatcttcc gtttccacat ctatggaatc acggcggccg      480
   cccgtatctc atcctaaaga gcagaaactc atcttccgtt tccacatcta atgcgtcggc      540
   gtgcggccgc tgccagttgc ataaagagca gaaactcatc ttccgtttcc acatctttgg      600
   ctgtcctagg gtacctggac gctccttgta aagagcagaa actcatcttc cgtttccaca      660
   tcttggctgg tgccgcaccg gtaagcagag gatcttagag ggtatataat ggaagctcga      720
   cttccagctc gagggcaatc cggtactgtt ggtaaagcca ccgaattcgc caccatgcac      780
   caccaccacc accacgacta caaagaccat gacggtgatt ataaagatca tgacatcgat      840
   tacaaggatg acgatgacaa gatggccccc aagaagaaga ggaaggtggg cattcaccgc      900
   ggggtaccta tggtggactt gaggacactc ggttattcgc aacagcaaca ggagaaaatc      960
   aagcctaagg tcaggagcac cgtcgcgcaa caccacgagg cgcttgtggg gcatggcttc     1020
   actcatgcgc atattgtcgc gctttcacag caccctgcgg cgcttgggac ggtggctgtc     1080
   aaataccaag atatgattgc ggccctgccc gaagccacgc acgaggcaat tgtaggggtc     1140
   ggtaaacagt ggtcgggagc gcgagcactt gaggcgctgc tgactgtggc gggtgagctt     1200
   agggggcctc cgctccagct cgacaccggg cagctgctga agatcgcgaa gagaggggga     1260
   gtaacagcgg tagaggcagt gcacgcctgg cgcaatgcgc tcaccggggc ccccttgaac    1320
   ctgaccccag accaggtagt cgcaatcgcg tcaaacggag ggggaaagca agccctggaa    1380
   accgtgcaaa ggttgttgcc ggtcctttgt caagaccacg gccttacacc ggagcaagtc     1440
   gtggccattg caagcaatgg gggtggcaaa caggctcttg agacggttca gagacttctc     1500
   ccagttctct gtcaagccca cgggctgact cccgatcaag ttgtagcgat tgcgtcgaac     1560
   attggaggga aacaagcatt ggagactgtc caacggctcc ttcccgtgtt gtgtcaagcc     1620
   cacggtttga cgcctgcaca agtggtcgcc atcgccagcc atgatggcgg taagcaggcg     1680
   ctggaaacag tacagcgcct gctgcctgta ctgtgccagg atcatggact gaccccagac     1740
   caggtagtcg caatcgcgtc aaacggaggg ggaaagcaag ccctggaaac cgtgcaaagg     1800
   ttgttgccgg tcctttgtca agaccacggc cttacaccgg agcaagtcgt ggccattgca     1860
   aataataacg gtggcaaaca ggctcttgag acggttcaga gacttctccc agttctctgt     1920
   caagcccacg ggctgactcc cgatcaagtt gtagcgattg cgtcgcatga cggagggaaa     1980
   caagcattgg agactgtcca acggctcctt cccgtgttgt gtcaagccca cggtttgacg     2040
   cctgcacaag tggtcgccat cgcctcgaat ggcggcggta agcaggcgct ggaaacagta     2100
   cagcgcctgc tgcctgtact gtgccaggat catggactga ccccagacca ggtagtcgca     2160
   atcgcgaaca ataatggggg aaagcaagcc ctggaaaccg tgcaaaggtt gttgccggtc     2220
   ctttgtcaag accacggcct tacaccggag caagtcgtgg ccattgcatc ccacgacggt     2280
   ggcaaacagg ctcttgagac ggttcagaga cttctcccag ttctctgtca agcccacggg     2340
   ctgactcccg atcaagttgt agcgattgcg tccaacggtg gagggaaaca agcattggag     2400
   actgtccaac ggctccttcc cgtgttgtgt caagcccacg gtttgacgcc tgcacaagtg     2460
   gtcgccatcg ccagccatga tggcggtaag caggcgctgg aaacagtaca gcgcctgctg     2520
   cctgtactgt gccaggatca tggactgacc ccagaccagg tagtcgcaat cgcgtcacat     2580
   gacgggggaa agcaagccct ggaaaccgtg caaaggttgt tgccggtcct ttgtcaagac     2640
   cacggcctta caccggagca agtcgtggcc attgcatccc acgacggtgg caaacaggct     2700
   cttgagacgg ttcagagact tctcccagtt ctctgtcaag cccacgggct gactcccgat     2760
   caagttgtag cgattgcgaa taacaatgga gggaaacaag cattggagac tgtccaacgg     2820
   ctccttcccg tgttgtgtca agcccacggt ttgacgcctg cacaagtggt cgccatcgcc     2880
   agccatgatg gcggtaagca ggcgctggaa acagtacagc gcctgctgcc tgtactgtgc     2940
   caggatcatg gactgacacc cgaacaggtg gtcgccattg cttctaatgg gggaggacgg     3000
   ccagccttgg agtccatcgt agcccaattg tccaggcccg atcccgcgtt ggctgcgtta     3060
   acgaatgacc atctggtggc gttggcatgt cttggtggac gacccgcgct cgatgcagtc     3120
   aaaaagggtc tgcctcatgc tcccgcattg atcaaaagaa ccaaccggcg gattcccgag     3180
   agaacttccc atcgagtcgc gggatccgat ccaaaaaaga agagaaaggt accaaaaaag     3240
   aagagaaagg tcgacggcgg tggtgctttg tctcctcagc actctgctgt cactcaagga     3300
   agtatcatca agaacaagga gggcatggat gctaagtcac taactgcctg gtcccggaca     3360
   ctggtgacct tcaaggatgt atttgtggac ttcaccaggg aggagtggaa gctgctggac     3420
   actgctcagc agatcgtgta cagaaatgtg atgctggaga actataagaa cctggtttcc     3480
   ttgggttatc agcttactaa gccagatgtg atcctccggt tggagaaggg agaagagccc     3540
   tggctggtgg agagagaaat tcaccaagag acccatcctg attcagagac tgcatttgaa     3600
   atcaaatcat cagttagagc cgaggggaga ggaagtcttc tgacctgcgg agacgtcgaa     3660
   gagaatcctg gacccatggt gagcaagggc gaggagctgt tcaccggggt ggtgcccatc     3720
   ctggtcgagc tggacggcga cgtaaacggc cacaagttca gcgtgtccgg cgagggcgag     3780
   ggcgatgcca cctacggcaa gctgaccctg aagttcatct gcaccaccgg caagctgccc     3840
   gtgccctggc ccaccctcgt gaccaccttc ggctacggcc tgatgtgctt cgcccgctac     3900
   cccgaccaca tgaagcagca cgacttcttc aagtccgcca tgcccgaagg ctacgtccag     3960
   gagcgcacca tcttcttcaa ggacgacggc aactacaaga cccgcgccga ggtgaagttc     4020
   gagggcgaca ccctggtgaa ccgcatcgag ctgaagggca tcgacttcaa ggaggacggc     4080
   aacatcctgg ggcacaagct ggagtacaac tacaacagcc acaacgtcta tatcatggcc     4140
   gacaagcaga agaacggcat caaggtgaac ttcaagatcc gccacaacat cgaggacggc     4200
   agcgtgcagc tcgccgacca ctaccagcag aacaccccca tcggcgacgg ccccgtgctg     4260
   ctgcccgaca accactacct gagctaccag tccgccctga gcaaagaccc caacgagaag     4320
   cgcgatcaca tggtcctgct ggagttcgtg accgccgccg ggatcactct cggcatggac     4380
   gagctgtaca agtaa                                                      4395
<210> 3
   <211> 7125
   <212> DNA
   <213> Unknown
<220>
   <223> Operon composed of operator DNA-binding elements, minimal or constitutive promoter, structural genes
<400> 3
   taaagagcag aaactcatct tccgtttcca catcttgctg gcagccgggg taccgcaggc      60
   aagtcgctaa agagcagaaa ctcatcttcc gtttccacat ctatggaatc acggcggccg     120
   cccgtatctc atcctaaaga gcagaaactc atcttccgtt tccacatcta atgcgtcggc     180
   gtgcggccgc tgccagttgc ataaagagca gaaactcatc ttccgtttcc acatctttgg     240
   ctgtcctagg gtacctggac gctccttgta aagagcagaa actcatcttc cgtttccaca     300
   tcttggctgg tgccgcaccg gtaagcagaa ctagaggtgg caccggtttg attgcacagc     360
   taaagagcag aaactcatct tccgtttcca catcttgctg gcagccgggg taccgcaggc     420
   aagtcgctaa agagcagaaa ctcatcttcc gtttccacat ctatggaatc acggcggccg     480
   cccgtatctc atcctaaaga gcagaaactc atcttccgtt tccacatcta atgcgtcggc     540
   gtgcggccgc tgccagttgc ataaagagca gaaactcatc ttccgtttcc acatctttgg     600
   ctgtcctagg gtacctggac gctccttgta aagagcagaa actcatcttc cgtttccaca     660
   tcttggctgg tgccgcaccg gtaagcagag gatcttagag ggtatataat ggaagctcga     720
   cttccagctc gagggcaatc cggtactgtt ggtaaagcca ccgaattcgc caccatgcac     780
   caccaccacc accacgacta caaagaccat gacggtgatt ataaagatca tgacatcgat     840
   tacaaggatg acgatgacaa gatggccccc aagaagaaga ggaaggtggg cattcaccgc     900
   ggggtaccta tggtggactt gaggacactc ggttattcgc aacagcaaca ggagaaaatc     960
   aagcctaagg tcaggagcac cgtcgcgcaa caccacgagg cgcttgtggg gcatggcttc    1020
   actcatgcgc atattgtcgc gctttcacag caccctgcgg cgcttgggac ggtggctgtc    1080
   aaataccaag atatgattgc ggccctgccc gaagccacgc acgaggcaat tgtaggggtc    1140
   ggtaaacagt ggtcgggagc gcgagcactt gaggcgctgc tgactgtggc gggtgagctt    1200
   agggggcctc cgctccagct cgacaccggg cagctgctga agatcgcgaa gagaggggga    1260
   gtaacagcgg tagaggcagt gcacgcctgg cgcaatgcgc tcaccggggc ccccttgaac 1320
   ctgaccccag accaggtagt cgcaatcgcg tcaaacggag ggggaaagca agccctggaa 1380
   accgtgcaaa ggttgttgcc ggtcctttgt caagaccacg gccttacacc ggagcaagtc 1440
   gtggccattg caagcaatgg gggtggcaaa caggctcttg agacggttca gagacttctc 1500
   ccagttctct gtcaagccca cgggctgact cccgatcaag ttgtagcgat tgcgtcgaac 1560
   attggaggga aacaagcatt ggagactgtc caacggctcc ttcccgtgtt gtgtcaagcc 1620
   cacggtttga cgcctgcaca agtggtcgcc atcgccagcc atgatggcgg taagcaggcg 1680
   ctggaaacag tacagcgcct gctgcctgta ctgtgccagg atcatggact gaccccagac 1740
   caggtagtcg caatcgcgtc aaacggaggg ggaaagcaag ccctggaaac cgtgcaaagg 1800
   ttgttgccgg tcctttgtca agaccacggc cttacaccgg agcaagtcgt ggccattgca 1860
   aataataacg gtggcaaaca ggctcttgag acggttcaga gacttctccc agttctctgt 1920
   caagcccacg ggctgactcc cgatcaagtt gtagcgattg cgtcgcatga cggagggaaa 1980
   caagcattgg agactgtcca acggctcctt cccgtgttgt gtcaagccca cggtttgacg 2040
   cctgcacaag tggtcgccat cgcctcgaat ggcggcggta agcaggcgct ggaaacagta 2100
   cagcgcctgc tgcctgtact gtgccaggat catggactga ccccagacca ggtagtcgca 2160
   atcgcgaaca ataatggggg aaagcaagcc ctggaaaccg tgcaaaggtt gttgccggtc 2220
   ctttgtcaag accacggcct tacaccggag caagtcgtgg ccattgcatc ccacgacggt 2280
   ggcaaacagg ctcttgagac ggttcagaga cttctcccag ttctctgtca agcccacggg 2340
   ctgactcccg atcaagttgt agcgattgcg tccaacggtg gagggaaaca agcattggag 2400
   actgtccaac ggctccttcc cgtgttgtgt caagcccacg gtttgacgcc tgcacaagtg 2460
   gtcgccatcg ccagccatga tggcggtaag caggcgctgg aaacagtaca gcgcctgctg 2520
   cctgtactgt gccaggatca tggactgacc ccagaccagg tagtcgcaat cgcgtcacat 2580
   gacgggggaa agcaagccct ggaaaccgtg caaaggttgt tgccggtcct ttgtcaagac 2640
   cacggcctta caccggagca agtcgtggcc attgcatccc acgacggtgg caaacaggct 2700
   cttgagacgg ttcagagact tctcccagtt ctctgtcaag cccacgggct gactcccgat 2760
   caagttgtag cgattgcgaa taacaatgga gggaaacaag cattggagac tgtccaacgg 2820
   ctccttcccg tgttgtgtca agcccacggt ttgacgcctg cacaagtggt cgccatcgcc 2880
   agccatgatg gcggtaagca ggcgctggaa acagtacagc gcctgctgcc tgtactgtgc 2940
   caggatcatg gactgacacc cgaacaggtg gtcgccattg cttctaatgg gggaggacgg 3000
   ccagccttgg agtccatcgt agcccaattg tccaggcccg atcccgcgtt ggctgcgtta 3060
   acgaatgacc atctggtggc gttggcatgt cttggtggac gacccgcgct cgatgcagtc 3120
   aaaaagggtc tgcctcatgc tcccgcattg atcaaaagaa ccaaccggcg gattcccgag 3180
   agaacttccc atcgagtcgc gggatccgat ccaaaaaaga agagaaaggt accaaaaaag 3240
   aagagaaagg tcgacggcgg tggtgctttg tctcctcagc actctgctgt cactcaagga 3300
   agtatcatca agaacaagga gggcatggat gctaagtcac taactgcctg gtcccggaca 3360
   ctggtgacct tcaaggatgt atttgtggac ttcaccaggg aggagtggaa gctgctggac 3420
   actgctcagc agatcgtgta cagaaatgtg atgctggaga actataagaa cctggtttcc 3480
   ttgggttatc agcttactaa gccagatgtg atcctccggt tggagaaggg agaagagccc 3540
   tggctggtgg agagagaaat tcaccaagag acccatcctg attcagagac tgcatttgaa 3600
   atcaaatcat cagttagagc cgaggggaga ggaagtcttc tgacctgcgg agacgtcgaa 3660
   gagaatcctg gacccgacta caaagaccat gacggtgatt ataaagatca tgacatcgat 3720
   tacaaggatg acgatgacaa gatggccccc aagaagaaga ggaaggtggg cattcaccgc 3780
   ggggtaccta tggtggactt gaggacactc ggttattcgc aacagcaaca ggagaaaatc 3840
   aagcctaagg tcaggagcac cgtcgcgcaa caccacgagg cgcttgtggg gcatggcttc 3900
   actcatgcgc atattgtcgc gctttcacag caccctgcgg cgcttgggac ggtggctgtc 3960
   aaataccaag atatgattgc ggccctgccc gaagccacgc acgaggcaat tgtaggggtc 4020
   ggtaaacagt ggtcgggagc gcgagcactt gaggcgctgc tgactgtggc gggtgagctt      4080
   agggggcctc cgctccagct cgacaccggg cagctgctga agatcgcgaa gagaggggga      4140
   gtaacagcgg tagaggcagt gcacgcctgg cgcaatgcgc tcaccggggc ccccttgaac 4200
   ctgaccccag accaggtagt cgcaatcgcg tcacatgacg ggggaaagca agccctggaa 4260
   accgtgcaaa ggttgttgcc ggtcctttgt caagaccacg gccttacacc ggagcaagtc 4320
   gtggccattg caagcaatgg gggtggcaaa caggctcttg agacggttca gagacttctc 4380
   ccagttctct gtcaagccca cgggctgact cccgatcaag ttgtagcgat tgcgtccaac 4440
   ggtggaggga aacaagcatt ggagactgtc caacggctcc ttcccgtgtt gtgtcaagcc 4500
   cacggtttga cgcctgcaca agtggtcgcc atcgccagcc atgatggcgg taagcaggcg 4560
   ctggaaacag tacagcgcct gctgcctgta ctgtgccagg atcatggact gaccccagac 4620
   caggtagtcg caatcgcgtc acatgacggg ggaaagcaag ccctggaaac cgtgcaaagg 4680
   ttgttgccgg tcctttgtca agaccacggc cttacaccgg agcaagtcgt ggccattgca 4740
   aataataacg gtggcaaaca ggctcttgag acggttcaga gacttctccc agttctctgt 4800
   caagcccacg ggctgactcc cgatcaagtt gtagcgattg cgtccaacgg tggagggaaa 4860
   caagcattgg agactgtcca acggctcctt cccgtgttgt gtcaagccca cggtttgacg 4920
   cctgcacaag tggtcgccat cgcctcgaat ggcggcggta agcaggcgct ggaaacagta 4980
   cagcgcctgc tgcctgtact gtgccaggat catggactga ccccagacca ggtagtcgca 5040
   atcgcgtcaa acggaggggg aaagcaagcc ctggaaaccg tgcaaaggtt gttgccggtc 5100
   ctttgtcaag accacggcct tacaccggag caagtcgtgg ccattgcatc ccacgacggt 5160
   ggcaaacagg ctcttgagac ggttcagaga cttctcccag ttctctgtca agcccacggg 5220
   ctgactcccg atcaagttgt agcgattgcg tcgcatgacg gagggaaaca agcattggag 5280
   actgtccaac ggctccttcc cgtgttgtgt caagcccacg gtttgacgcc tgcacaagtg 5340
   gtcgccatcg cctccaatat tggcggtaag caggcgctgg aaacagtaca gcgcctgctg      5400
   cctgtactgt gccaggatca tggactgacc ccagaccagg tagtcgcaat cgcgtcacat      5460
   gacgggggaa agcaagccct ggaaaccgtg caaaggttgt tgccggtcct ttgtcaagac      5520
   cacggcctta caccggagca agtcgtggcc attgcaagca acatcggtgg caaacaggct      5580
   cttgagacgg ttcagagact tctcccagtt ctctgtcaag cccacgggct gactcccgat      5640
   caagttgtag cgattgcgtc caacggtgga gggaaacaag cattggagac tgtccaacgg      5700
   ctccttcccg tgttgtgtca agcccacggt ttgacgcctg cacaagtggt cgccatcgcc      5760
   agccatgatg gcggtaagca ggcgctggaa acagtacagc gcctgctgcc tgtactgtgc      5820
   caggatcatg gactgacacc cgaacaggtg gtcgccattg cttctaatgg gggaggacgg      5880
   ccagccttgg agtccatcgt agcccaattg tccaggcccg atcccgcgtt ggctgcgtta      5940
   acgaatgacc atctggtggc gttggcatgt cttggtggac gacccgcgct cgatgcagtc      6000
   aaaaagggtc tgcctcatgc tcccgcattg atcaaaagaa ccaaccggcg gattcccgag      6060
   agaacttccc atcgagtcgc gggatccgat ccaaaaaaga agagaaaggt agcccccccg      6120
   accgatgtca gcctggggga cgagctccac ttagacggcg aggacgtggc gatggcgcat      6180
   gccgacgcgc tagacgattt cgatctggac atgttggggg acggggattc cccgggtccg      6240
   ggatttaccc cccacgactc cgccccctac ggcgctctgg atatggccga cttcgagttt      6300
   gagcagatgt ttaccgatgc ccttggaatt gacgagtacg gtgggagagc cgaggggaga      6360
   ggaagtcttc tgacctgcgg agacgtcgaa gagaatcctg gacccatggt gagcaagggc      6420
   gaggagctgt tcaccggggt ggtgcccatc ctggtcgagc tggacggcga cgtaaacggc      6480
   cacaagttca gcgtgtccgg cgagggcgag ggcgatgcca cctacggcaa gctgaccctg      6540
   aagttcatct gcaccaccgg caagctgccc gtgccctggc ccaccctcgt gaccaccttc      6600
   ggctacggcc tgatgtgctt cgcccgctac cccgaccaca tgaagcagca cgacttcttc      6660
   aagtccgcca tgcccgaagg ctacgtccag gagcgcacca tcttcttcaa ggacgacggc      6720
   aactacaaga cccgcgccga ggtgaagttc gagggcgaca ccctggtgaa ccgcatcgag      6780
   ctgaagggca tcgacttcaa ggaggacggc aacatcctgg ggcacaagct ggagtacaac      6840
   tacaacagcc acaacgtcta tatcatggcc gacaagcaga agaacggcat caaggtgaac      6900
   ttcaagatcc gccacaacat cgaggacggc agcgtgcagc tcgccgacca ctaccagcag      6960
   aacaccccca tcggcgacgg ccccgtgctg ctgcccgaca accactacct gagctaccag      7020
   tccgccctga gcaaagaccc caacgagaag cgcgatcaca tggtcctgct ggagttcgtg      7080
   accgccgccg ggatcactct cggcatggac gagctgtaca agtaa                      7125
<210> 4
   <211> 3615
   <212> DNA
   <213> Unknown
<220>
   <223> Operon composed of operator DNA-binding elements, minimal or constitutive promoter, structural genes
<400> 4
<210> 5
   <211> 4224
   <212> DNA
   <213> Unknown
<220>
   <223> Operon composed of operator DNA-binding elements, minimal or constitutive promoter, structural genes
<400> 5
   tttactgctg ctcccgctta aagagcagaa actcatgctg gcagccgggg taccgcaggc      60
   aagtcgcttt actgctgctc ccgcttaaag agcagaaact caatggaatc acggcggccg     120
   cccgtatctc atcctttact gctgctcccg cttaaagagc agaaactcaa atgcgtcggc     180
   gtgcggccgc tgccagttgc atttactgct gctcccgctt aaagagcaga aactcattgg     240
   ctgtcctagg gtacctggac gctccttgtt tactgctgct cccgcttaaa gagcagaaac     300
   tcatggctgg tgccgcaccg gtaagcagaa ctagaggtgg caccggtttg attgcacagc     360
   tttactgctg ctcccgctta aagagcagaa actcatgctg gcagccgggg taccgcaggc     420
   aagtcgcttt actgctgctc ccgcttaaag agcagaaact caatggaatc acggcggccg     480
   cccgtatctc atcctttact gctgctcccg cttaaagagc agaaactcaa atgcgtcggc     540
   gtgcggccgc tgccagttgc atttactgct gctcccgctt aaagagcaga aactcattgg     600
   ctgtcctagg gtacctggac gctccttgtt tactgctgct cccgcttaaa gagcagaaac     660
   tcatggctgg tgccgcaccg gtaagcagag gatcttagag ggtatataat ggaagctcga     720
   cttccagctc gagggcaatc cggtactgtt ggtaaagcca ccgaattcgc caccatgcac     780
   caccaccacc accacgacta caaagaccat gacggtgatt ataaagatca tgacatcgat     840
   tacaaggatg acgatgacaa gatggccccc aagaagaaga ggaaggtggg cattcaccgc     900
   ggggtaccta tggtggactt gaggacactc ggttattcgc aacagcaaca ggagaaaatc     960
   aagcctaagg tcaggagcac cgtcgcgcaa caccacgagg cgcttgtggg gcatggcttc    1020
   actcatgcgc atattgtcgc gctttcacag caccctgcgg cgcttgggac ggtggctgtc    1080
   aaataccaag atatgattgc ggccctgccc gaagccacgc acgaggcaat tgtaggggtc    1140
   ggtaaacagt ggtcgggagc gcgagcactt gaggcgctgc tgactgtggc gggtgagctt    1200
   agggggcctc cgctccagct cgacaccggg cagctgctga agatcgcgaa gagaggggga    1260
   gtaacagcgg tagaggcagt gcacgcctgg cgcaatgcgc tcaccggggc ccccttgaac   1320
   ctgaccccag accaggtagt cgcaatcgcg tcaaacggag ggggaaagca agccctggaa    1380
   accgtgcaaa ggttgttgcc ggtcctttgt caagaccacg gccttacacc ggagcaagtc    1440
   gtggccattg caagcaatgg gggtggcaaa caggctcttg agacggttca gagacttctc    1500
   ccagttctct gtcaagccca cgggctgact cccgatcaag ttgtagcgat tgcgtcgaac     1560
   attggaggga aacaagcatt ggagactgtc caacggctcc ttcccgtgtt gtgtcaagcc     1620
   cacggtttga cgcctgcaca agtggtcgcc atcgccagcc atgatggcgg taagcaggcg     1680
   ctggaaacag tacagcgcct gctgcctgta ctgtgccagg atcatggact gaccccagac     1740
   caggtagtcg caatcgcgtc aaacggaggg ggaaagcaag ccctggaaac cgtgcaaagg     1800
   ttgttgccgg tcctttgtca agaccacggc cttacaccgg agcaagtcgt ggccattgca     1860
   aataataacg gtggcaaaca ggctcttgag acggttcaga gacttctccc agttctctgt     1920
   caagcccacg ggctgactcc cgatcaagtt gtagcgattg cgtcgcatga cggagggaaa     1980
   caagcattgg agactgtcca acggctcctt cccgtgttgt gtcaagccca cggtttgacg     2040
   cctgcacaag tggtcgccat cgcctcgaat ggcggcggta agcaggcgct ggaaacagta     2100
   cagcgcctgc tgcctgtact gtgccaggat catggactga ccccagacca ggtagtcgca     2160
   atcgcgaaca ataatggggg aaagcaagcc ctggaaaccg tgcaaaggtt gttgccggtc     2220
   ctttgtcaag accacggcct tacaccggag caagtcgtgg ccattgcatc ccacgacggt     2280
   ggcaaacagg ctcttgagac ggttcagaga cttctcccag ttctctgtca agcccacggg     2340
   ctgactcccg atcaagttgt agcgattgcg tccaacggtg gagggaaaca agcattggag     2400
   actgtccaac ggctccttcc cgtgttgtgt caagcccacg gtttgacgcc tgcacaagtg     2460
   gtcgccatcg ccagccatga tggcggtaag caggcgctgg aaacagtaca gcgcctgctg     2520
   cctgtactgt gccaggatca tggactgacc ccagaccagg tagtcgcaat cgcgtcacat     2580
   gacgggggaa agcaagccct ggaaaccgtg caaaggttgt tgccggtcct ttgtcaagac     2640
   cacggcctta caccggagca agtcgtggcc attgcatccc acgacggtgg caaacaggct     2700
   cttgagacgg ttcagagact tctcccagtt ctctgtcaag cccacgggct gactcccgat     2760
   caagttgtag cgattgcgaa taacaatgga gggaaacaag cattggagac tgtccaacgg     2820
   ctccttcccg tgttgtgtca agcccacggt ttgacgcctg cacaagtggt cgccatcgcc     2880
   agccatgatg gcggtaagca ggcgctggaa acagtacagc gcctgctgcc tgtactgtgc     2940
   caggatcatg gactgacacc cgaacaggtg gtcgccattg cttctaatgg gggaggacgg     3000
   ccagccttgg agtccatcgt agcccaattg tccaggcccg atcccgcgtt ggctgcgtta     3060
   acgaatgacc atctggtggc gttggcatgt cttggtggac gacccgcgct cgatgcagtc     3120
   aaaaagggtc tgcctcatgc tcccgcattg atcaaaagaa ccaaccggcg gattcccgag     3180
   agaacttccc atcgagtcgc gggatccgat ccaaaaaaga agagaaaggt agcccccccg     3240
   accgatgtca gcctggggga cgagctccac ttagacggcg aggacgtggc gatggcgcat     3300
   gccgacgcgc tagacgattt cgatctggac atgttggggg acggggattc cccgggtccg     3360
   ggatttaccc cccacgactc cgccccctac ggcgctctgg atatggccga cttcgagttt     3420
   gagcagatgt ttaccgatgc ccttggaatt gacgagtacg gtgggagagc cgaggggaga     3480
   ggaagtcttc tgacctgcgg agacgtcgaa gagaatcctg gacccatgag cgagctgatt     3540
   aaggagaaca tgcacatgaa gctgtacatg gagggcaccg tggacaacca tcacttcaag     3600
   tgcacatccg agggcgaagg caagccctac gagggcaccc agaccatgag aatcaaggtg     3660
   gtcgagggcg gccctctccc cttcgccttc gacatcctgg ctactagctt cctctacggc     3720
   agcaagacct tcatcaacca cacccagggc atccccgact tcttcaagca gtccttccct     3780
   gagggcttca catgggagag agtcaccaca tacgaagacg ggggcgtgct gaccgctacc      3840
   caggacacca gcctccagga cggctgcctc atctacaacg tcaagatcag aggggtgaac     3900
   ttcacatcca acggccctgt gatgcagaag aaaacactcg gctgggaggc cttcaccgag     3960
   acgctgtacc ccgctgacgg cggcctggaa ggcagaaacg acatggccct gaagctcgtg     4020
   ggcgggagcc atctgatcgc aaacatcaag accacatata gatccaagaa acccgctaag     4080
   aacctcaaga tgcctggcgt ctactatgtg gactacagac tggaaagaat caaggaggcc     4140
   aacaacgaga cctacgtcga gcagcacgag gtggcagtgg ccagatactg cgacctccct     4200
   agcaaactgg ggcacaagct caat                                            4224
<210> 6
   <211> 7104
   <212> DNA
   <213> Unknown
<220>
   <223> Operon composed of operator DNA-binding elements, minimal or constitutive promoter, structural genes
<400> 6
<210> 7
   <211> 3645
   <212> DNA
   <213> Unknown
<220>
   <223> Operon composed of operator DNA-binding elements, minimal or constitutive promoter, structural genes
<400> 7
   acattgatta ttgactagtt attaatagta atcaattacg gggtcattag ttcatagccc    60
   atatatggag ttccgcgtta cataacttac ggtaaatggc ccgcctggct gaccgcccaa   120
   cgacccccgc ccattgacgt caataatgac gtatgttccc atagtaacgc caatagggac   180
   tttccattga cgtcaatggg tggactattt acggtaaact gcccacttgg cagtacatca   240
   agtgtatcat atgccaagta cgccccctat tgacgtcaat gacggtaaat ggcccgcctg   300
   gcattatgcc cagtacatga ccttatggga ctttcctact tggcagtaca tctacgtatt   360
   agtcatcgct attaccatgg tgatgcggtt ttggcagtac atcaatgggc gtggatagcg   420
   gtttgactca cggggatttc caagtctcca ccccattgac gtcaatggga gtttgttttg   480
   gcaccaaaat caacgggact ttccaaaatg tcgtaacaac tccgccccat tgacgcaaat   540
   gggcggtagg cgtgtacggt gggaggtcta tataagcaga gctctctggc taactagaga   600
   acccactgct tactggctta tcgaaattaa tacgactcac tatagggaga cccaagctta   660
   tcgataggta ccctcgagtt taccactccc tatcagtgat agagaaaagt gaaagtcgag   720
   tttaccactc cctatcagtg atagagaaaa gtgaaagtcg agtttaccac tccctatcag   780
   tgatagagaa aagtgaaagt cgagtttacc actccctatc agtgatagag aaaagtgaaa   840
   gtcgagttta ccactcccta tcagtgatag agaaaagtga aagtcgagtt taccactccc   900
   tatcagtgat agagaaaagt gaaagtcgag ctcgctagcg aattcgccac catgcaccac   960
   caccaccacc acgactacaa agaccatgac ggtgattata aagatcatga catcgattac  1020
   aaggatgacg atgacaagat ggcccccaag aagaagagga aggtgggcat tcaccgcggg  1080
   gtacctatgg tggacttgag gacactcggt tattcgcaac agcaacagga gaaaatcaag  1140
   cctaaggtca ggagcaccgt cgcgcaacac cacgaggcgc ttgtggggca tggcttcact  1200
   catgcgcata ttgtcgcgct ttcacagcac cctgcggcgc ttgggacggt ggctgtcaaa  1260
   taccaagata tgattgcggc cctgcccgaa gccacgcacg aggcaattgt aggggtcggt  1320
   aaacagtggt cgggagcgcg agcacttgag gcgctgctga ctgtggcggg tgagcttagg  1380
   gggcctccgc tccagctcga caccgggcag ctgctgaaga tcgcgaagag agggggagta  1440
   acagcggtag aggcagtgca cgcctggcgc aatgcgctca ccggggcccc cttgaacctg  1500
   accccagacc aggtagtcgc aatcgcgtca catgacgggg gaaagcaagc cctggaaacc  1560
   gtgcaaaggt tgttgccggt cctttgtcaa gaccacggcc ttacaccgga gcaagtcgtg  1620
   gccattgcaa gcaatggggg tggcaaacag gctcttgaga cggttcagag acttctccca  1680
   gttctctgtc aagcccacgg gctgactccc gatcaagttg tagcgattgc gtccaacggt  1740
   ggagggaaac aagcattgga gactgtccaa cggctccttc ccgtgttgtg tcaagcccac  1800
   ggtttgacgc ctgcacaagt ggtcgccatc gccagccatg atggcggtaa gcaggcgctg  1860
   gaaacagtac agcgcctgct gcctgtactg tgccaggatc atggactgac cccagaccag    1920
   gtagtcgcaa tcgcgtcaca tgacggggga aagcaagccc tggaaaccgt gcaaaggttg    1980
   ttgccggtcc tttgtcaaga ccacggcctt acaccggagc aagtcgtggc cattgcaaat  2040
   aataacggtg gcaaacaggc tcttgagacg gttcagagac ttctcccagt tctctgtcaa  2100
   gcccacgggc tgactcccga tcaagttgta gcgattgcgt ccaacggtgg agggaaacaa  2160
   gcattggaga ctgtccaacg gctccttccc gtgttgtgtc aagcccacgg tttgacgcct  2220
   gcacaagtgg tcgccatcgc ctcgaatggc ggcggtaagc aggcgctgga aacagtacag  2280
   cgcctgctgc ctgtactgtg ccaggatcat ggactgaccc cagaccaggt agtcgcaatc  2340
   gcgtcaaacg gagggggaaa gcaagccctg gaaaccgtgc aaaggttgtt gccggtcctt  2400
   tgtcaagacc acggccttac accggagcaa gtcgtggcca ttgcatccca cgacggtggc  2460
   aaacaggctc ttgagacggt tcagagactt ctcccagttc tctgtcaagc ccacgggctg  2520
   actcccgatc aagttgtagc gattgcgtcg catgacggag ggaaacaagc attggagact  2580
   gtccaacggc tccttcccgt gttgtgtcaa gcccacggtt tgacgcctgc acaagtggtc  2640
   gccatcgcct ccaatattgg cggtaagcag gcgctggaaa cagtacagcg cctgctgcct  2700
   gtactgtgcc aggatcatgg actgacccca gaccaggtag tcgcaatcgc gtcacatgac  2760
   gggggaaagc aagccctgga aaccgtgcaa aggttgttgc cggtcctttg tcaagaccac  2820
   ggccttacac cggagcaagt cgtggccatt gcaagcaaca tcggtggcaa acaggctctt  2880
   gagacggttc agagacttct cccagttctc tgtcaagccc acgggctgac tcccgatcaa  2940
   gttgtagcga ttgcgtccaa cggtggaggg aaacaagcat tggagactgt ccaacggctc  3000
   cttcccgtgt tgtgtcaagc ccacggtttg acgcctgcac aagtggtcgc catcgccagc  3060
   catgatggcg gtaagcaggc gctggaaaca gtacagcgcc tgctgcctgt actgtgccag  3120
   gatcatggac tgacacccga acaggtggtc gccattgctt ctaatggggg aggacggcca  3180
   gccttggagt ccatcgtagc ccaattgtcc aggcccgatc ccgcgttggc tgcgttaacg  3240
   aatgaccatc tggtggcgtt ggcatgtctt ggtggacgac ccgcgctcga tgcagtcaaa  3300
   aagggtctgc ctcatgctcc cgcattgatc aaaagaacca accggcggat tcccgagaga  3360
   acttcccatc gagtcgcggg atccgatcca aaaaagaaga gaaaggtagc acccccgacc  3420
   gatgtcagcc tgggggacga gctccactta gacggcgagg acgtggcgat ggcgcatgcc  3480
   gacgcgctag acgatttcga tctggacatg ttgggggacg gggattcccc gggtccggga  3540
   tttacccccc acgactccgc cccctacggc gctctggata tggccgactt cgagtttgag  3600
   cagatgttta ccgatgccct tggaattgac gagtacggtg ggtag                  3645
<210> 8
   <211> 3792
   <212> DNA
   <213> Unknown
<220>
   <223> Operon composed of operator DNA-binding elements, minimal or constitutive promoter, structural genes
<400> 8
   acattgatta ttgactagtt attaatagta atcaattacg gggtcattag ttcatagccc      60
   atatatggag ttccgcgtta cataacttac ggtaaatggc ccgcctggct gaccgcccaa     120
   cgacccccgc ccattgacgt caataatgac gtatgttccc atagtaacgc caatagggac     180
   tttccattga cgtcaatggg tggactattt acggtaaact gcccacttgg cagtacatca     240
   agtgtatcat atgccaagta cgccccctat tgacgtcaat gacggtaaat ggcccgcctg     300
   gcattatgcc cagtacatga ccttatggga ctttcctact tggcagtaca tctacgtatt     360
   agtcatcgct attaccatgg tgatgcggtt ttggcagtac atcaatgggc gtggatagcg     420
   gtttgactca cggggatttc caagtctcca ccccattgac gtcaatggga gtttgttttg     480
   gcaccaaaat caacgggact ttccaaaatg tcgtaacaac tccgccccat tgacgcaaat     540
   gggcggtagg cgtgtacggt gggaggtcta tataagcaga gctctctggc taactagaga     600
   acccactgct tactggctta tcgaaattaa tacgactcac tatagggaga cccaagctta     660
   tcgataggta ccctcgagtt taccactccc tatcagtgat agagaaaagt gaaagtcgag     720
   tttaccactc cctatcagtg atagagaaaa gtgaaagtcg agtttaccac tccctatcag     780
   tgatagagaa aagtgaaagt cgagtttacc actccctatc agtgatagag aaaagtgaaa     840
   gtcgagttta ccactcccta tcagtgatag agaaaagtga aagtcgagtt taccactccc     900
   tatcagtgat agagaaaagt gaaagtcgag ctcgctagcg aattcgccac catgcaccac     960
   caccaccacc acgactacaa agaccatgac ggtgattata aagatcatga catcgattac      1020
   aaggatgacg atgacaagat ggcccccaag aagaagagga aggtgggcat tcaccgcggg      1080
   gtacctatgg tggacttgag gacactcggt tattcgcaac agcaacagga gaaaatcaag      1140
   cctaaggtca ggagcaccgt cgcgcaacac cacgaggcgc ttgtggggca tggcttcact      1200
   catgcgcata ttgtcgcgct ttcacagcac cctgcggcgc ttgggacggt ggctgtcaaa      1260
   taccaagata tgattgcggc cctgcccgaa gccacgcacg aggcaattgt aggggtcggt      1320
   aaacagtggt cgggagcgcg agcacttgag gcgctgctga ctgtggcggg tgagcttagg      1380
   gggcctccgc tccagctcga caccgggcag ctgctgaaga tcgcgaagag agggggagta      1440
   acagcggtag aggcagtgca cgcctggcgc aatgcgctca ccggggcccc cttgaacctg      1500
   accccagacc aggtagtcgc aatcgcgtca aacggagggg gaaagcaagc cctggaaacc      1560
   gtgcaaaggt tgttgccggt cctttgtcaa gaccacggcc ttacaccgga gcaagtcgtg      1620
   gccattgcaa gcaatggggg tggcaaacag gctcttgaga cggttcagag acttctccca      1680
   gttctctgtc aagcccacgg gctgactccc gatcaagttg tagcgattgc gtcgaacatt      1740
   ggagggaaac aagcattgga gactgtccaa cggctccttc ccgtgttgtg tcaagcccac      1800
   ggtttgacgc ctgcacaagt ggtcgccatc gccagccatg atggcggtaa gcaggcgctg      1860
   gaaacagtac agcgcctgct gcctgtactg tgccaggatc atggactgac cccagaccag      1920
   gtagtcgcaa tcgcgtcaaa cggaggggga aagcaagccc tggaaaccgt gcaaaggttg      1980
   ttgccggtcc tttgtcaaga ccacggcctt acaccggagc aagtcgtggc cattgcaaat      2040
   aataacggtg gcaaacaggc tcttgagacg gttcagagac ttctcccagt tctctgtcaa      2100
   gcccacgggc tgactcccga tcaagttgta gcgattgcgt cgcatgacgg agggaaacaa      2160
   gcattggaga ctgtccaacg gctccttccc gtgttgtgtc aagcccacgg tttgacgcct      2220
   gcacaagtgg tcgccatcgc ctcgaatggc ggcggtaagc aggcgctgga aacagtacag      2280
   cgcctgctgc ctgtactgtg ccaggatcat ggactgaccc cagaccaggt agtcgcaatc      2340
   gcgaacaata atgggggaaa gcaagccctg gaaaccgtgc aaaggttgtt gccggtcctt      2400
   tgtcaagacc acggccttac accggagcaa gtcgtggcca ttgcatccca cgacggtggc      2460
   aaacaggctc ttgagacggt tcagagactt ctcccagttc tctgtcaagc ccacgggctg      2520
   actcccgatc aagttgtagc gattgcgtcc aacggtggag ggaaacaagc attggagact      2580
   gtccaacggc tccttcccgt gttgtgtcaa gcccacggtt tgacgcctgc acaagtggtc      2640
   gccatcgcca gccatgatgg cggtaagcag gcgctggaaa cagtacagcg cctgctgcct      2700
   gtactgtgcc aggatcatgg actgacccca gaccaggtag tcgcaatcgc gtcacatgac      2760
   gggggaaagc aagccctgga aaccgtgcaa aggttgttgc cggtcctttg tcaagaccac      2820
   ggccttacac cggagcaagt cgtggccatt gcatcccacg acggtggcaa acaggctctt      2880
   gagacggttc agagacttct cccagttctc tgtcaagccc acgggctgac tcccgatcaa      2940
   gttgtagcga ttgcgaataa caatggaggg aaacaagcat tggagactgt ccaacggctc      3000
   cttcccgtgt tgtgtcaagc ccacggtttg acgcctgcac aagtggtcgc catcgccagc      3060
   catgatggcg gtaagcaggc gctggaaaca gtacagcgcc tgctgcctgt actgtgccag      3120
   gatcatggac tgacacccga acaggtggtc gccattgctt ctaatggggg aggacggcca      3180
   gccttggagt ccatcgtagc ccaattgtcc aggcccgatc ccgcgttggc tgcgttaacg      3240
   aatgaccatc tggtggcgtt ggcatgtctt ggtggacgac ccgcgctcga tgcagtcaaa      3300
   aagggtctgc ctcatgctcc cgcattgatc aaaagaacca accggcggat tcccgagaga      3360
   acttcccatc gagtcgcggg atccgatcca aaaaagaaga gaaaggtacc aaaaaagaag      3420
   agaaaggtcg acggcggtgg tgctttgtct cctcagcact ctgctgtcac tcaaggaagt      3480
   atcatcaaga acaaggaggg catggatgct aagtcactaa ctgcctggtc ccggacactg      3540
   gtgaccttca aggatgtatt tgtggacttc accagggagg agtggaagct gctggacact      3600
   gctcagcaga tcgtgtacag aaatgtgatg ctggagaact ataagaacct ggtttccttg      3660
   ggttatcagc ttactaagcc agatgtgatc ctccggttgg agaagggaga agagccctgg      3720
   ctggtggaga gagaaattca ccaagagacc catcctgatt cagagactgc atttgaaatc      3780
   aaatcatcag tt                                                          3792
<210> 9
   <211> 3170
   <212> DNA
   <213> Unknown
<220>
   <223> Synthetic construct/combination of sequences
<400> 9
   gatctcggcc gcatattaag tgcattgttc tcgataccgc taagtgcatt gttctcgtta      60
   gctcgatgga caagtgcatt gttctcttgc tgaaagctcg atggacaagt gcattgttct     120
   cttgctgaaa gctcgatgga caagtgcatt gttctcttgc tgaaagctca gtacccgggt     180
   cggagtactg ccccgcccct agcgattagc cccggccccg catagctccg ccccgggagt     240
   accttcgacc tagagggtat ataatggaag ctcgacttcc agctcgaggg caatccggta     300
   ctgttggtaa agccaccgaa ttcgccacca tgcaccacca ccaccaccac gactacaaag     360
   accatgacgg tgattataaa gatcatgaca tcgattacaa ggatgacgat gacaagatgg     420
   cccccaagaa gaagaggaag gtgggcattc accgcggggt acctatggtg gacttgagga     480
   cactcggtta ttcgcaacag caacaggaga aaatcaagcc taaggtcagg agcaccgtcg     540
   cgcaacacca cgaggcgctt gtggggcatg gcttcactca tgcgcatatt gtcgcgcttt     600
   cacagcaccc tgcggcgctt gggacggtgg ctgtcaaata ccaagatatg attgcggccc     660
   tgcccgaagc cacgcacgag gcaattgtag gggtcggtaa acagtggtcg ggagcgcgag     720
   cacttgaggc gctgctgact gtggcgggtg agcttagggg gcctccgctc cagctcgaca     780
   ccgggcagct gctgaagatc gcgaagagag ggggagtaac agcggtagag gcagtgcacg     840
   cctggcgcaa tgcgctcacc ggggccccct tgaacctgac cccagaccag gtagtcgcaa     900
   tcgcgtcaca tgacggggga aagcaagccc tggaaaccgt gcaaaggttg ttgccggtcc     960
   tttgtcaaga ccacggcctt acaccggagc aagtcgtggc cattgcaagc aatgggggtg    1020
   gcaaacaggc tcttgagacg gttcagagac ttctcccagt tctctgtcaa gcccacgggc    1080
   tgactcccga tcaagttgta gcgattgcgt ccaacggtgg agggaaacaa gcattggaga    1140
   ctgtccaacg gctccttccc gtgttgtgtc aagcccacgg tttgacgcct gcacaagtgg    1200
   tcgccatcgc cagccatgat ggcggtaagc aggcgctgga aacagtacag cgcctgctgc    1260
   ctgtactgtg ccaggatcat ggactgaccc cagaccaggt agtcgcaatc gcgtcacatg    1320
   acgggggaaa gcaagccctg gaaaccgtgc aaaggttgtt gccggtcctt tgtcaagacc    1380
   acggccttac accggagcaa gtcgtggcca ttgcaaataa taacggtggc aaacaggctc    1440
   ttgagacggt tcagagactt ctcccagttc tctgtcaagc ccacgggctg actcccgatc    1500
   aagttgtagc gattgcgtcc aacggtggag ggaaacaagc attggagact gtccaacggc    1560
   tccttcccgt gttgtgtcaa gcccacggtt tgacgcctgc acaagtggtc gccatcgcct    1620
   cgaatggcgg cggtaagcag gcgctggaaa cagtacagcg cctgctgcct gtactgtgcc    1680
   aggatcatgg actgacccca gaccaggtag tcgcaatcgc gtcaaacgga gggggaaagc    1740
   aagccctgga aaccgtgcaa aggttgttgc cggtcctttg tcaagaccac ggccttacac    1800
   cggagcaagt cgtggccatt gcatcccacg acggtggcaa acaggctctt gagacggttc    1860
   agagacttct cccagttctc tgtcaagccc acgggctgac tcccgatcaa gttgtagcga    1920
   ttgcgtcgca tgacggaggg aaacaagcat tggagactgt ccaacggctc cttcccgtgt    1980
   tgtgtcaagc ccacggtttg acgcctgcac aagtggtcgc catcgcctcc aatattggcg    2040
   gtaagcaggc gctggaaaca gtacagcgcc tgctgcctgt actgtgccag gatcatggac    2100
   tgaccccaga ccaggtagtc gcaatcgcgt cacatgacgg gggaaagcaa gccctggaaa    2160
   ccgtgcaaag gttgttgccg gtcctttgtc aagaccacgg ccttacaccg gagcaagtcg    2220
   tggccattgc aagcaacatc ggtggcaaac aggctcttga gacggttcag agacttctcc    2280
   cagttctctg tcaagcccac gggctgactc ccgatcaagt tgtagcgatt gcgtccaacg    2340
   gtggagggaa acaagcattg gagactgtcc aacggctcct tcccgtgttg tgtcaagccc    2400
   acggtttgac gcctgcacaa gtggtcgcca tcgccagcca tgatggcggt aagcaggcgc    2460
   tggaaacagt acagcgcctg ctgcctgtac tgtgccagga tcatggactg acacccgaac    2520
   aggtggtcgc cattgcttct aatgggggag gacggccagc cttggagtcc atcgtagccc    2580
   aattgtccag gcccgatccc gcgttggctg cgttaacgaa tgaccatctg gtggcgttgg    2640
   catgtcttgg tggacgaccc gcgctcgatg cagtcaaaaa gggtctgcct catgctcccg    2700
   cattgatcaa aagaaccaac cggcggattc ccgagagaac ttcccatcga gtcgcgggat    2760
   ccgatccaaa aaagaagaga aaggtaccaa aaaagaagag aaaggtcgac ggcggtggtg    2820
   ctttgtctcc tcagcactct gctgtcactc aaggaagtat catcaagaac aaggagggca    2880
   tggatgctaa gtcactaact gcctggtccc ggacactggt gaccttcaag gatgtatttg    2940
   tggacttcac cagggaggag tggaagctgc tggacactgc tcagcagatc gtgtacagaa    3000
   atgtgatgct ggagaactat aagaacctgg tttccttggg ttatcagctt actaagccag    3060
   atgtgatcct ccggttggag aagggagaag agccctggct ggtggagaga gaaattcacc    3120
   aagagaccca tcctgattca gagactgcat ttgaaatcaa atcatcagtt                3170
<210> 10
   <211> 3020
   <212> DNA
   <213> Unknown
<220>
   <223> Operon composed of operator DNA-binding elements, minimal or constitutive promoter, structural genes
<400> 10
   gatctcggcc gcatattaag tgcattgttc tcgataccgc taagtgcatt gttctcgtta      60
   gctcgatgga caagtgcatt gttctcttgc tgaaagctcg atggacaagt gcattgttct     120
   cttgctgaaa gctcgatgga caagtgcatt gttctcttgc tgaaagctca gtacccgggt     180
   cggagtactg ccccgcccct agcgattagc cccggccccg catagctccg ccccgggagt     240
   accttcgacc tagagggtat ataatggaag ctcgacttcc agctcgaggg caatccggta     300
   ctgttggtaa agccaccgaa ttcgccacca tgcaccacca ccaccaccac gactacaaag     360
   accatgacgg tgattataaa gatcatgaca tcgattacaa ggatgacgat gacaagatgg     420
   cccccaagaa gaagaggaag gtgggcattc accgcggggt acctatggtg gacttgagga     480
   cactcggtta ttcgcaacag caacaggaga aaatcaagcc taaggtcagg agcaccgtcg     540
   cgcaacacca cgaggcgctt gtggggcatg gcttcactca tgcgcatatt gtcgcgcttt     600
   cacagcaccc tgcggcgctt gggacggtgg ctgtcaaata ccaagatatg attgcggccc     660
   tgcccgaagc cacgcacgag gcaattgtag gggtcggtaa acagtggtcg ggagcgcgag     720
   cacttgaggc gctgctgact gtggcgggtg agcttagggg gcctccgctc cagctcgaca     780
   ccgggcagct gctgaagatc gcgaagagag ggggagtaac agcggtagag gcagtgcacg     840
   cctggcgcaa tgcgctcacc ggggccccct tgaacctgac cccagaccag gtagtcgcaa     900
   tcgcgtcaaa cggaggggga aagcaagccc tggaaaccgt gcaaaggttg ttgccggtcc     960
   tttgtcaaga ccacggcctt acaccggagc aagtcgtggc cattgcaagc aatgggggtg    1020
   gcaaacaggc tcttgagacg gttcagagac ttctcccagt tctctgtcaa gcccacgggc    1080
   tgactcccga tcaagttgta gcgattgcgt cgaacattgg agggaaacaa gcattggaga    1140
   ctgtccaacg gctccttccc gtgttgtgtc aagcccacgg tttgacgcct gcacaagtgg    1200
   tcgccatcgc cagccatgat ggcggtaagc aggcgctgga aacagtacag cgcctgctgc    1260
   ctgtactgtg ccaggatcat ggactgaccc cagaccaggt agtcgcaatc gcgtcaaacg    1320
   gagggggaaa gcaagccctg gaaaccgtgc aaaggttgtt gccggtcctt tgtcaagacc    1380
   acggccttac accggagcaa gtcgtggcca ttgcaaataa taacggtggc aaacaggctc    1440
   ttgagacggt tcagagactt ctcccagttc tctgtcaagc ccacgggctg actcccgatc    1500
   aagttgtagc gattgcgtcg catgacggag ggaaacaagc attggagact gtccaacggc    1560
   tccttcccgt gttgtgtcaa gcccacggtt tgacgcctgc acaagtggtc gccatcgcct    1620
   cgaatggcgg cggtaagcag gcgctggaaa cagtacagcg cctgctgcct gtactgtgcc    1680
   aggatcatgg actgacccca gaccaggtag tcgcaatcgc gaacaataat gggggaaagc    1740
   aagccctgga aaccgtgcaa aggttgttgc cggtcctttg tcaagaccac ggccttacac    1800
   cggagcaagt cgtggccatt gcatcccacg acggtggcaa acaggctctt gagacggttc    1860
   agagacttct cccagttctc tgtcaagccc acgggctgac tcccgatcaa gttgtagcga    1920
   ttgcgtccaa cggtggaggg aaacaagcat tggagactgt ccaacggctc cttcccgtgt    1980
   tgtgtcaagc ccacggtttg acgcctgcac aagtggtcgc catcgccagc catgatggcg    2040
   gtaagcaggc gctggaaaca gtacagcgcc tgctgcctgt actgtgccag gatcatggac    2100
   tgaccccaga ccaggtagtc gcaatcgcgt cacatgacgg gggaaagcaa gccctggaaa    2160
   ccgtgcaaag gttgttgccg gtcctttgtc aagaccacgg ccttacaccg gagcaagtcg    2220
   tggccattgc atcccacgac ggtggcaaac aggctcttga gacggttcag agacttctcc    2280
   cagttctctg tcaagcccac gggctgactc ccgatcaagt tgtagcgatt gcgaataaca    2340
   atggagggaa acaagcattg gagactgtcc aacggctcct tcccgtgttg tgtcaagccc    2400
   acggtttgac gcctgcacaa gtggtcgcca tcgccagcca tgatggcggt aagcaggcgc    2460
   tggaaacagt acagcgcctg ctgcctgtac tgtgccagga tcatggactg acacccgaac    2520
   aggtggtcgc cattgcttct aatgggggag gacggccagc cttggagtcc atcgtagccc    2580
   aattgtccag gcccgatccc gcgttggctg cgttaacgaa tgaccatctg gtggcgttgg    2640
   catgtcttgg tggacgaccc gcgctcgatg cagtcaaaaa gggtctgcct catgctcccg    2700
   cattgatcaa aagaaccaac cggcggattc ccgagagaac ttcccatcga gtcgcgggat    2760
   ccgatccaaa aaagaagaga aaggtagccc ccccgaccga tgtcagcctg ggggacgagc     2820
   tccacttaga cggcgaggac gtggcgatgg cgcatgccga cgcgctagac gatttcgatc    2880
   tggacatgtt gggggacggg gattccccgg gtccgggatt taccccccac gactccgccc    2940
   cctacggcgc tctggatatg gccgacttcg agtttgagca gatgtttacc gatgcccttg    3000
   gaattgacga gtacggtggg                                                3020
<210> 11
   <211> 2670
   <212> PRT
   <213> Unknown
<220>
   <223> Synthetic genetic construct
<400> 11
   Gly Ala Cys Thr Ala Cys Ala Ala Ala Gly Ala Cys Cys Ala Thr Gly
   1               5                   10                  15
   Ala Cys Gly Gly Thr Gly Ala Thr Thr Ala Thr Ala Ala Ala Gly Ala
               20                  25                  30
   Thr Cys Ala Thr Gly Ala Cys Ala Thr Cys Gly Ala Thr Thr Ala Cys
           35                  40                  45
   Ala Ala Gly Gly Ala Thr Gly Ala Cys Gly Ala Thr Gly Ala Cys Ala
       50                  55                  60
   Ala Gly Ala Thr Gly Gly Cys Cys Cys Cys Cys Ala Ala Gly Ala Ala
   65                  70                  75                  80
   Gly Ala Ala Gly Ala Gly Gly Ala Ala Gly Gly Thr Gly Gly Gly Cys
                   85                  90                  95
   Ala Thr Thr Cys Ala Cys Cys Gly Cys Gly Gly Gly Gly Thr Ala Cys
               100                 105                 110
   Cys Thr Ala Thr Gly Gly Thr Gly Gly Ala Cys Thr Thr Gly Ala Gly
           115                 120                 125
   Gly Ala Cys Ala Cys Thr Cys Gly Gly Thr Thr Ala Thr Thr Cys Gly
       130                 135                 140
   Cys Ala Ala Cys Ala Gly Cys Ala Ala Cys Ala Gly Gly Ala Gly Ala
   145                 150                 155                 160
   Ala Ala Ala Thr Cys Ala Ala Gly Cys Cys Thr Ala Ala Gly Gly Thr
                   165                 170                 175
   Cys Ala Gly Gly Ala Gly Cys Ala Cys Cys Gly Thr Cys Gly Cys Gly
               180                 185                 190
   Cys Ala Ala Cys Ala Cys Cys Ala Cys Gly Ala Gly Gly Cys Gly Cys
           195                 200                 205
   Thr Thr Gly Thr Gly Gly Gly Gly Cys Ala Thr Gly Gly Cys Thr Thr
       210                 215                 220
   Cys Ala Cys Thr Cys Ala Thr Gly Cys Gly Cys Ala Thr Ala Thr Thr
   225                 230                 235                 240
   Gly Thr Cys Gly Cys Gly Cys Thr Thr Thr Cys Ala Cys Ala Gly Cys
                   245                 250                 255
   Ala Cys Cys Cys Thr Gly Cys Gly Gly Cys Gly Cys Thr Thr Gly Gly
               260                 265                 270
   Gly Ala Cys Gly Gly Thr Gly Gly Cys Thr Gly Thr Cys Ala Ala Ala
           275                 280                 285
   Thr Ala Cys Cys Ala Ala Gly Ala Thr Ala Thr Gly Ala Thr Thr Gly
       290                 295                 300
   Cys Gly Gly Cys Cys Cys Thr Gly Cys Cys Cys Gly Ala Ala Gly Cys
   305                 310                 315                 320
   Cys Ala Cys Gly Cys Ala Cys Gly Ala Gly Gly Cys Ala Ala Thr Thr
                   325                 330                 335
   Gly Thr Ala Gly Gly Gly Gly Thr Cys Gly Gly Thr Ala Ala Ala Cys
               340                 345                 350
   Ala Gly Thr Gly Gly Thr Cys Gly Gly Gly Ala Gly Cys Gly Cys Gly
           355                 360                 365
   Ala Gly Cys Ala Cys Thr Thr Gly Ala Gly Gly Cys Gly Cys Thr Gly
       370                 375                 380
   Cys Thr Gly Ala Cys Thr Gly Thr Gly Gly Cys Gly Gly Gly Thr Gly
   385                 390                 395                 400
   Ala Gly Cys Thr Thr Ala Gly Gly Gly Gly Gly Cys Cys Thr Cys Cys
                   405                 410                 415
   Gly Cys Thr Cys Cys Ala Gly Cys Thr Cys Gly Ala Cys Ala Cys Cys
               420                 425                 430
   Gly Gly Gly Cys Ala Gly Cys Thr Gly Cys Thr Gly Ala Ala Gly Ala
           435                 440                 445
   Thr Cys Gly Cys Gly Ala Ala Gly Ala Gly Ala Gly Gly Gly Gly Gly
       450                 455                 460
   Ala Gly Thr Ala Ala Cys Ala Gly Cys Gly Gly Thr Ala Gly Ala Gly
   465                 470                 475                 480
   Gly Cys Ala Gly Thr Gly Cys Ala Cys Gly Cys Cys Thr Gly Gly Cys
                   485                 490                 495
   Gly Cys Ala Ala Thr Gly Cys Gly Cys Thr Cys Ala Cys Cys Gly Gly
               500                 505                 510
   Gly Gly Cys Cys Cys Cys Cys Thr Thr Gly Ala Ala Cys Cys Thr Gly
           515                 520                 525
   Ala Cys Cys Cys Cys Ala Gly Ala Cys Cys Ala Gly Gly Thr Ala Gly
       530                 535                 540
   Thr Cys Gly Cys Ala Ala Thr Cys Gly Cys Gly Thr Cys Ala Ala Ala
   545                 550                 555                 560
   Cys Gly Gly Ala Gly Gly Gly Gly Gly Ala Ala Ala Gly Cys Ala Ala
                   565                 570                 575
   Gly Cys Cys Cys Thr Gly Gly Ala Ala Ala Cys Cys Gly Thr Gly Cys
               580                 585                 590
   Ala Ala Ala Gly Gly Thr Thr Gly Thr Thr Gly Cys Cys Gly Gly Thr
           595                 600                 605
   Cys Cys Thr Thr Thr Gly Thr Cys Ala Ala Gly Ala Cys Cys Ala Cys
       610                 615                 620
   Gly Gly Cys Cys Thr Thr Ala Cys Ala Cys Cys Gly Gly Ala Gly Cys
   625                 630                 635                 640
   Ala Ala Gly Thr Cys Gly Thr Gly Gly Cys Cys Ala Thr Thr Gly Cys
                   645                 650                 655
   Ala Ala Gly Cys Ala Ala Thr Gly Gly Gly Gly Gly Thr Gly Gly Cys
               660                 665                 670
   Ala Ala Ala Cys Ala Gly Gly Cys Thr Cys Thr Thr Gly Ala Gly Ala
           675                 680                 685
   Cys Gly Gly Thr Thr Cys Ala Gly Ala Gly Ala Cys Thr Thr Cys Thr
       690                 695                 700
   Cys Cys Cys Ala Gly Thr Thr Cys Thr Cys Thr Gly Thr Cys Ala Ala
   705                 710                 715                 720
   Gly Cys Cys Cys Ala Cys Gly Gly Gly Cys Thr Gly Ala Cys Thr Cys
                   725                 730                 735
   Cys Cys Gly Ala Thr Cys Ala Ala Gly Thr Thr Gly Thr Ala Gly Cys
   740 745 750
   Gly Ala Thr Thr Gly Cys Gly Thr Cys Gly Ala Ala Cys Ala Thr Thr
   755 760 765
   Gly Gly Ala Gly Gly Gly Ala Ala Ala Cys Ala Ala Gly Cys Ala Thr
   770 775 780
   Thr Gly Gly Ala Gly Ala Cys Thr Gly Thr Cys Cys Ala Ala Cys Gly
   785 790 795 800
   Gly Cys Thr Cys Cys Thr Thr Cys Cys Cys Gly Thr Gly Thr Thr Gly
   805 810 815
   Thr Gly Thr Cys Ala Ala Gly Cys Cys Cys Ala Cys Gly Gly Thr Thr
   820 825 830
   Thr Gly Ala Cys Gly Cys Cys Thr Gly Cys Ala Cys Ala Ala Gly Thr
   835 840 845
   Gly Gly Thr Cys Gly Cys Cys Ala Thr Cys Gly Cys Cys Ala Gly Cys
   850 855 860
   Cys Ala Thr Gly Ala Thr Gly Gly Cys Gly Gly Thr Ala Ala Gly Cys
   865 870 875 880
   Ala Gly Gly Cys Gly Cys Thr Gly Gly Ala Ala Ala Cys Ala Gly Thr
   885 890 895
   Ala Cys Ala Gly Cys Gly Cys Cys Thr Gly Cys Thr Gly Cys Cys Thr
   900 905 910
   Gly Thr Ala Cys Thr Gly Thr Gly Cys Cys Ala Gly Gly Ala Thr Cys
   915 920 925
   Ala Thr Gly Gly Ala Cys Thr Gly Ala Cys Cys Cys Cys Ala Gly Ala
   930 935 940
   Cys Cys Ala Gly Gly Thr Ala Gly Thr Cys Gly Cys Ala Ala Thr Cys
   945 950 955 960
   Gly Cys Gly Thr Cys Ala Ala Ala Cys Gly Gly Ala Gly Gly Gly Gly
   965 970 975
   Gly Ala Ala Ala Gly Cys Ala Ala Gly Cys Cys Cys Thr Gly Gly Ala
   980 985 990
   Ala Ala Cys Cys Gly Thr Gly Cys Ala Ala Ala Gly Gly Thr Thr Gly
   995 1000 1005
   Thr Thr Gly Cys Cys Gly Gly Thr Cys Cys Thr Thr Thr Gly Thr
   1010 1015 1020
   Cys Ala Ala Gly Ala Cys Cys Ala Cys Gly Gly Cys Cys Thr Thr
   1025 1030 1035
   Ala Cys Ala Cys Cys Gly Gly Ala Gly Cys Ala Ala Gly Thr Cys
   1040 1045 1050
   Gly Thr Gly Gly Cys Cys Ala Thr Thr Gly Cys Ala Ala Ala Thr
   1055 1060 1065
   Ala Ala Thr Ala Ala Cys Gly Gly Thr Gly Gly Cys Ala Ala Ala
   1070 1075 1080
   Cys Ala Gly Gly Cys Thr Cys Thr Thr Gly Ala Gly Ala Cys Gly
   1085 1090 1095
   Gly Thr Thr Cys Ala Gly Ala Gly Ala Cys Thr Thr Cys Thr Cys
   1100 1105 1110
   Cys Cys Ala Gly Thr Thr Cys Thr Cys Thr Gly Thr Cys Ala Ala
   1115 1120 1125
   Gly Cys Cys Cys Ala Cys Gly Gly Gly Cys Thr Gly Ala Cys Thr
   1130 1135 1140
   Cys Cys Cys Gly Ala Thr Cys Ala Ala Gly Thr Thr Gly Thr Ala
   1145 1150 1155
   Gly Cys Gly Ala Thr Thr Gly Cys Gly Thr Cys Gly Cys Ala Thr
   1160 1165 1170
   Gly Ala Cys Gly Gly Ala Gly Gly Gly Ala Ala Ala Cys Ala Ala
   1175 1180 1185
   Gly Cys Ala Thr Thr Gly Gly Ala Gly Ala Cys Thr Gly Thr Cys
   1190 1195 1200
   Cys Ala Ala Cys Gly Gly Cys Thr Cys Cys Thr Thr Cys Cys Cys
   1205 1210 1215
   Gly Thr Gly Thr Thr Gly Thr Gly Thr Cys Ala Ala Gly Cys Cys
   1220 1225 1230
   Cys Ala Cys Gly Gly Thr Thr Thr Gly Ala Cys Gly Cys Cys Thr
   1235 1240 1245
   Gly Cys Ala Cys Ala Ala Gly Thr Gly Gly Thr Cys Gly Cys Cys
   1250 1255 1260
   Ala Thr Cys Gly Cys Cys Thr Cys Gly Ala Ala Thr Gly Gly Cys
   1265 1270 1275
   Gly Gly Cys Gly Gly Thr Ala Ala Gly Cys Ala Gly Gly Cys Gly
   1280 1285 1290
   Cys Thr Gly Gly Ala Ala Ala Cys Ala Gly Thr Ala Cys Ala Gly
   1295 1300 1305
   Cys Gly Cys Cys Thr Gly Cys Thr Gly Cys Cys Thr Gly Thr Ala
   1310 1315 1320
   Cys Thr Gly Thr Gly Cys Cys Ala Gly Gly Ala Thr Cys Ala Thr
   1325 1330 1335
   Gly Gly Ala Cys Thr Gly Ala Cys Cys Cys Cys Ala Gly Ala Cys
   1340 1345 1350
   Cys Ala Gly Gly Thr Ala Gly Thr Cys Gly Cys Ala Ala Thr Cys
   1355 1360 1365
   Gly Cys Gly Ala Ala Cys Ala Ala Thr Ala Ala Thr Gly Gly Gly
   1370 1375 1380
   Gly Gly Ala Ala Ala Gly Cys Ala Ala Gly Cys Cys Cys Thr Gly
   1385 1390 1395
   Gly Ala Ala Ala Cys Cys Gly Thr Gly Cys Ala Ala Ala Gly Gly
   1400 1405 1410
   Thr Thr Gly Thr Thr Gly Cys Cys Gly Gly Thr Cys Cys Thr Thr
   1415 1420 1425
   Thr Gly Thr Cys Ala Ala Gly Ala Cys Cys Ala Cys Gly Gly Cys
   1430 1435 1440
   Cys Thr Thr Ala Cys Ala Cys Cys Gly Gly Ala Gly Cys Ala Ala
   1445 1450 1455
   Gly Thr Cys Gly Thr Gly Gly Cys Cys Ala Thr Thr Gly Cys Ala
   1460 1465 1470
   Thr Cys Cys Cys Ala Cys Gly Ala Cys Gly Gly Thr Gly Gly Cys
   1475 1480 1485
   Ala Ala Ala Cys Ala Gly Gly Cys Thr Cys Thr Thr Gly Ala Gly
   1490 1495 1500
   Ala Cys Gly Gly Thr Thr Cys Ala Gly Ala Gly Ala Cys Thr Thr
   1505 1510 1515
   Cys Thr Cys Cys Cys Ala Gly Thr Thr Cys Thr Cys Thr Gly Thr
   1520 1525 1530
   Cys Ala Ala Gly Cys Cys Cys Ala Cys Gly Gly Gly Cys Thr Gly
   1535 1540 1545
   Ala Cys Thr Cys Cys Cys Gly Ala Thr Cys Ala Ala Gly Thr Thr
   1550 1555 1560
   Gly Thr Ala Gly Cys Gly Ala Thr Thr Gly Cys Gly Thr Cys Cys
   1565 1570 1575
   Ala Ala Cys Gly Gly Thr Gly Gly Ala Gly Gly Gly Ala Ala Ala
   1580 1585 1590
   Cys Ala Ala Gly Cys Ala Thr Thr Gly Gly Ala Gly Ala Cys Thr
   1595 1600 1605
   Gly Thr Cys Cys Ala Ala Cys Gly Gly Cys Thr Cys Cys Thr Thr
   1610 1615 1620
   Cys Cys Cys Gly Thr Gly Thr Thr Gly Thr Gly Thr Cys Ala Ala
   1625 1630 1635
   Gly Cys Cys Cys Ala Cys Gly Gly Thr Thr Thr Gly Ala Cys Gly
   1640 1645 1650
   Cys Cys Thr Gly Cys Ala Cys Ala Ala Gly Thr Gly Gly Thr Cys
   1655 1660 1665
   Gly Cys Cys Ala Thr Cys Gly Cys Cys Ala Gly Cys Cys Ala Thr
   1670 1675 1680
   Gly Ala Thr Gly Gly Cys Gly Gly Thr Ala Ala Gly Cys Ala Gly
   1685 1690 1695
   Gly Cys Gly Cys Thr Gly Gly Ala Ala Ala Cys Ala Gly Thr Ala
   1700 1705 1710
   Cys Ala Gly Cys Gly Cys Cys Thr Gly Cys Thr Gly Cys Cys Thr
   1715 1720 1725
   Gly Thr Ala Cys Thr Gly Thr Gly Cys Cys Ala Gly Gly Ala Thr
   1730 1735 1740
   Cys Ala Thr Gly Gly Ala Cys Thr Gly Ala Cys Cys Cys Cys Ala
   1745 1750 1755
   Gly Ala Cys Cys Ala Gly Gly Thr Ala Gly Thr Cys Gly Cys Ala
   1760 1765 1770
   Ala Thr Cys Gly Cys Gly Thr Cys Ala Cys Ala Thr Gly Ala Cys
   1775 1780 1785
   Gly Gly Gly Gly Gly Ala Ala Ala Gly Cys Ala Ala Gly Cys Cys
   1790 1795 1800
   Cys Thr Gly Gly Ala Ala Ala Cys Cys Gly Thr Gly Cys Ala Ala
   1805 1810 1815
   Ala Gly Gly Thr Thr Gly Thr Thr Gly Cys Cys Gly Gly Thr Cys
   1820 1825 1830
   Cys Thr Thr Thr Gly Thr Cys Ala Ala Gly Ala Cys Cys Ala Cys
   1835 1840 1845
   Gly Gly Cys Cys Thr Thr Ala Cys Ala Cys Cys Gly Gly Ala Gly
   1850 1855 1860
   Cys Ala Ala Gly Thr Cys Gly Thr Gly Gly Cys Cys Ala Thr Thr
   1865 1870 1875
   Gly Cys Ala Thr Cys Cys Cys Ala Cys Gly Ala Cys Gly Gly Thr
   1880 1885 1890
   Gly Gly Cys Ala Ala Ala Cys Ala Gly Gly Cys Thr Cys Thr Thr
   1895 1900 1905
   Gly Ala Gly Ala Cys Gly Gly Thr Thr Cys Ala Gly Ala Gly Ala
   1910 1915 1920
   Cys Thr Thr Cys Thr Cys Cys Cys Ala Gly Thr Thr Cys Thr Cys
   1925 1930 1935
   Thr Gly Thr Cys Ala Ala Gly Cys Cys Cys Ala Cys Gly Gly Gly
   1940 1945 1950
   Cys Thr Gly Ala Cys Thr Cys Cys Cys Gly Ala Thr Cys Ala Ala
   1955 1960 1965
   Gly Thr Thr Gly Thr Ala Gly Cys Gly Ala Thr Thr Gly Cys Gly
   1970 1975 1980
   Ala Ala Thr Ala Ala Cys Ala Ala Thr Gly Gly Ala Gly Gly Gly
   1985 1990 1995
   Ala Ala Ala Cys Ala Ala Gly Cys Ala Thr Thr Gly Gly Ala Gly
   2000 2005 2010
   Ala Cys Thr Gly Thr Cys Cys Ala Ala Cys Gly Gly Cys Thr Cys
   2015 2020 2025
   Cys Thr Thr Cys Cys Cys Gly Thr Gly Thr Thr Gly Thr Gly Thr
   2030 2035 2040
   Cys Ala Ala Gly Cys Cys Cys Ala Cys Gly Gly Thr Thr Thr Gly
   2045 2050 2055
   Ala Cys Gly Cys Cys Thr Gly Cys Ala Cys Ala Ala Gly Thr Gly
   2060 2065 2070
   Gly Thr Cys Gly Cys Cys Ala Thr Cys Gly Cys Cys Ala Gly Cys
   2075 2080 2085
   Cys Ala Thr Gly Ala Thr Gly Gly Cys Gly Gly Thr Ala Ala Gly
   2090 2095 2100
   Cys Ala Gly Gly Cys Gly Cys Thr Gly Gly Ala Ala Ala Cys Ala
   2105 2110 2115
   Gly Thr Ala Cys Ala Gly Cys Gly Cys Cys Thr Gly Cys Thr Gly
   2120 2125 2130
   Cys Cys Thr Gly Thr Ala Cys Thr Gly Thr Gly Cys Cys Ala Gly
   2135 2140 2145
   Gly Ala Thr Cys Ala Thr Gly Gly Ala Cys Thr Gly Ala Cys Ala
   2150 2155 2160
   Cys Cys Cys Gly Ala Ala Cys Ala Gly Gly Thr Gly Gly Thr Cys
   2165 2170 2175
   Gly Cys Cys Ala Thr Thr Gly Cys Thr Thr Cys Thr Ala Ala Thr
   2180 2185 2190
   Gly Gly Gly Gly Gly Ala Gly Gly Ala Cys Gly Gly Cys Cys Ala
   2195 2200 2205
   Gly Cys Cys Thr Thr Gly Gly Ala Gly Thr Cys Cys Ala Thr Cys
   2210 2215 2220
   Gly Thr Ala Gly Cys Cys Cys Ala Ala Thr Thr Gly Thr Cys Cys
   2225 2230 2235
   Ala Gly Gly Cys Cys Cys Gly Ala Thr Cys Cys Cys Gly Cys Gly
   2240 2245 2250
   Thr Thr Gly Gly Cys Thr Gly Cys Gly Thr Thr Ala Ala Cys Gly
   2255 2260 2265
   Ala Ala Thr Gly Ala Cys Cys Ala Thr Cys Thr Gly Gly Thr Gly
   2270 2275 2280
   Gly Cys  Gly Thr Thr Gly Gly  Cys Ala Thr Gly Thr  Cys Thr Thr
       2285                 2290                 2295
   Gly Gly  Thr Gly Gly Ala Cys  Gly Ala Cys Cys Cys  Gly Cys Gly
       2300                 2305                 2310
   Cys Thr  Cys Gly Ala Thr Gly  Cys Ala Gly Thr Cys  Ala Ala Ala
       2315                 2320                 2325
   Ala Ala  Gly Gly Gly Thr Cys  Thr Gly Cys Cys Thr  Cys Ala Thr
       2330                 2335                 2340
   Gly Cys  Thr Cys Cys Cys Gly  Cys Ala Thr Thr Gly  Ala Thr Cys
       2345                 2350                 2355
   Ala Ala  Ala Ala Gly Ala Ala  Cys Cys Ala Ala Cys  Cys Gly Gly
       2360                 2365                 2370
   Cys Gly  Gly Ala Thr Thr Cys  Cys Cys Gly Ala Gly  Ala Gly Ala
       2375                 2380                 2385
   Ala Cys  Thr Thr Cys Cys Cys  Ala Thr Cys Gly Ala  Gly Thr Cys
       2390                 2395                 2400
   Gly Cys  Gly Gly Gly Ala Thr  Cys Cys Gly Ala Thr  Cys Cys Ala
       2405                 2410                 2415
   Ala Ala  Ala Ala Ala Gly Ala  Ala Gly Ala Gly Ala  Ala Ala Gly
       2420                 2425                 2430
   Gly Thr  Ala Gly Cys Cys Cys  Cys Cys Cys Cys Gly  Ala Cys Cys
       2435                 2440                 2445
   Gly Ala  Thr Gly Thr Cys Ala  Gly Cys Cys Thr Gly  Gly Gly Gly
       2450                 2455                 2460
   Gly Ala  Cys Gly Ala Gly Cys  Thr Cys Cys Ala Cys  Thr Thr Ala
       2465                 2470                 2475
   Gly Ala  Cys Gly Gly Cys Gly  Ala Gly Gly Ala Cys  Gly Thr Gly
       2480                 2485                 2490
   Gly Cys  Gly Ala Thr Gly Gly  Cys Gly Cys Ala Thr  Gly Cys Cys
       2495                 2500                 2505
   Gly Ala  Cys Gly Cys Gly Cys  Thr Ala Gly Ala Cys  Gly Ala Thr
       2510                 2515                 2520
   Thr Thr  Cys Gly Ala Thr Cys  Thr Gly Gly Ala Cys  Ala Thr Gly
       2525                 2530                 2535
   Thr Thr  Gly Gly Gly Gly Gly  Ala Cys Gly Gly Gly  Gly Ala Thr
       2540                 2545                 2550
   Thr Cys  Cys Cys Cys Gly Gly  Gly Thr Cys Cys Gly  Gly Gly Ala
       2555                 2560                 2565
   Thr Thr  Thr Ala Cys Cys Cys  Cys Cys Cys Ala Cys  Gly Ala Cys
       2570                 2575                 2580
   Thr Cys  Cys Gly Cys Cys Cys  Cys Cys Thr Ala Cys  Gly Gly Cys
       2585                 2590                 2595
   Gly Cys  Thr Cys Thr Gly Gly  Ala Thr Ala Thr Gly  Gly Cys Cys
       2600                 2605                 2610
   Gly Ala  Cys Thr Thr Cys Gly  Ala Gly Thr Thr Thr  Gly Ala Gly
       2615                 2620                 2625
   Cys Ala  Gly Ala Thr Gly Thr  Thr Thr Ala Cys Cys  Gly Ala Thr
       2630                 2635                 2640
   Gly Cys  Cys Cys Thr Thr Gly  Gly Ala Ala Thr Thr  Gly Ala Cys
       2645                 2650                 2655
   Gly Ala  Gly Thr Ala Cys Gly  Gly Thr Gly Gly Gly
       2660                 2665                 2670
<210> 12
   <211> 2670
   <212> PRT
   <213> Unknown
<220>
   <223> Synthetic genetic construct
<400> 12
   Gly Ala Cys Thr Ala Cys Ala Ala Ala Gly Ala Cys Cys Ala Thr Gly
   1               5                   10                  15
   Ala Cys Gly Gly Thr Gly Ala Thr Thr Ala Thr Ala Ala Ala Gly Ala
               20                  25                  30
   Thr Cys Ala Thr Gly Ala Cys Ala Thr Cys Gly Ala Thr Thr Ala Cys
           35                  40                  45
   Ala Ala Gly Gly Ala Thr Gly Ala Cys Gly Ala Thr Gly Ala Cys Ala
   50 55 60
   Ala Gly Ala Thr Gly Gly Cys Cys Cys Cys Cys Ala Ala Gly Ala Ala
   65 70 75 80
   Gly Ala Ala Gly Ala Gly Gly Ala Ala Gly Gly Thr Gly Gly Gly Cys
   85 90 95
   Ala Thr Thr Cys Ala Cys Cys Gly Cys Gly Gly Gly Gly Thr Ala Cys
   100 105 110
   Cys Thr Ala Thr Gly Gly Thr Gly Gly Ala Cys Thr Thr Gly Ala Gly
   115 120 125
   Gly Ala Cys Ala Cys Thr Cys Gly Gly Thr Thr Ala Thr Thr Cys Gly
   130 135 140
   Cys Ala Ala Cys Ala Gly Cys Ala Ala Cys Ala Gly Gly Ala Gly Ala
   145 150 155 160
   Ala Ala Ala Thr Cys Ala Ala Gly Cys Cys Thr Ala Ala Gly Gly Thr
   165 170 175
   Cys Ala Gly Gly Ala Gly Cys Ala Cys Cys Gly Thr Cys Gly Cys Gly
   180 185 190
   Cys Ala Ala Cys Ala Cys Cys Ala Cys Gly Ala Gly Gly Cys Gly Cys
   195 200 205
   Thr Thr Gly Thr Gly Gly Gly Gly Cys Ala Thr Gly Gly Cys Thr Thr
   210 215 220
   Cys Ala Cys Thr Cys Ala Thr Gly Cys Gly Cys Ala Thr Ala Thr Thr
   225 230 235 240
   Gly Thr Cys Gly Cys Gly Cys Thr Thr Thr Cys Ala Cys Ala Gly Cys
   245 250 255
   Ala Cys Cys Cys Thr Gly Cys Gly Gly Cys Gly Cys Thr Thr Gly Gly
   260 265 270
   Gly Ala Cys Gly Gly Thr Gly Gly Cys Thr Gly Thr Cys Ala Ala Ala
   275 280 285
   Thr Ala Cys Cys Ala Ala Gly Ala Thr Ala Thr Gly Ala Thr Thr Gly
   290 295 300
   Cys Gly Gly Cys Cys Cys Thr Gly Cys Cys Cys Gly Ala Ala Gly Cys
   305 310 315 320
   Cys Ala Cys Gly Cys Ala Cys Gly Ala Gly Gly Cys Ala Ala Thr Thr
   325 330 335
   Gly Thr Ala Gly Gly Gly Gly Thr Cys Gly Gly Thr Ala Ala Ala Cys
   340 345 350
   Ala Gly Thr Gly Gly Thr Cys Gly Gly Gly Ala Gly Cys Gly Cys Gly
   355 360 365
   Ala Gly Cys Ala Cys Thr Thr Gly Ala Gly Gly Cys Gly Cys Thr Gly
   370 375 380
   Cys Thr Gly Ala Cys Thr Gly Thr Gly Gly Cys Gly Gly Gly Thr Gly
   385 390 395 400
   Ala Gly Cys Thr Thr Ala Gly Gly Gly Gly Gly Cys Cys Thr Cys Cys
   405 410 415
   Gly Cys Thr Cys Cys Ala Gly Cys Thr Cys Gly Ala Cys Ala Cys Cys
   420 425 430
   Gly Gly Gly Cys Ala Gly Cys Thr Gly Cys Thr Gly Ala Ala Gly Ala
   435 440 445
   Thr Cys Gly Cys Gly Ala Ala Gly Ala Gly Ala Gly Gly Gly Gly Gly
   450 455 460
   Ala Gly Thr Ala Ala Cys Ala Gly Cys Gly Gly Thr Ala Gly Ala Gly
   465 470 475 480
   Gly Cys Ala Gly Thr Gly Cys Ala Cys Gly Cys Cys Thr Gly Gly Cys
   485 490 495
   Gly Cys Ala Ala Thr Gly Cys Gly Cys Thr Cys Ala Cys Cys Gly Gly
   500 505 510
   Gly Gly Cys Cys Cys Cys Cys Thr Thr Gly Ala Ala Cys Cys Thr Gly
   515 520 525
   Ala Cys Cys Cys Cys Ala Gly Ala Cys Cys Ala Gly Gly Thr Ala Gly
   530 535 540
   Thr Cys Gly Cys Ala Ala Thr Cys Gly Cys Gly Thr Cys Ala Cys Ala
   545 550 555 560
   Thr Gly Ala Cys Gly Gly Gly Gly Gly Ala Ala Ala Gly Cys Ala Ala
   565 570 575
   Gly Cys Cys Cys Thr Gly Gly Ala Ala Ala Cys Cys Gly Thr Gly Cys
   580 585 590
   Ala Ala Ala Gly Gly Thr Thr Gly Thr Thr Gly Cys Cys Gly Gly Thr
           595                 600                 605
   Cys Cys Thr Thr Thr Gly Thr Cys Ala Ala Gly Ala Cys Cys Ala Cys
       610                 615                 620
   Gly Gly Cys Cys Thr Thr Ala Cys Ala Cys Cys Gly Gly Ala Gly Cys
   625                 630                 635                 640
   Ala Ala Gly Thr Cys Gly Thr Gly Gly Cys Cys Ala Thr Thr Gly Cys
                   645                 650                 655
   Ala Ala Gly Cys Ala Ala Thr Gly Gly Gly Gly Gly Thr Gly Gly Cys
               660                 665                 670
   Ala Ala Ala Cys Ala Gly Gly Cys Thr Cys Thr Thr Gly Ala Gly Ala
           675                 680                 685
   Cys Gly Gly Thr Thr Cys Ala Gly Ala Gly Ala Cys Thr Thr Cys Thr
       690                 695                 700
   Cys Cys Cys Ala Gly Thr Thr Cys Thr Cys Thr Gly Thr Cys Ala Ala
   705                 710                 715                 720
   Gly Cys Cys Cys Ala Cys Gly Gly Gly Cys Thr Gly Ala Cys Thr Cys
                   725                 730                 735
   Cys Cys Gly Ala Thr Cys Ala Ala Gly Thr Thr Gly Thr Ala Gly Cys
               740                 745                 750
   Gly Ala Thr Thr Gly Cys Gly Thr Cys Cys Ala Ala Cys Gly Gly Thr
           755                 760                 765
   Gly Gly Ala Gly Gly Gly Ala Ala Ala Cys Ala Ala Gly Cys Ala Thr
       770                 775                 780
   Thr Gly Gly Ala Gly Ala Cys Thr Gly Thr Cys Cys Ala Ala Cys Gly
   785                 790                 795                 800
   Gly Cys Thr Cys Cys Thr Thr Cys Cys Cys Gly Thr Gly Thr Thr Gly
                   805                 810                 815
   Thr Gly Thr Cys Ala Ala Gly Cys Cys Cys Ala Cys Gly Gly Thr Thr
               820                 825                 830
   Thr Gly Ala Cys Gly Cys Cys Thr Gly Cys Ala Cys Ala Ala Gly Thr
           835                 840                 845
   Gly Gly Thr Cys Gly Cys Cys Ala Thr Cys Gly Cys Cys Ala Gly Cys
       850                 855                 860
   Cys Ala Thr Gly Ala Thr Gly Gly Cys Gly Gly Thr Ala Ala Gly Cys
   865                 870                 875                 880
   Ala Gly Gly Cys Gly Cys Thr Gly Gly Ala Ala Ala Cys Ala Gly Thr
                   885                 890                 895
   Ala Cys Ala Gly Cys Gly Cys Cys Thr Gly Cys Thr Gly Cys Cys Thr
               900                 905                 910
   Gly Thr Ala Cys Thr Gly Thr Gly Cys Cys Ala Gly Gly Ala Thr Cys
           915                 920                 925
   Ala Thr Gly Gly Ala Cys Thr Gly Ala Cys Cys Cys Cys Ala Gly Ala
       930                 935                 940
   Cys Cys Ala Gly Gly Thr Ala Gly Thr Cys Gly Cys Ala Ala Thr Cys
   945                 950                 955                 960
   Gly Cys Gly Thr Cys Ala Cys Ala Thr Gly Ala Cys Gly Gly Gly Gly
                   965                 970                 975
   Gly Ala Ala Ala Gly Cys Ala Ala Gly Cys Cys Cys Thr Gly Gly Ala
               980                 985                 990
   Ala Ala Cys Cys Gly Thr Gly Cys  Ala Ala Ala Gly Gly  Thr Thr Gly
           995                 1000                1005
   Thr Thr  Gly Cys Cys Gly Gly  Thr Cys Cys Thr Thr  Thr Gly Thr
       1010                1015                1020
   Cys Ala  Ala Gly Ala Cys Cys  Ala Cys Gly Gly Cys  Cys Thr Thr
       1025                1030                1035
   Ala Cys  Ala Cys Cys Gly Gly  Ala Gly Cys Ala Ala  Gly Thr Cys
       1040                1045                1050
   Gly Thr  Gly Gly Cys Cys Ala  Thr Thr Gly Cys Ala  Ala Ala Thr
       1055                1060                1065
   Ala Ala  Thr Ala Ala Cys Gly  Gly Thr Gly Gly Cys  Ala Ala Ala
       1070                1075                1080
   Cys Ala  Gly Gly Cys Thr Cys  Thr Thr Gly Ala Gly  Ala Cys Gly
       1085                1090                1095
   Gly Thr  Thr Cys Ala Gly Ala  Gly Ala Cys Thr Thr  Cys Thr Cys
       1100                1105                1110
   Cys Cys  Ala Gly Thr Thr Cys  Thr Cys Thr Gly Thr  Cys Ala Ala
       1115                1120                1125
   Gly Cys  Cys Cys Ala Cys Gly  Gly Gly Cys Thr Gly  Ala Cys Thr
           1130                    1135                    1140
   Cys Cys  Cys Gly Ala Thr Cys  Ala Ala Gly Thr Thr  Gly Thr Ala
           1145                    1150                    1155
   Gly Cys  Gly Ala Thr Thr Gly  Cys Gly Thr Cys Cys  Ala Ala Cys
           1160                    1165                    1170
   Gly Gly  Thr Gly Gly Ala Gly  Gly Gly Ala Ala Ala  Cys Ala Ala
           1175                    1180                    1185
   Gly Cys  Ala Thr Thr Gly Gly  Ala Gly Ala Cys Thr  Gly Thr Cys
           1190                    1195                    1200
   Cys Ala  Ala Cys Gly Gly Cys  Thr Cys Cys Thr Thr  Cys Cys Cys
           1205                    1210                    1215
   Gly Thr  Gly Thr Thr Gly Thr  Gly Thr Cys Ala Ala  Gly Cys Cys
           1220                    1225                    1230
   Cys Ala  Cys Gly Gly Thr Thr  Thr Gly Ala Cys Gly  Cys Cys Thr
           1235                    1240                    1245
   Gly Cys  Ala Cys Ala Ala Gly  Thr Gly Gly Thr Cys  Gly Cys Cys
           1250                    1255                    1260
   Ala Thr  Cys Gly Cys Cys Thr  Cys Gly Ala Ala Thr  Gly Gly Cys
           1265                    1270                    1275
   Gly Gly  Cys Gly Gly Thr Ala  Ala Gly Cys Ala Gly  Gly Cys Gly
           1280                    1285                    1290
   Cys Thr  Gly Gly Ala Ala Ala  Cys Ala Gly Thr Ala  Cys Ala Gly
           1295                    1300                    1305
   Cys Gly  Cys Cys Thr Gly Cys  Thr Gly Cys Cys Thr  Gly Thr Ala
           1310                    1315                    1320
   Cys Thr  Gly Thr Gly Cys Cys  Ala Gly Gly Ala Thr  Cys Ala Thr
           1325                    1330                    1335
   Gly Gly  Ala Cys Thr Gly Ala  Cys Cys Cys Cys Ala  Gly Ala Cys
           1340                    1345                    1350
   Cys Ala  Gly Gly Thr Ala Gly  Thr Cys Gly Cys Ala  Ala Thr Cys
           1355                    1360                    1365
   Gly Cys  Gly Thr Cys Ala Ala  Ala Cys Gly Gly Ala  Gly Gly Gly
           1370                    1375                    1380
   Gly Gly  Ala Ala Ala Gly Cys  Ala Ala Gly Cys Cys  Cys Thr Gly
           1385                    1390                    1395
   Gly Ala  Ala Ala Cys Cys Gly  Thr Gly Cys Ala Ala  Ala Gly Gly
           1400                    1405                    1410
   Thr Thr  Gly Thr Thr Gly Cys  Cys Gly Gly Thr Cys  Cys Thr Thr
           1415                    1420                    1425
   Thr Gly  Thr Cys Ala Ala Gly  Ala Cys Cys Ala Cys  Gly Gly Cys
           1430                    1435                    1440
   Cys Thr  Thr Ala Cys Ala Cys  Cys Gly Gly Ala Gly  Cys Ala Ala
           1445                    1450                    1455
   Gly Thr  Cys Gly Thr Gly Gly  Cys Cys Ala Thr Thr  Gly Cys Ala
           1460                    1465                    1470
   Thr Cys  Cys Cys Ala Cys Gly  Ala Cys Gly Gly Thr  Gly Gly Cys
           1475                    1480                    1485
   Ala Ala  Ala Cys Ala Gly Gly  Cys Thr Cys Thr Thr  Gly Ala Gly
           1490                    1495                    1500
   Ala Cys  Gly Gly Thr Thr Cys  Ala Gly Ala Gly Ala  Cys Thr Thr
           1505                    1510                    1515
   Cys Thr  Cys Cys Cys Ala Gly  Thr Thr Cys Thr Cys  Thr Gly Thr
           1520                    1525                    1530
   Cys Ala  Ala Gly Cys Cys Cys  Ala Cys Gly Gly Gly  Cys Thr Gly
           1535                    1540                    1545
   Ala Cys  Thr Cys Cys Cys Gly  Ala Thr Cys Ala Ala  Gly Thr Thr
           1550                    1555                    1560
   Gly Thr  Ala Gly Cys Gly Ala  Thr Thr Gly Cys Gly  Thr Cys Gly
           1565                    1570                    1575
   Cys Ala  Thr Gly Ala Cys Gly  Gly Ala Gly Gly Gly  Ala Ala Ala
           1580                    1585                    1590
   Cys Ala  Ala Gly Cys Ala Thr  Thr Gly Gly Ala Gly  Ala Cys Thr
           1595                    1600                    1605
   Gly Thr  Cys Cys Ala Ala Cys  Gly Gly Cys Thr Cys  Cys Thr Thr
           1610                    1615                    1620
   Cys Cys  Cys Gly Thr Gly Thr  Thr Gly Thr Gly Thr  Cys Ala Ala
           1625                    1630                    1635
   Gly Cys  Cys Cys Ala Cys Gly  Gly Thr Thr Thr Gly  Ala Cys Gly
       1640                1645                1650
   Cys Cys  Thr Gly Cys Ala Cys  Ala Ala Gly Thr Gly  Gly Thr Cys
       1655                1660                1665
   Gly Cys  Cys Ala Thr Cys Gly  Cys Cys Thr Cys Cys  Ala Ala Thr
       1670                1675                1680
   Ala Thr  Thr Gly Gly Cys Gly  Gly Thr Ala Ala Gly  Cys Ala Gly
       1685                1690                1695
   Gly Cys  Gly Cys Thr Gly Gly  Ala Ala Ala Cys Ala  Gly Thr Ala
       1700                1705                1710
   Cys Ala  Gly Cys Gly Cys Cys  Thr Gly Cys Thr Gly  Cys Cys Thr
       1715                1720                1725
   Gly Thr  Ala Cys Thr Gly Thr  Gly Cys Cys Ala Gly  Gly Ala Thr
       1730                1735                1740
   Cys Ala  Thr Gly Gly Ala Cys  Thr Gly Ala Cys Cys  Cys Cys Ala
       1745                1750                1755
   Gly Ala  Cys Cys Ala Gly Gly  Thr Ala Gly Thr Cys  Gly Cys Ala
       1760                1765                1770
   Ala Thr  Cys Gly Cys Gly Thr  Cys Ala Cys Ala Thr  Gly Ala Cys
       1775                1780                1785
   Gly Gly  Gly Gly Gly Ala Ala  Ala Gly Cys Ala Ala  Gly Cys Cys
       1790                1795                1800
   Cys Thr  Gly Gly Ala Ala Ala  Cys Cys Gly Thr Gly  Cys Ala Ala
       1805                1810                1815
   Ala Gly  Gly Thr Thr Gly Thr  Thr Gly Cys Cys Gly  Gly Thr Cys
       1820                1825                1830
   Cys Thr  Thr Thr Gly Thr Cys  Ala Ala Gly Ala Cys  Cys Ala Cys
       1835                1840                1845
   Gly Gly  Cys Cys Thr Thr Ala  Cys Ala Cys Cys Gly  Gly Ala Gly
       1850                1855                1860
   Cys Ala  Ala Gly Thr Cys Gly  Thr Gly Gly Cys Cys  Ala Thr Thr
       1865                1870                1875
   Gly Cys  Ala Ala Gly Cys Ala  Ala Cys Ala Thr Cys  Gly Gly Thr
       1880                1885                1890
   Gly Gly  Cys Ala Ala Ala Cys  Ala Gly Gly Cys Thr  Cys Thr Thr
       1895                1900                1905
   Gly Ala  Gly Ala Cys Gly Gly  Thr Thr Cys Ala Gly  Ala Gly Ala
       1910                1915                1920
   Cys Thr  Thr Cys Thr Cys Cys  Cys Ala Gly Thr Thr  Cys Thr Cys
       1925                1930                1935
   Thr Gly  Thr Cys Ala Ala Gly  Cys Cys Cys Ala Cys  Gly Gly Gly
       1940                1945                1950
   Cys Thr  Gly Ala Cys Thr Cys  Cys Cys Gly Ala Thr  Cys Ala Ala
       1955                1960                1965
   Gly Thr  Thr Gly Thr Ala Gly  Cys Gly Ala Thr Thr  Gly Cys Gly
       1970                1975                1980
   Thr Cys  Cys Ala Ala Cys Gly  Gly Thr Gly Gly Ala  Gly Gly Gly
       1985                1990                1995
   Ala Ala  Ala Cys Ala Ala Gly  Cys Ala Thr Thr Gly  Gly Ala Gly
       2000                2005                2010
   Ala Cys  Thr Gly Thr Cys Cys  Ala Ala Cys Gly Gly  Cys Thr Cys
       2015                2020                2025
   Cys Thr  Thr Cys Cys Cys Gly  Thr Gly Thr Thr Gly  Thr Gly Thr
       2030                2035                2040
   Cys Ala  Ala Gly Cys Cys Cys  Ala Cys Gly Gly Thr  Thr Thr Gly
       2045                2050                2055
   Ala Cys  Gly Cys Cys Thr Gly  Cys Ala Cys Ala Ala  Gly Thr Gly
       2060                2065                2070
   Gly Thr  Cys Gly Cys Cys Ala  Thr Cys Gly Cys Cys  Ala Gly Cys
       2075                2080                2085
   Cys Ala  Thr Gly Ala Thr Gly  Gly Cys Gly Gly Thr  Ala Ala Gly
       2090                2095                2100
   Cys Ala  Gly Gly Cys Gly Cys  Thr Gly Gly Ala Ala  Ala Cys Ala
       2105                2110                2115
   Gly Thr  Ala Cys Ala Gly Cys  Gly Cys Cys Thr Gly  Cys Thr Gly
       2120                2125                2130
   Cys Cys  Thr Gly Thr Ala Cys  Thr Gly Thr Gly Cys  Cys Ala Gly
       2135                2140                2145
   Gly Ala  Thr Cys Ala Thr Gly  Gly Ala Cys Thr Gly  Ala Cys Ala
               2150                2155                2160
   Cys Cys  Cys Gly Ala Ala Cys  Ala Gly Gly Thr Gly  Gly Thr Cys
       2165                2170                2175
   Gly Cys  Cys Ala Thr Thr Gly  Cys Thr Thr Cys Thr  Ala Ala Thr
       2180                2185                2190
   Gly Gly  Gly Gly Gly Ala Gly  Gly Ala Cys Gly Gly  Cys Cys Ala
       2195                2200                2205
   Gly Cys  Cys Thr Thr Gly Gly  Ala Gly Thr Cys Cys  Ala Thr Cys
       2210                2215                2220
   Gly Thr  Ala Gly Cys Cys Cys  Ala Ala Thr Thr Gly  Thr Cys Cys
       2225                2230                2235
   Ala Gly  Gly Cys Cys Cys Gly  Ala Thr Cys Cys Cys  Gly Cys Gly
       2240                2245                2250
   Thr Thr  Gly Gly Cys Thr Gly  Cys Gly Thr Thr Ala  Ala Cys Gly
       2255                2260                2265
   Ala Ala  Thr Gly Ala Cys Cys  Ala Thr Cys Thr Gly  Gly Thr Gly
       2270                2275                2280
   Gly Cys  Gly Thr Thr Gly Gly  Cys Ala Thr Gly Thr  Cys Thr Thr
       2285                2290                2295
   Gly Gly  Thr Gly Gly Ala Cys  Gly Ala Cys Cys Cys  Gly Cys Gly
       2300                2305                2310
   Cys Thr  Cys Gly Ala Thr Gly  Cys Ala Gly Thr Cys  Ala Ala Ala
       2315                2320                2325
   Ala Ala  Gly Gly Gly Thr Cys  Thr Gly Cys Cys Thr  Cys Ala Thr
       2330                2335                2340
   Gly Cys  Thr Cys Cys Cys Gly  Cys Ala Thr Thr Gly  Ala Thr Cys
       2345                2350                2355
   Ala Ala  Ala Ala Gly Ala Ala  Cys Cys Ala Ala Cys  Cys Gly Gly
       2360                2365                2370
   Cys Gly  Gly Ala Thr Thr Cys  Cys Cys Gly Ala Gly  Ala Gly Ala
       2375                2380                2385
   Ala Cys  Thr Thr Cys Cys Cys  Ala Thr Cys Gly Ala  Gly Thr Cys
       2390                2395                2400
   Gly Cys  Gly Gly Gly Ala Thr  Cys Cys Gly Ala Thr  Cys Cys Ala
       2405                2410                2415
   Ala Ala  Ala Ala Ala Gly Ala  Ala Gly Ala Gly Ala  Ala Ala Gly
       2420                2425                2430
   Gly Thr  Ala Gly Cys Cys Cys  Cys Cys Cys Cys Gly  Ala Cys Cys
       2435                2440                2445
   Gly Ala  Thr Gly Thr Cys Ala  Gly Cys Cys Thr Gly  Gly Gly Gly
       2450                2455                2460
   Gly Ala  Cys Gly Ala Gly Cys  Thr Cys Cys Ala Cys  Thr Thr Ala
       2465                2470                2475
   Gly Ala  Cys Gly Gly Cys Gly  Ala Gly Gly Ala Cys  Gly Thr Gly
       2480                2485                2490
   Gly Cys  Gly Ala Thr Gly Gly  Cys Gly Cys Ala Thr  Gly Cys Cys
       2495                2500                2505
   Gly Ala  Cys Gly Cys Gly Cys  Thr Ala Gly Ala Cys  Gly Ala Thr
       2510                2515                2520
   Thr Thr  Cys Gly Ala Thr Cys  Thr Gly Gly Ala Cys  Ala Thr Gly
       2525                2530                2535
   Thr Thr  Gly Gly Gly Gly Gly  Ala Cys Gly Gly Gly  Gly Ala Thr
       2540                2545                2550
   Thr Cys  Cys Cys Cys Gly Gly  Gly Thr Cys Cys Gly  Gly Gly Ala
       2555                2560                2565
   Thr Thr  Thr Ala Cys Cys Cys  Cys Cys Cys Ala Cys  Gly Ala Cys
       2570                2575                2580
   Thr Cys  Cys Gly Cys Cys Cys  Cys Cys Thr Ala Cys  Gly Gly Cys
       2585                2590                2595
   Gly Cys  Thr Cys Thr Gly Gly  Ala Thr Ala Thr Gly  Gly Cys Cys
       2600                2605                2610
   Gly Ala  Cys Thr Thr Cys Gly  Ala Gly Thr Thr Thr  Gly Ala Gly
       2615                2620                2625
   Cys Ala  Gly Ala Thr Gly Thr  Thr Thr Ala Cys Cys  Gly Ala Thr
       2630                2635                2640
   Gly Cys  Cys Cys Thr Thr Gly  Gly Ala Ala Thr Thr  Gly Ala Cys
       2645                2650                2655
           Gly Ala  Gly Thr Ala Cys Gly  Gly Thr Gly Gly Gly
   2660 2665 2670
<210> 13
   <211> 2820
   <212> PRT
   <213> Unknown
<220>
   <223> Synthetic genetic construct
<400> 13
<210> 14
   <211> 2820
   <212> PRT
   <213> Unknown
<220>
   <223> Synthetic genetic construct
<400> 14
   Gly Ala Cys Thr Ala Cys Ala Ala Ala Gly Ala Cys Cys Ala Thr Gly
   1               5                   10                  15
   Ala Cys Gly Gly Thr Gly Ala Thr Thr Ala Thr Ala Ala Ala Gly Ala
               20                  25                  30
   Thr Cys Ala Thr Gly Ala Cys Ala Thr Cys Gly Ala Thr Thr Ala Cys
           35                  40                  45
   Ala Ala Gly Gly Ala Thr Gly Ala Cys Gly Ala Thr Gly Ala Cys Ala
       50                  55                  60
   Ala Gly Ala Thr Gly Gly Cys Cys Cys Cys Cys Ala Ala Gly Ala Ala
   65                  70                  75                  80
   Gly Ala Ala Gly Ala Gly Gly Ala Ala Gly Gly Thr Gly Gly Gly Cys
                   85                  90                  95
   Ala Thr Thr Cys Ala Cys Cys Gly Cys Gly Gly Gly Gly Thr Ala Cys
               100                 105                 110
   Cys Thr Ala Thr Gly Gly Thr Gly Gly Ala Cys Thr Thr Gly Ala Gly
           115                 120                 125
   Gly Ala Cys Ala Cys Thr Cys Gly Gly Thr Thr Ala Thr Thr Cys Gly
       130                 135                 140
   Cys Ala Ala Cys Ala Gly Cys Ala Ala Cys Ala Gly Gly Ala Gly Ala
   145                 150                 155                 160
   Ala Ala Ala Thr Cys Ala Ala Gly Cys Cys Thr Ala Ala Gly Gly Thr
                       165                 170                 175
   Cys Ala Gly Gly Ala Gly Cys Ala Cys Cys Gly Thr Cys Gly Cys Gly
                   180                 185                 190
   Cys Ala Ala Cys Ala Cys Cys Ala Cys Gly Ala Gly Gly Cys Gly Cys
               195                 200                 205
   Thr Thr Gly Thr Gly Gly Gly Gly Cys Ala Thr Gly Gly Cys Thr Thr
       210                 215                 220
   Cys Ala Cys Thr Cys Ala Thr Gly Cys Gly Cys Ala Thr Ala Thr Thr
   225                 230                 235                 240
   Gly Thr Cys Gly Cys Gly Cys Thr Thr Thr Cys Ala Cys Ala Gly Cys
                   245                 250                 255
   Ala Cys Cys Cys Thr Gly Cys Gly Gly Cys Gly Cys Thr Thr Gly Gly
               260                 265                 270
   Gly Ala Cys Gly Gly Thr Gly Gly Cys Thr Gly Thr Cys Ala Ala Ala
           275                 280                 285
   Thr Ala Cys Cys Ala Ala Gly Ala Thr Ala Thr Gly Ala Thr Thr Gly
       290                 295                 300
   Cys Gly Gly Cys Cys Cys Thr Gly Cys Cys Cys Gly Ala Ala Gly Cys
   305                 310                 315                 320
   Cys Ala Cys Gly Cys Ala Cys Gly Ala Gly Gly Cys Ala Ala Thr Thr
                   325                 330                 335
   Gly Thr Ala Gly Gly Gly Gly Thr Cys Gly Gly Thr Ala Ala Ala Cys
               340                 345                 350
   Ala Gly Thr Gly Gly Thr Cys Gly Gly Gly Ala Gly Cys Gly Cys Gly
           355                 360                 365
   Ala Gly Cys Ala Cys Thr Thr Gly Ala Gly Gly Cys Gly Cys Thr Gly
       370                 375                 380
   Cys Thr Gly Ala Cys Thr Gly Thr Gly Gly Cys Gly Gly Gly Thr Gly
   385                 390                 395                 400
   Ala Gly Cys Thr Thr Ala Gly Gly Gly Gly Gly Cys Cys Thr Cys Cys
                   405                 410                 415
   Gly Cys Thr Cys Cys Ala Gly Cys Thr Cys Gly Ala Cys Ala Cys Cys
               420                 425                 430
   Gly Gly Gly Cys Ala Gly Cys Thr Gly Cys Thr Gly Ala Ala Gly Ala
           435                 440                 445
   Thr Cys Gly Cys Gly Ala Ala Gly Ala Gly Ala Gly Gly Gly Gly Gly
       450                 455                 460
   Ala Gly Thr Ala Ala Cys Ala Gly Cys Gly Gly Thr Ala Gly Ala Gly
   465                 470                 475                 480
   Gly Cys Ala Gly Thr Gly Cys Ala Cys Gly Cys Cys Thr Gly Gly Cys
                   485                 490                 495
   Gly Cys Ala Ala Thr Gly Cys Gly Cys Thr Cys Ala Cys Cys Gly Gly
               500                 505                 510
   Gly Gly Cys Cys Cys Cys Cys Thr Thr Gly Ala Ala Cys Cys Thr Gly
           515                 520                 525
   Ala Cys Cys Cys Cys Ala Gly Ala Cys Cys Ala Gly Gly Thr Ala Gly
       530                 535                 540
   Thr Cys Gly Cys Ala Ala Thr Cys Gly Cys Gly Thr Cys Ala Cys Ala
   545                 550                 555                 560
   Thr Gly Ala Cys Gly Gly Gly Gly Gly Ala Ala Ala Gly Cys Ala Ala
                   565                 570                 575
   Gly Cys Cys Cys Thr Gly Gly Ala Ala Ala Cys Cys Gly Thr Gly Cys
               580                 585                 590
   Ala Ala Ala Gly Gly Thr Thr Gly Thr Thr Gly Cys Cys Gly Gly Thr
           595                 600                 605
   Cys Cys Thr Thr Thr Gly Thr Cys Ala Ala Gly Ala Cys Cys Ala Cys
       610                 615                 620
   Gly Gly Cys Cys Thr Thr Ala Cys Ala Cys Cys Gly Gly Ala Gly Cys
   625                 630                 635                 640
   Ala Ala Gly Thr Cys Gly Thr Gly Gly Cys Cys Ala Thr Thr Gly Cys
                   645                 650                 655
   Ala Ala Gly Cys Ala Ala Thr Gly Gly Gly Gly Gly Thr Gly Gly Cys
               660                 665                 670
   Ala Ala Ala Cys Ala Gly Gly Cys Thr Cys Thr Thr Gly Ala Gly Ala
           675                 680                 685
   Cys Gly Gly Thr Thr Cys Ala Gly Ala Gly Ala Cys Thr Thr Cys Thr
       690                 695                 700
   Cys Cys Cys Ala Gly Thr Thr Cys Thr Cys Thr Gly Thr Cys Ala Ala
   705                 710                 715                 720
   Gly Cys Cys Cys Ala Cys Gly Gly Gly Cys Thr Gly Ala Cys Thr Cys
                   725                 730                 735
   Cys Cys Gly Ala Thr Cys Ala Ala Gly Thr Thr Gly Thr Ala Gly Cys
               740                 745                 750
   Gly Ala Thr Thr Gly Cys Gly Thr Cys Cys Ala Ala Cys Gly Gly Thr
           755                 760                 765
   Gly Gly Ala Gly Gly Gly Ala Ala Ala Cys Ala Ala Gly Cys Ala Thr
       770                 775                 780
   Thr Gly Gly Ala Gly Ala Cys Thr Gly Thr Cys Cys Ala Ala Cys Gly
   785                 790                 795                 800
   Gly Cys Thr Cys Cys Thr Thr Cys Cys Cys Gly Thr Gly Thr Thr Gly
                   805                 810                 815
   Thr Gly Thr Cys Ala Ala Gly Cys Cys Cys Ala Cys Gly Gly Thr Thr
               820                 825                 830
   Thr Gly Ala Cys Gly Cys Cys Thr Gly Cys Ala Cys Ala Ala Gly Thr
           835                 840                 845
   Gly Gly Thr Cys Gly Cys Cys Ala Thr Cys Gly Cys Cys Ala Gly Cys
       850                 855                 860
   Cys Ala Thr Gly Ala Thr Gly Gly Cys Gly Gly Thr Ala Ala Gly Cys
   865                 870                 875                 880
   Ala Gly Gly Cys Gly Cys Thr Gly Gly Ala Ala Ala Cys Ala Gly Thr
                   885                 890                 895
   Ala Cys Ala Gly Cys Gly Cys Cys Thr Gly Cys Thr Gly Cys Cys Thr
               900                 905                 910
   Gly Thr Ala Cys Thr Gly Thr Gly Cys Cys Ala Gly Gly Ala Thr Cys
           915                 920                 925
   Ala Thr Gly Gly Ala Cys Thr Gly Ala Cys Cys Cys Cys Ala Gly Ala
       930                 935                 940
   Cys Cys Ala Gly Gly Thr Ala Gly Thr Cys Gly Cys Ala Ala Thr Cys
   945                 950                 955                 960
   Gly Cys Gly Thr Cys Ala Cys Ala Thr Gly Ala Cys Gly Gly Gly Gly
                   965                 970                 975
   Gly Ala Ala Ala Gly Cys Ala Ala Gly Cys Cys Cys Thr Gly Gly Ala
               980                 985                 990
   Ala Ala Cys Cys Gly Thr Gly Cys  Ala Ala Ala Gly Gly  Thr Thr Gly
           995                 1000                1005
   Thr Thr  Gly Cys Cys Gly Gly  Thr Cys Cys Thr Thr  Thr Gly Thr
       1010                1015                1020
   Cys Ala  Ala Gly Ala Cys Cys  Ala Cys Gly Gly Cys  Cys Thr Thr
       1025                1030                1035
   Ala Cys  Ala Cys Cys Gly Gly  Ala Gly Cys Ala Ala  Gly Thr Cys
       1040                1045                1050
   Gly Thr  Gly Gly Cys Cys Ala  Thr Thr Gly Cys Ala  Ala Ala Thr
       1055                1060                1065
   Ala Ala  Thr Ala Ala Cys Gly  Gly Thr Gly Gly Cys  Ala Ala Ala
       1070                1075                1080
   Cys Ala  Gly Gly Cys Thr Cys  Thr Thr Gly Ala Gly  Ala Cys Gly
       1085                1090                1095
   Gly Thr  Thr Cys Ala Gly Ala  Gly Ala Cys Thr Thr  Cys Thr Cys
       1100                1105                1110
   Cys Cys  Ala Gly Thr Thr Cys  Thr Cys Thr Gly Thr  Cys Ala Ala
       1115                1120                1125
   Gly Cys  Cys Cys Ala Cys Gly  Gly Gly Cys Thr Gly  Ala Cys Thr
       1130                1135                1140
   Cys Cys  Cys Gly Ala Thr Cys  Ala Ala Gly Thr Thr  Gly Thr Ala
       1145                1150                1155
   Gly Cys  Gly Ala Thr Thr Gly  Cys Gly Thr Cys Cys  Ala Ala Cys
       1160                1165                1170
   Gly Gly  Thr Gly Gly Ala Gly  Gly Gly Ala Ala Ala  Cys Ala Ala
       1175                1180                1185
   Gly Cys  Ala Thr Thr Gly Gly  Ala Gly Ala Cys Thr  Gly Thr Cys
       1190                1195                1200
   Cys Ala  Ala Cys Gly Gly Cys  Thr Cys Cys Thr Thr  Cys Cys Cys
       1205                1210                1215
   Gly Thr  Gly Thr Thr Gly Thr  Gly Thr Cys Ala Ala  Gly Cys Cys
       1220                1225                1230
   Cys Ala  Cys Gly Gly Thr Thr  Thr Gly Ala Cys Gly  Cys Cys Thr
           1235                    1240                    1245
   Gly Cys  Ala Cys Ala Ala Gly  Thr Gly Gly Thr Cys  Gly Cys Cys
       1250                    1255                    1260
   Ala Thr  Cys Gly Cys Cys Thr  Cys Gly Ala Ala Thr  Gly Gly Cys
       1265                    1270                    1275
   Gly Gly  Cys Gly Gly Thr Ala  Ala Gly Cys Ala Gly  Gly Cys Gly
       1280                    1285                    1290
   Cys Thr  Gly Gly Ala Ala Ala  Cys Ala Gly Thr Ala  Cys Ala Gly
       1295                    1300                    1305
   Cys Gly  Cys Cys Thr Gly Cys  Thr Gly Cys Cys Thr  Gly Thr Ala
       1310                    1315                    1320
   Cys Thr  Gly Thr Gly Cys Cys  Ala Gly Gly Ala Thr  Cys Ala Thr
       1325                    1330                    1335
   Gly Gly  Ala Cys Thr Gly Ala  Cys Cys Cys Cys Ala  Gly Ala Cys
       1340                    1345                    1350
   Cys Ala  Gly Gly Thr Ala Gly  Thr Cys Gly Cys Ala  Ala Thr Cys
       1355                    1360                    1365
   Gly Cys  Gly Thr Cys Ala Ala  Ala Cys Gly Gly Ala  Gly Gly Gly
       1370                    1375                    1380
   Gly Gly  Ala Ala Ala Gly Cys  Ala Ala Gly Cys Cys  Cys Thr Gly
       1385                    1390                    1395
   Gly Ala  Ala Ala Cys Cys Gly  Thr Gly Cys Ala Ala  Ala Gly Gly
       1400                    1405                    1410
   Thr Thr  Gly Thr Thr Gly Cys  Cys Gly Gly Thr Cys  Cys Thr Thr
       1415                    1420                    1425
   Thr Gly  Thr Cys Ala Ala Gly  Ala Cys Cys Ala Cys  Gly Gly Cys
       1430                    1435                    1440
   Cys Thr  Thr Ala Cys Ala Cys  Cys Gly Gly Ala Gly  Cys Ala Ala
       1445                    1450                    1455
   Gly Thr  Cys Gly Thr Gly Gly  Cys Cys Ala Thr Thr  Gly Cys Ala
       1460                    1465                    1470
   Thr Cys  Cys Cys Ala Cys Gly  Ala Cys Gly Gly Thr  Gly Gly Cys
       1475                    1480                    1485
   Ala Ala  Ala Cys Ala Gly Gly  Cys Thr Cys Thr Thr  Gly Ala Gly
       1490                    1495                    1500
   Ala Cys  Gly Gly Thr Thr Cys  Ala Gly Ala Gly Ala  Cys Thr Thr
       1505                    1510                    1515
   Cys Thr  Cys Cys Cys Ala Gly  Thr Thr Cys Thr Cys  Thr Gly Thr
       1520                    1525                    1530
   Cys Ala  Ala Gly Cys Cys Cys  Ala Cys Gly Gly Gly  Cys Thr Gly
       1535                    1540                    1545
   Ala Cys  Thr Cys Cys Cys Gly  Ala Thr Cys Ala Ala  Gly Thr Thr
       1550                    1555                    1560
   Gly Thr  Ala Gly Cys Gly Ala  Thr Thr Gly Cys Gly  Thr Cys Gly
       1565                    1570                    1575
   Cys Ala  Thr Gly Ala Cys Gly  Gly Ala Gly Gly Gly  Ala Ala Ala
       1580                    1585                    1590
   Cys Ala  Ala Gly Cys Ala Thr  Thr Gly Gly Ala Gly  Ala Cys Thr
       1595                    1600                    1605
   Gly Thr  Cys Cys Ala Ala Cys  Gly Gly Cys Thr Cys  Cys Thr Thr
       1610                    1615                    1620
   Cys Cys  Cys Gly Thr Gly Thr  Thr Gly Thr Gly Thr  Cys Ala Ala
       1625                    1630                    1635
   Gly Cys  Cys Cys Ala Cys Gly  Gly Thr Thr Thr Gly  Ala Cys Gly
       1640                    1645                    1650
   Cys Cys  Thr Gly Cys Ala Cys  Ala Ala Gly Thr Gly  Gly Thr Cys
       1655                    1660                    1665
   Gly Cys  Cys Ala Thr Cys Gly  Cys Cys Thr Cys Cys  Ala Ala Thr
       1670                    1675                    1680
   Ala Thr  Thr Gly Gly Cys Gly  Gly Thr Ala Ala Gly  Cys Ala Gly
       1685                    1690                    1695
   Gly Cys  Gly Cys Thr Gly Gly  Ala Ala Ala Cys Ala  Gly Thr Ala
       1700                    1705                    1710
   Cys Ala  Gly Cys Gly Cys Cys  Thr Gly Cys Thr Gly  Cys Cys Thr
       1715                    1720                    1725
   Gly Thr  Ala Cys Thr Gly Thr  Gly Cys Cys Ala Gly  Gly Ala Thr
       1730                    1735                    1740
   Cys Ala  Thr Gly Gly Ala Cys  Thr Gly Ala Cys Cys  Cys Cys Ala
   1745                1750                1755
   Gly Ala  Cys Cys Ala Gly Gly  Thr Ala Gly Thr Cys  Gly Cys Ala
       1760                1765                1770
   Ala Thr  Cys Gly Cys Gly Thr  Cys Ala Cys Ala Thr  Gly Ala Cys
       1775                1780                1785
   Gly Gly  Gly Gly Gly Ala Ala  Ala Gly Cys Ala Ala  Gly Cys Cys
       1790                1795                1800
   Cys Thr  Gly Gly Ala Ala Ala  Cys Cys Gly Thr Gly  Cys Ala Ala
       1805                1810                1815
   Ala Gly  Gly Thr Thr Gly Thr  Thr Gly Cys Cys Gly  Gly Thr Cys
       1820                1825                1830
   Cys Thr  Thr Thr Gly Thr Cys  Ala Ala Gly Ala Cys  Cys Ala Cys
       1835                1840                1845
   Gly Gly  Cys Cys Thr Thr Ala  Cys Ala Cys Cys Gly  Gly Ala Gly
       1850                1855                1860
   Cys Ala  Ala Gly Thr Cys Gly  Thr Gly Gly Cys Cys  Ala Thr Thr
       1865                1870                1875
   Gly Cys  Ala Ala Gly Cys Ala  Ala Cys Ala Thr Cys  Gly Gly Thr
       1880                1885                1890
   Gly Gly  Cys Ala Ala Ala Cys  Ala Gly Gly Cys Thr  Cys Thr Thr
       1895                1900                1905
   Gly Ala  Gly Ala Cys Gly Gly  Thr Thr Cys Ala Gly  Ala Gly Ala
       1910                1915                1920
   Cys Thr  Thr Cys Thr Cys Cys  Cys Ala Gly Thr Thr  Cys Thr Cys
       1925                1930                1935
   Thr Gly  Thr Cys Ala Ala Gly  Cys Cys Cys Ala Cys  Gly Gly Gly
       1940                1945                1950
   Cys Thr  Gly Ala Cys Thr Cys  Cys Cys Gly Ala Thr  Cys Ala Ala
       1955                1960                1965
   Gly Thr  Thr Gly Thr Ala Gly  Cys Gly Ala Thr Thr  Gly Cys Gly
       1970                1975                1980
   Thr Cys  Cys Ala Ala Cys Gly  Gly Thr Gly Gly Ala  Gly Gly Gly
       1985                1990                1995
   Ala Ala  Ala Cys Ala Ala Gly  Cys Ala Thr Thr Gly  Gly Ala Gly
       2000                2005                2010
   Ala Cys  Thr Gly Thr Cys Cys  Ala Ala Cys Gly Gly  Cys Thr Cys
       2015                2020                2025
   Cys Thr  Thr Cys Cys Cys Gly  Thr Gly Thr Thr Gly  Thr Gly Thr
       2030                2035                2040
   Cys Ala  Ala Gly Cys Cys Cys  Ala Cys Gly Gly Thr  Thr Thr Gly
       2045                2050                2055
   Ala Cys  Gly Cys Cys Thr Gly  Cys Ala Cys Ala Ala  Gly Thr Gly
       2060                2065                2070
   Gly Thr  Cys Gly Cys Cys Ala  Thr Cys Gly Cys Cys  Ala Gly Cys
       2075                2080                2085
   Cys Ala  Thr Gly Ala Thr Gly  Gly Cys Gly Gly Thr  Ala Ala Gly
       2090                2095                2100
   Cys Ala  Gly Gly Cys Gly Cys  Thr Gly Gly Ala Ala  Ala Cys Ala
       2105                2110                2115
   Gly Thr  Ala Cys Ala Gly Cys  Gly Cys Cys Thr Gly  Cys Thr Gly
       2120                2125                2130
   Cys Cys  Thr Gly Thr Ala Cys  Thr Gly Thr Gly Cys  Cys Ala Gly
       2135                2140                2145
   Gly Ala  Thr Cys Ala Thr Gly  Gly Ala Cys Thr Gly  Ala Cys Ala
       2150                2155                2160
   Cys Cys  Cys Gly Ala Ala Cys  Ala Gly Gly Thr Gly  Gly Thr Cys
       2165                2170                2175
   Gly Cys  Cys Ala Thr Thr Gly  Cys Thr Thr Cys Thr  Ala Ala Thr
       2180                2185                2190
   Gly Gly  Gly Gly Gly Ala Gly  Gly Ala Cys Gly Gly  Cys Cys Ala
       2195                2200                2205
   Gly Cys  Cys Thr Thr Gly Gly  Ala Gly Thr Cys Cys  Ala Thr Cys
       2210                2215                2220
   Gly Thr  Ala Gly Cys Cys Cys  Ala Ala Thr Thr Gly  Thr Cys Cys
       2225                2230                2235
   Ala Gly  Gly Cys Cys Cys Gly  Ala Thr Cys Cys Cys  Gly Cys Gly
       2240                2245                2250
   Thr Thr  Gly Gly Cys Thr Gly  Cys Gly Thr Thr Ala  Ala Cys Gly
   2255 2260 2265
   Ala Ala Thr Gly Ala Cys Cys Ala Thr Cys Thr Gly Gly Thr Gly
   2270 2275 2280
   Gly Cys Gly Thr Thr Gly Gly Cys Ala Thr Gly Thr Cys Thr Thr
   2285 2290 2295
   Gly Gly Thr Gly Gly Ala Cys Gly Ala Cys Cys Cys Gly Cys Gly
   2300 2305 2310
   Cys Thr Cys Gly Ala Thr Gly Cys Ala Gly Thr Cys Ala Ala Ala
   2315 2320 2325
   Ala Ala Gly Gly Gly Thr Cys Thr Gly Cys Cys Thr Cys Ala Thr
   2330 2335 2340
   Gly Cys Thr Cys Cys Cys Gly Cys Ala Thr Thr Gly Ala Thr Cys
   2345 2350 2355
   Ala Ala Ala Ala Gly Ala Ala Cys Cys Ala Ala Cys Cys Gly Gly
   2360 2365 2370
   Cys Gly Gly Ala Thr Thr Cys Cys Cys Gly Ala Gly Ala Gly Ala
   2375 2380 2385
   Ala Cys Thr Thr Cys Cys Cys Ala Thr Cys Gly Ala Gly Thr Cys
   2390 2395 2400
   Gly Cys Gly Gly Gly Ala Thr Cys Cys Gly Ala Thr Cys Cys Ala
   2405 2410 2415
   Ala Ala Ala Ala Ala Gly Ala Ala Gly Ala Gly Ala Ala Ala Gly
   2420 2425 2430
   Gly Thr Ala Cys Cys Ala Ala Ala Ala Ala Ala Gly Ala Ala Gly
   2435 2440 2445
   Ala Gly Ala Ala Ala Gly Gly Thr Cys Gly Ala Cys Gly Gly Cys
   2450 2455 2460
   Gly Gly Thr Gly Gly Thr Gly Cys Thr Thr Thr Gly Thr Cys Thr
   2465 2470 2475
   Cys Cys Thr Cys Ala Gly Cys Ala Cys Thr Cys Thr Gly Cys Thr
   2480 2485 2490
   Gly Thr Cys Ala Cys Thr Cys Ala Ala Gly Gly Ala Ala Gly Thr
   2495 2500 2505
   Ala Thr Cys Ala Thr Cys Ala Ala Gly Ala Ala Cys Ala Ala Gly
   2510 2515 2520
   Gly Ala Gly Gly Gly Cys Ala Thr Gly Gly Ala Thr Gly Cys Thr
   2525 2530 2535
   Ala Ala Gly Thr Cys Ala Cys Thr Ala Ala Cys Thr Gly Cys Cys
   2540 2545 2550
   Thr Gly Gly Thr Cys Cys Cys Gly Gly Ala Cys Ala Cys Thr Gly
   2555 2560 2565
   Gly Thr Gly Ala Cys Cys Thr Thr Cys Ala Ala Gly Gly Ala Thr
   2570 2575 2580
   Gly Thr Ala Thr Thr Thr Gly Thr Gly Gly Ala Cys Thr Thr Cys
   2585 2590 2595
   Ala Cys Cys Ala Gly Gly Gly Ala Gly Gly Ala Gly Thr Gly Gly
   2600 2605 2610
   Ala Ala Gly Cys Thr Gly Cys Thr Gly Gly Ala Cys Ala Cys Thr
   2615 2620 2625
   Gly Cys Thr Cys Ala Gly Cys Ala Gly Ala Thr Cys Gly Thr Gly
   2630 2635 2640
   Thr Ala Cys Ala Gly Ala Ala Ala Thr Gly Thr Gly Ala Thr Gly
   2645 2650 2655
   Cys Thr Gly Gly Ala Gly Ala Ala Cys Thr Ala Thr Ala Ala Gly
   2660 2665 2670
   Ala Ala Cys Cys Thr Gly Gly Thr Thr Thr Cys Cys Thr Thr Gly
   2675 2680 2685
   Gly Gly Thr Thr Ala Thr Cys Ala Gly Cys Thr Thr Ala Cys Thr
   2690 2695 2700
   Ala Ala Gly Cys Cys Ala Gly Ala Thr Gly Thr Gly Ala Thr Cys
   2705 2710 2715
   Cys Thr Cys Cys Gly Gly Thr Thr Gly Gly Ala Gly Ala Ala Gly
   2720 2725 2730
   Gly Gly Ala Gly Ala Ala Gly Ala Gly Cys Cys Cys Thr Gly Gly
   2735 2740 2745
   Cys Thr Gly Gly Thr Gly Gly Ala Gly Ala Gly Ala Gly Ala Ala
   2750 2755 2760
   Ala Thr Thr Cys Ala Cys Cys Ala Ala Gly Ala Gly Ala Cys Cys
       2765                2770                2775
   Cys Ala  Thr Cys Cys Thr Gly  Ala Thr Thr Cys Ala  Gly Ala Gly
       2780                2785                2790
   Ala Cys  Thr Gly Cys Ala Thr  Thr Thr Gly Ala Ala  Ala Thr Cys
       2795                2800                2805
   Ala Ala  Ala Thr Cys Ala Thr  Cys Ala Gly Thr Thr
       2810                2815                2820
<210> 15
   <211> 720
   <212> PRT
   <213> Unknown
<220>
   <223> Synthetic genetic construct
<400> 15
   Ala Thr Gly Gly Thr Gly Ala Gly Cys Ala Ala Gly Gly Gly Cys Gly
   1               5                   10                  15
   Ala Gly Gly Ala Gly Cys Thr Gly Thr Thr Cys Ala Cys Cys Gly Gly
               20                  25                  30
   Gly Gly Thr Gly Gly Thr Gly Cys Cys Cys Ala Thr Cys Cys Thr Gly
           35                  40                  45
   Gly Thr Cys Gly Ala Gly Cys Thr Gly Gly Ala Cys Gly Gly Cys Gly
       50                  55                  60
   Ala Cys Gly Thr Ala Ala Ala Cys Gly Gly Cys Cys Ala Cys Ala Ala
   65                  70                  75                  80
   Gly Thr Thr Cys Ala Gly Cys Gly Thr Gly Thr Cys Cys Gly Gly Cys
                   85                  90                  95
   Gly Ala Gly Gly Gly Cys Gly Ala Gly Gly Gly Cys Gly Ala Thr Gly
               100                 105                 110
   Cys Cys Ala Cys Cys Thr Ala Cys Gly Gly Cys Ala Ala Gly Cys Thr
           115                 120                 125
   Gly Ala Cys Cys Cys Thr Gly Ala Ala Gly Thr Thr Cys Ala Thr Cys
       130                 135                 140
   Thr Gly Cys Ala Cys Cys Ala Cys Cys Gly Gly Cys Ala Ala Gly Cys
   145                 150                 155                 160
   Thr Gly Cys Cys Cys Gly Thr Gly Cys Cys Cys Thr Gly Gly Cys Cys
                   165                 170                 175
   Cys Ala Cys Cys Cys Thr Cys Gly Thr Gly Ala Cys Cys Ala Cys Cys
               180                 185                 190
   Thr Thr Cys Gly Gly Cys Thr Ala Cys Gly Gly Cys Cys Thr Gly Ala
           195                 200                 205
   Thr Gly Thr Gly Cys Thr Thr Cys Gly Cys Cys Cys Gly Cys Thr Ala
       210                 215                 220
   Cys Cys Cys Cys Gly Ala Cys Cys Ala Cys Ala Thr Gly Ala Ala Gly
   225                 230                 235                 240
   Cys Ala Gly Cys Ala Cys Gly Ala Cys Thr Thr Cys Thr Thr Cys Ala
                   245                 250                 255
   Ala Gly Thr Cys Cys Gly Cys Cys Ala Thr Gly Cys Cys Cys Gly Ala
               260                 265                 270
   Ala Gly Gly Cys Thr Ala Cys Gly Thr Cys Cys Ala Gly Gly Ala Gly
           275                 280                 285
   Cys Gly Cys Ala Cys Cys Ala Thr Cys Thr Thr Cys Thr Thr Cys Ala
       290                 295                 300
   Ala Gly Gly Ala Cys Gly Ala Cys Gly Gly Cys Ala Ala Cys Thr Ala
   305                 310                 315                 320
   Cys Ala Ala Gly Ala Cys Cys Cys Gly Cys Gly Cys Cys Gly Ala Gly
                   325                 330                 335
   Gly Thr Gly Ala Ala Gly Thr Thr Cys Gly Ala Gly Gly Gly Cys Gly
               340                 345                 350
   Ala Cys Ala Cys Cys Cys Thr Gly Gly Thr Gly Ala Ala Cys Cys Gly
           355                 360                 365
   Cys Ala Thr Cys Gly Ala Gly Cys Thr Gly Ala Ala Gly Gly Gly Cys
       370                 375                 380
   Ala Thr Cys Gly Ala Cys Thr Thr Cys Ala Ala Gly Gly Ala Gly Gly
   385                 390                 395                 400
   Ala Cys Gly Gly Cys Ala Ala Cys Ala Thr Cys Cys Thr Gly Gly Gly
                   405                 410                 415
   Gly Cys Ala Cys Ala Ala Gly Cys Thr Gly Gly Ala Gly Thr Ala Cys
               420                 425                 430
   Ala Ala Cys Thr Ala Cys Ala Ala Cys Ala Gly Cys Cys Ala Cys Ala
           435                 440                 445
   Ala Cys Gly Thr Cys Thr Ala Thr Ala Thr Cys Ala Thr Gly Gly Cys
       450                 455                 460
   Cys Gly Ala Cys Ala Ala Gly Cys Ala Gly Ala Ala Gly Ala Ala Cys
   465                 470                 475                 480
   Gly Gly Cys Ala Thr Cys Ala Ala Gly Gly Thr Gly Ala Ala Cys Thr
                   485                 490                 495
   Thr Cys Ala Ala Gly Ala Thr Cys Cys Gly Cys Cys Ala Cys Ala Ala
               500                 505                 510
   Cys Ala Thr Cys Gly Ala Gly Gly Ala Cys Gly Gly Cys Ala Gly Cys
           515                 520                 525
   Gly Thr Gly Cys Ala Gly Cys Thr Cys Gly Cys Cys Gly Ala Cys Cys
       530                 535                 540
   Ala Cys Thr Ala Cys Cys Ala Gly Cys Ala Gly Ala Ala Cys Ala Cys
   545                 550                 555                 560
   Cys Cys Cys Cys Ala Thr Cys Gly Gly Cys Gly Ala Cys Gly Gly Cys
                   565                 570                 575
   Cys Cys Cys Gly Thr Gly Cys Thr Gly Cys Thr Gly Cys Cys Cys Gly
               580                 585                 590
   Ala Cys Ala Ala Cys Cys Ala Cys Thr Ala Cys Cys Thr Gly Ala Gly
           595                 600                 605
   Cys Thr Ala Cys Cys Ala Gly Thr Cys Cys Gly Cys Cys Cys Thr Gly
       610                 615                 620
   Ala Gly Cys Ala Ala Ala Gly Ala Cys Cys Cys Cys Ala Ala Cys Gly
   625                 630                 635                 640
   Ala Gly Ala Ala Gly Cys Gly Cys Gly Ala Thr Cys Ala Cys Ala Thr
                   645                 650                 655
   Gly Gly Thr Cys Cys Thr Gly Cys Thr Gly Gly Ala Gly Thr Thr Cys
               660                 665                 670
   Gly Thr Gly Ala Cys Cys Gly Cys Cys Gly Cys Cys Gly Gly Gly Ala
           675                 680                 685
   Thr Cys Ala Cys Thr Cys Thr Cys Gly Gly Cys Ala Thr Gly Gly Ala
       690                 695                 700
   Cys Gly Ala Gly Cys Thr Gly Thr Ala Cys Ala Ala Gly Thr Ala Ala
   705                 710                 715                 720
<210> 16
   <211> 699
   <212> PRT
   <213> Unknown
<220>
   <223> Synthetic genetic construct
<400> 16
   Ala Thr Gly Ala Gly Cys Gly Ala Gly Cys Thr Gly Ala Thr Thr Ala
   1               5                   10                  15
   Ala Gly Gly Ala Gly Ala Ala Cys Ala Thr Gly Cys Ala Cys Ala Thr
               20                  25                  30
   Gly Ala Ala Gly Cys Thr Gly Thr Ala Cys Ala Thr Gly Gly Ala Gly
           35                  40                  45
   Gly Gly Cys Ala Cys Cys Gly Thr Gly Gly Ala Cys Ala Ala Cys Cys
       50                  55                  60
   Ala Thr Cys Ala Cys Thr Thr Cys Ala Ala Gly Thr Gly Cys Ala Cys
   65                  70                  75                  80
   Ala Thr Cys Cys Gly Ala Gly Gly Gly Cys Gly Ala Ala Gly Gly Cys
                   85                  90                  95
   Ala Ala Gly Cys Cys Cys Thr Ala Cys Gly Ala Gly Gly Gly Cys Ala
               100                 105                 110
   Cys Cys Cys Ala Gly Ala Cys Cys Ala Thr Gly Ala Gly Ala Ala Thr
           115                 120                 125
   Cys Ala Ala Gly Gly Thr Gly Gly Thr Cys Gly Ala Gly Gly Gly Cys
       130                 135                 140
   Gly Gly Cys Cys Cys Thr Cys Thr Cys Cys Cys Cys Thr Thr Cys Gly
   145                 150                 155                 160
   Cys Cys Thr Thr Cys Gly Ala Cys Ala Thr Cys Cys Thr Gly Gly Cys
                   165                 170                 175
   Thr Ala Cys Thr Ala Gly Cys Thr Thr Cys Cys Thr Cys Thr Ala Cys
               180                 185                 190
   Gly Gly Cys Ala Gly Cys Ala Ala Gly Ala Cys Cys Thr Thr Cys Ala
           195                 200                 205
   Thr Cys Ala Ala Cys Cys Ala Cys Ala Cys Cys Cys Ala Gly Gly Gly
       210                 215                 220
   Cys Ala Thr Cys Cys Cys Cys Gly Ala Cys Thr Thr Cys Thr Thr Cys
   225                 230                 235                 240
   Ala Ala Gly Cys Ala Gly Thr Cys Cys Thr Thr Cys Cys Cys Thr Gly
                   245                 250                 255
   Ala Gly Gly Gly Cys Thr Thr Cys Ala Cys Ala Thr Gly Gly Gly Ala
               260                 265                 270
   Gly Ala Gly Ala Gly Thr Cys Ala Cys Cys Ala Cys Ala Thr Ala Cys
           275                 280                 285
   Gly Ala Ala Gly Ala Cys Gly Gly Gly Gly Gly Cys Gly Thr Gly Cys
       290                 295                 300
   Thr Gly Ala Cys Cys Gly Cys Thr Ala Cys Cys Cys Ala Gly Gly Ala
   305                 310                 315                 320
   Cys Ala Cys Cys Ala Gly Cys Cys Thr Cys Cys Ala Gly Gly Ala Cys
                   325                 330                 335
   Gly Gly Cys Thr Gly Cys Cys Thr Cys Ala Thr Cys Thr Ala Cys Ala
               340                 345                 350
   Ala Cys Gly Thr Cys Ala Ala Gly Ala Thr Cys Ala Gly Ala Gly Gly
           355                 360                 365
   Gly Gly Thr Gly Ala Ala Cys Thr Thr Cys Ala Cys Ala Thr Cys Cys
       370                 375                 380
   Ala Ala Cys Gly Gly Cys Cys Cys Thr Gly Thr Gly Ala Thr Gly Cys
   385                 390                 395                 400
   Ala Gly Ala Ala Gly Ala Ala Ala Ala Cys Ala Cys Thr Cys Gly Gly
                   405                 410                 415
   Cys Thr Gly Gly Gly Ala Gly Gly Cys Cys Thr Thr Cys Ala Cys Cys
               420                 425                 430
   Gly Ala Gly Ala Cys Gly Cys Thr Gly Thr Ala Cys Cys Cys Cys Gly
           435                 440                 445
   Cys Thr Gly Ala Cys Gly Gly Cys Gly Gly Cys Cys Thr Gly Gly Ala
       450                 455                 460
   Ala Gly Gly Cys Ala Gly Ala Ala Ala Cys Gly Ala Cys Ala Thr Gly
   465                 470                 475                 480
   Gly Cys Cys Cys Thr Gly Ala Ala Gly Cys Thr Cys Gly Thr Gly Gly
                   485                 490                 495
   Gly Cys Gly Gly Gly Ala Gly Cys Cys Ala Thr Cys Thr Gly Ala Thr
               500                 505                 510
   Cys Gly Cys Ala Ala Ala Cys Ala Thr Cys Ala Ala Gly Ala Cys Cys
           515                 520                 525
   Ala Cys Ala Thr Ala Thr Ala Gly Ala Thr Cys Cys Ala Ala Gly Ala
       530                 535                 540
   Ala Ala Cys Cys Cys Gly Cys Thr Ala Ala Gly Ala Ala Cys Cys Thr
   545                 550                 555                 560
   Cys Ala Ala Gly Ala Thr Gly Cys Cys Thr Gly Gly Cys Gly Thr Cys
                   565                 570                 575
   Thr Ala Cys Thr Ala Thr Gly Thr Gly Gly Ala Cys Thr Ala Cys Ala
               580                 585                 590
   Gly Ala Cys Thr Gly Gly Ala Ala Ala Gly Ala Ala Thr Cys Ala Ala
           595                 600                 605
   Gly Gly Ala Gly Gly Cys Cys Ala Ala Cys Ala Ala Cys Gly Ala Gly
       610                 615                 620
   Ala Cys Cys Thr Ala Cys Gly Thr Cys Gly Ala Gly Cys Ala Gly Cys
   625                 630                 635                 640
   Ala Cys Gly Ala Gly Gly Thr Gly Gly Cys Ala Gly Thr Gly Gly Cys
                   645                 650                 655
   Cys Ala Gly Ala Thr Ala Cys Thr Gly Cys Gly Ala Cys Cys Thr Cys
               660                 665                 670
   Cys Cys Thr Ala Gly Cys Ala Ala Ala Cys Thr Gly Gly Gly Gly Cys
           675                 680                 685
   Ala Cys Ala Ala Gly Cys Thr Cys Ala Ala Thr
       690                 695
<210> 17
   <211> 6309
   <212> PRT
   <213> Unknown
<220>
   <223> Synthetic genetic construct
<400> 17
   Gly Ala Cys Thr Ala Cys Ala Ala Ala Gly Ala Cys Cys Ala Thr Gly
   1               5                   10                  15
   Ala Cys Gly Gly Thr Gly Ala Thr Thr Ala Thr Ala Ala Ala Gly Ala
               20                  25                  30
   Thr Cys Ala Thr Gly Ala Cys Ala Thr Cys Gly Ala Thr Thr Ala Cys
           35                  40                  45
   Ala Ala Gly Gly Ala Thr Gly Ala Cys Gly Ala Thr Gly Ala Cys Ala
       50                  55                  60
   Ala Gly Ala Thr Gly Gly Cys Cys Cys Cys Cys Ala Ala Gly Ala Ala
   65                  70                  75                  80
   Gly Ala Ala Gly Ala Gly Gly Ala Ala Gly Gly Thr Gly Gly Gly Cys
                   85                  90                  95
   Ala Thr Thr Cys Ala Cys Cys Gly Cys Gly Gly Gly Gly Thr Ala Cys
               100                 105                 110
   Cys Thr Ala Thr Gly Gly Thr Gly Gly Ala Cys Thr Thr Gly Ala Gly
           115                 120                 125
   Gly Ala Cys Ala Cys Thr Cys Gly Gly Thr Thr Ala Thr Thr Cys Gly
       130                 135                 140
   Cys Ala Ala Cys Ala Gly Cys Ala Ala Cys Ala Gly Gly Ala Gly Ala
   145                 150                 155                 160
   Ala Ala Ala Thr Cys Ala Ala Gly Cys Cys Thr Ala Ala Gly Gly Thr
                   165                 170                 175
   Cys Ala Gly Gly Ala Gly Cys Ala Cys Cys Gly Thr Cys Gly Cys Gly
               180                 185                 190
   Cys Ala Ala Cys Ala Cys Cys Ala Cys Gly Ala Gly Gly Cys Gly Cys
           195                 200                 205
   Thr Thr Gly Thr Gly Gly Gly Gly Cys Ala Thr Gly Gly Cys Thr Thr
       210                 215                 220
   Cys Ala Cys Thr Cys Ala Thr Gly Cys Gly Cys Ala Thr Ala Thr Thr
   225                 230                 235                 240
   Gly Thr Cys Gly Cys Gly Cys Thr Thr Thr Cys Ala Cys Ala Gly Cys
                   245                 250                 255
   Ala Cys Cys Cys Thr Gly Cys Gly Gly Cys Gly Cys Thr Thr Gly Gly
               260                 265                 270
   Gly Ala Cys Gly Gly Thr Gly Gly Cys Thr Gly Thr Cys Ala Ala Ala
           275                 280                 285
   Thr Ala Cys Cys Ala Ala Gly Ala Thr Ala Thr Gly Ala Thr Thr Gly
       290                 295                 300
   Cys Gly Gly Cys Cys Cys Thr Gly Cys Cys Cys Gly Ala Ala Gly Cys
   305                 310                 315                 320
   Cys Ala Cys Gly Cys Ala Cys Gly Ala Gly Gly Cys Ala Ala Thr Thr
                   325                 330                 335
   Gly Thr Ala Gly Gly Gly Gly Thr Cys Gly Gly Thr Ala Ala Ala Cys
               340                 345                 350
   Ala Gly Thr Gly Gly Thr Cys Gly Gly Gly Ala Gly Cys Gly Cys Gly
           355                 360                 365
   Ala Gly Cys Ala Cys Thr Thr Gly Ala Gly Gly Cys Gly Cys Thr Gly
       370                 375                 380
   Cys Thr Gly Ala Cys Thr Gly Thr Gly Gly Cys Gly Gly Gly Thr Gly
   385                 390                 395                 400
   Ala Gly Cys Thr Thr Ala Gly Gly Gly Gly Gly Cys Cys Thr Cys Cys
                   405                 410                 415
   Gly Cys Thr Cys Cys Ala Gly Cys Thr Cys Gly Ala Cys Ala Cys Cys
               420                 425                 430
   Gly Gly Gly Cys Ala Gly Cys Thr Gly Cys Thr Gly Ala Ala Gly Ala
           435                 440                 445
   Thr Cys Gly Cys Gly Ala Ala Gly Ala Gly Ala Gly Gly Gly Gly Gly
       450                 455                 460
   Ala Gly Thr Ala Ala Cys Ala Gly Cys Gly Gly Thr Ala Gly Ala Gly
   465                 470                 475                 480
   Gly Cys Ala Gly Thr Gly Cys Ala Cys Gly Cys Cys Thr Gly Gly Cys
                   485                 490                 495
   Gly Cys Ala Ala Thr Gly Cys Gly Cys Thr Cys Ala Cys Cys Gly Gly
               500                 505                 510
   Gly Gly Cys Cys Cys Cys Cys Thr Thr Gly Ala Ala Cys Cys Thr Gly
           515                 520                 525
   Ala Cys Cys Cys Cys Ala Gly Ala Cys Cys Ala Gly Gly Thr Ala Gly
       530                 535                 540
   Thr Cys Gly Cys Ala Ala Thr Cys Gly Cys Gly Thr Cys Ala Cys Ala
   545                 550                 555                 560
   Thr Gly Ala Cys Gly Gly Gly Gly Gly Ala Ala Ala Gly Cys Ala Ala
                   565                 570                 575
   Gly Cys Cys Cys Thr Gly Gly Ala Ala Ala Cys Cys Gly Thr Gly Cys
               580                 585                 590
   Ala Ala Ala Gly Gly Thr Thr Gly Thr Thr Gly Cys Cys Gly Gly Thr
           595                 600                 605
   Cys Cys Thr Thr Thr Gly Thr Cys Ala Ala Gly Ala Cys Cys Ala Cys
       610                 615                 620
   Gly Gly Cys Cys Thr Thr Ala Cys Ala Cys Cys Gly Gly Ala Gly Cys
   625                 630                 635                 640
   Ala Ala Gly Thr Cys Gly Thr Gly Gly Cys Cys Ala Thr Thr Gly Cys
                   645                 650                 655
   Ala Ala Gly Cys Ala Ala Thr Gly Gly Gly Gly Gly Thr Gly Gly Cys
               660                 665                 670
   Ala Ala Ala Cys Ala Gly Gly Cys Thr Cys Thr Thr Gly Ala Gly Ala
           675                 680                 685
   Cys Gly Gly Thr Thr Cys Ala Gly Ala Gly Ala Cys Thr Thr Cys Thr
       690                 695                 700
   Cys Cys Cys Ala Gly Thr Thr Cys Thr Cys Thr Gly Thr Cys Ala Ala
   705                 710                 715                 720
   Gly Cys Cys Cys Ala Cys Gly Gly Gly Cys Thr Gly Ala Cys Thr Cys
                   725                 730                 735
   Cys Cys Gly Ala Thr Cys Ala Ala Gly Thr Thr Gly Thr Ala Gly Cys
               740                 745                 750
   Gly Ala Thr Thr Gly Cys Gly Thr Cys Cys Ala Ala Cys Gly Gly Thr
           755                 760                 765
   Gly Gly Ala Gly Gly Gly Ala Ala Ala Cys Ala Ala Gly Cys Ala Thr
       770                 775                 780
   Thr Gly Gly Ala Gly Ala Cys Thr Gly Thr Cys Cys Ala Ala Cys Gly
   785                 790                 795                 800
   Gly Cys Thr Cys Cys Thr Thr Cys Cys Cys Gly Thr Gly Thr Thr Gly
                   805                 810                 815
   Thr Gly Thr Cys Ala Ala Gly Cys Cys Cys Ala Cys Gly Gly Thr Thr
               820                 825                 830
   Thr Gly Ala Cys Gly Cys Cys Thr Gly Cys Ala Cys Ala Ala Gly Thr
           835                 840                 845
   Gly Gly Thr Cys Gly Cys Cys Ala Thr Cys Gly Cys Cys Ala Gly Cys
       850                 855                 860
   Cys Ala Thr Gly Ala Thr Gly Gly Cys Gly Gly Thr Ala Ala Gly Cys
   865                 870                 875                 880
   Ala Gly Gly Cys Gly Cys Thr Gly Gly Ala Ala Ala Cys Ala Gly Thr
                   885                 890                 895
   Ala Cys Ala Gly Cys Gly Cys Cys Thr Gly Cys Thr Gly Cys Cys Thr
               900                 905                 910
   Gly Thr Ala Cys Thr Gly Thr Gly Cys Cys Ala Gly Gly Ala Thr Cys
           915                 920                 925
   Ala Thr Gly Gly Ala Cys Thr Gly Ala Cys Cys Cys Cys Ala Gly Ala
       930                 935                 940
   Cys Cys Ala Gly Gly Thr Ala Gly Thr Cys Gly Cys Ala Ala Thr Cys
   945                 950                 955                 960
   Gly Cys Gly Thr Cys Ala Cys Ala Thr Gly Ala Cys Gly Gly Gly Gly
                   965                 970                 975
   Gly Ala Ala Ala Gly Cys Ala Ala Gly Cys Cys Cys Thr Gly Gly Ala
               980                 985                 990
   Ala Ala Cys Cys Gly Thr Gly Cys  Ala Ala Ala Gly Gly  Thr Thr Gly
           995                 1000                1005
   Thr Thr  Gly Cys Cys Gly Gly  Thr Cys Cys Thr Thr  Thr Gly Thr
       1010                1015                1020
   Cys Ala  Ala Gly Ala Cys Cys  Ala Cys Gly Gly Cys  Cys Thr Thr
       1025                 1030                 1035
   Ala Cys  Ala Cys Cys Gly Gly  Ala Gly Cys Ala Ala  Gly Thr Cys
       1040                 1045                 1050
   Gly Thr  Gly Gly Cys Cys Ala  Thr Thr Gly Cys Ala  Ala Ala Thr
       1055                 1060                 1065
   Ala Ala  Thr Ala Ala Cys Gly  Gly Thr Gly Gly Cys  Ala Ala Ala
       1070                 1075                 1080
   Cys Ala  Gly Gly Cys Thr Cys  Thr Thr Gly Ala Gly  Ala Cys Gly
       1085                 1090                 1095
   Gly Thr  Thr Cys Ala Gly Ala  Gly Ala Cys Thr Thr  Cys Thr Cys
       1100                 1105                 1110
   Cys Cys  Ala Gly Thr Thr Cys  Thr Cys Thr Gly Thr  Cys Ala Ala
       1115                 1120                 1125
   Gly Cys  Cys Cys Ala Cys Gly  Gly Gly Cys Thr Gly  Ala Cys Thr
       1130                 1135                 1140
   Cys Cys  Cys Gly Ala Thr Cys  Ala Ala Gly Thr Thr  Gly Thr Ala
       1145                 1150                 1155
   Gly Cys  Gly Ala Thr Thr Gly  Cys Gly Thr Cys Cys  Ala Ala Cys
       1160                 1165                 1170
   Gly Gly  Thr Gly Gly Ala Gly  Gly Gly Ala Ala Ala  Cys Ala Ala
       1175                 1180                 1185
   Gly Cys  Ala Thr Thr Gly Gly  Ala Gly Ala Cys Thr  Gly Thr Cys
       1190                 1195                 1200
   Cys Ala  Ala Cys Gly Gly Cys  Thr Cys Cys Thr Thr  Cys Cys Cys
       1205                 1210                 1215
   Gly Thr  Gly Thr Thr Gly Thr  Gly Thr Cys Ala Ala  Gly Cys Cys
       1220                 1225                 1230
   Cys Ala  Cys Gly Gly Thr Thr  Thr Gly Ala Cys Gly  Cys Cys Thr
       1235                 1240                 1245
   Gly Cys  Ala Cys Ala Ala Gly  Thr Gly Gly Thr Cys  Gly Cys Cys
       1250                 1255                 1260
   Ala Thr  Cys Gly Cys Cys Thr  Cys Gly Ala Ala Thr  Gly Gly Cys
       1265                 1270                 1275
   Gly Gly  Cys Gly Gly Thr Ala  Ala Gly Cys Ala Gly  Gly Cys Gly
       1280                 1285                 1290
   Cys Thr  Gly Gly Ala Ala Ala  Cys Ala Gly Thr Ala  Cys Ala Gly
       1295                 1300                 1305
   Cys Gly  Cys Cys Thr Gly Cys  Thr Gly Cys Cys Thr  Gly Thr Ala
       1310                 1315                 1320
   Cys Thr  Gly Thr Gly Cys Cys  Ala Gly Gly Ala Thr  Cys Ala Thr
       1325                 1330                 1335
   Gly Gly  Ala Cys Thr Gly Ala  Cys Cys Cys Cys Ala  Gly Ala Cys
       1340                 1345                 1350
   Cys Ala  Gly Gly Thr Ala Gly  Thr Cys Gly Cys Ala  Ala Thr Cys
       1355                 1360                 1365
   Gly Cys  Gly Thr Cys Ala Ala  Ala Cys Gly Gly Ala  Gly Gly Gly
       1370                 1375                 1380
   Gly Gly  Ala Ala Ala Gly Cys  Ala Ala Gly Cys Cys  Cys Thr Gly
       1385                 1390                 1395
   Gly Ala  Ala Ala Cys Cys Gly  Thr Gly Cys Ala Ala  Ala Gly Gly
       1400                 1405                 1410
   Thr Thr  Gly Thr Thr Gly Cys  Cys Gly Gly Thr Cys  Cys Thr Thr
       1415                 1420                 1425
   Thr Gly  Thr Cys Ala Ala Gly  Ala Cys Cys Ala Cys  Gly Gly Cys
       1430                 1435                 1440
   Cys Thr  Thr Ala Cys Ala Cys  Cys Gly Gly Ala Gly  Cys Ala Ala
       1445                 1450                 1455
   Gly Thr  Cys Gly Thr Gly Gly  Cys Cys Ala Thr Thr  Gly Cys Ala
       1460                 1465                 1470
   Thr Cys  Cys Cys Ala Cys Gly  Ala Cys Gly Gly Thr  Gly Gly Cys
       1475                 1480                 1485
   Ala Ala  Ala Cys Ala Gly Gly  Cys Thr Cys Thr Thr  Gly Ala Gly
       1490                 1495                 1500
   Ala Cys  Gly Gly Thr Thr Cys  Ala Gly Ala Gly Ala  Cys Thr Thr
       1505                 1510                 1515
   Cys Thr  Cys Cys Cys Ala Gly  Thr Thr Cys Thr Cys  Thr Gly Thr
       1520                 1525                 1530
   Cys Ala Ala Gly Cys Cys Cys Ala Cys Gly Gly Gly Cys Thr Gly
   1535 1540 1545
   Ala Cys Thr Cys Cys Cys Gly Ala Thr Cys Ala Ala Gly Thr Thr
   1550 1555 1560
   Gly Thr Ala Gly Cys Gly Ala Thr Thr Gly Cys Gly Thr Cys Gly
   1565 1570 1575
   Cys Ala Thr Gly Ala Cys Gly Gly Ala Gly Gly Gly Ala Ala Ala
   1580 1585 1590
   Cys Ala Ala Gly Cys Ala Thr Thr Gly Gly Ala Gly Ala Cys Thr
   1595 1600 1605
   Gly Thr Cys Cys Ala Ala Cys Gly Gly Cys Thr Cys Cys Thr Thr
   1610 1615 1620
   Cys Cys Cys Gly Thr Gly Thr Thr Gly Thr Gly Thr Cys Ala Ala
   1625 1630 1635
   Gly Cys Cys Cys Ala Cys Gly Gly Thr Thr Thr Gly Ala Cys Gly
   1640 1645 1650
   Cys Cys Thr Gly Cys Ala Cys Ala Ala Gly Thr Gly Gly Thr Cys
   1655 1660 1665
   Gly Cys Cys Ala Thr Cys Gly Cys Cys Thr Cys Cys Ala Ala Thr
   1670 1675 1680
   Ala Thr Thr Gly Gly Cys Gly Gly Thr Ala Ala Gly Cys Ala Gly
   1685 1690 1695
   Gly Cys Gly Cys Thr Gly Gly Ala Ala Ala Cys Ala Gly Thr Ala
   1700 1705 1710
   Cys Ala Gly Cys Gly Cys Cys Thr Gly Cys Thr Gly Cys Cys Thr
   1715 1720 1725
   Gly Thr Ala Cys Thr Gly Thr Gly Cys Cys Ala Gly Gly Ala Thr
   1730 1735 1740
   Cys Ala Thr Gly Gly Ala Cys Thr Gly Ala Cys Cys Cys Cys Ala
   1745 1750 1755
   Gly Ala Cys Cys Ala Gly Gly Thr Ala Gly Thr Cys Gly Cys Ala
   1760 1765 1770
   Ala Thr Cys Gly Cys Gly Thr Cys Ala Cys Ala Thr Gly Ala Cys
   1775 1780 1785
   Gly Gly Gly Gly Gly Ala Ala Ala Gly Cys Ala Ala Gly Cys Cys
   1790 1795 1800
   Cys Thr Gly Gly Ala Ala Ala Cys Cys Gly Thr Gly Cys Ala Ala
   1805 1810 1815
   Ala Gly Gly Thr Thr Gly Thr Thr Gly Cys Cys Gly Gly Thr Cys
   1820 1825 1830
   Cys Thr Thr Thr Gly Thr Cys Ala Ala Gly Ala Cys Cys Ala Cys
   1835 1840 1845
   Gly Gly Cys Cys Thr Thr Ala Cys Ala Cys Cys Gly Gly Ala Gly
   1850 1855 1860
   Cys Ala Ala Gly Thr Cys Gly Thr Gly Gly Cys Cys Ala Thr Thr
   1865 1870 1875
   Gly Cys Ala Ala Gly Cys Ala Ala Cys Ala Thr Cys Gly Gly Thr
   1880 1885 1890
   Gly Gly Cys Ala Ala Ala Cys Ala Gly Gly Cys Thr Cys Thr Thr
   1895 1900 1905
   Gly Ala Gly Ala Cys Gly Gly Thr Thr Cys Ala Gly Ala Gly Ala
   1910 1915 1920
   Cys Thr Thr Cys Thr Cys Cys Cys Ala Gly Thr Thr Cys Thr Cys
   1925 1930 1935
   Thr Gly Thr Cys Ala Ala Gly Cys Cys Cys Ala Cys Gly Gly Gly
   1940 1945 1950
   Cys Thr Gly Ala Cys Thr Cys Cys Cys Gly Ala Thr Cys Ala Ala
   1955 1960 1965
   Gly Thr Thr Gly Thr Ala Gly Cys Gly Ala Thr Thr Gly Cys Gly
   1970 1975 1980
   Thr Cys Cys Ala Ala Cys Gly Gly Thr Gly Gly Ala Gly Gly Gly
   1985 1990 1995
   Ala Ala Ala Cys Ala Ala Gly Cys Ala Thr Thr Gly Gly Ala Gly
   2000 2005 2010
   Ala Cys Thr Gly Thr Cys Cys Ala Ala Cys Gly Gly Cys Thr Cys
   2015 2020 2025
   Cys Thr Thr Cys Cys Cys Gly Thr Gly Thr Thr Gly Thr Gly Thr
   2030 2035 2040
   Cys Ala Ala Gly Cys Cys Cys Ala Cys Gly Gly Thr Thr Thr Gly
   2045 2050 2055
   Ala Cys Gly Cys Cys Thr Gly Cys Ala Cys Ala Ala Gly Thr Gly
   2060 2065 2070
   Gly Thr Cys Gly Cys Cys Ala Thr Cys Gly Cys Cys Ala Gly Cys
   2075 2080 2085
   Cys Ala Thr Gly Ala Thr Gly Gly Cys Gly Gly Thr Ala Ala Gly
   2090 2095 2100
   Cys Ala Gly Gly Cys Gly Cys Thr Gly Gly Ala Ala Ala Cys Ala
   2105 2110 2115
   Gly Thr Ala Cys Ala Gly Cys Gly Cys Cys Thr Gly Cys Thr Gly
   2120 2125 2130
   Cys Cys Thr Gly Thr Ala Cys Thr Gly Thr Gly Cys Cys Ala Gly
   2135 2140 2145
   Gly Ala Thr Cys Ala Thr Gly Gly Ala Cys Thr Gly Ala Cys Ala
   2150 2155 2160
   Cys Cys Cys Gly Ala Ala Cys Ala Gly Gly Thr Gly Gly Thr Cys
   2165 2170 2175
   Gly Cys Cys Ala Thr Thr Gly Cys Thr Thr Cys Thr Ala Ala Thr
   2180 2185 2190
   Gly Gly Gly Gly Gly Ala Gly Gly Ala Cys Gly Gly Cys Cys Ala
   2195 2200 2205
   Gly Cys Cys Thr Thr Gly Gly Ala Gly Thr Cys Cys Ala Thr Cys
   2210 2215 2220
   Gly Thr Ala Gly Cys Cys Cys Ala Ala Thr Thr Gly Thr Cys Cys
   2225 2230 2235
   Ala Gly Gly Cys Cys Cys Gly Ala Thr Cys Cys Cys Gly Cys Gly
   2240 2245 2250
   Thr Thr Gly Gly Cys Thr Gly Cys Gly Thr Thr Ala Ala Cys Gly
   2255 2260 2265
   Ala Ala Thr Gly Ala Cys Cys Ala Thr Cys Thr Gly Gly Thr Gly
   2270 2275 2280
   Gly Cys Gly Thr Thr Gly Gly Cys Ala Thr Gly Thr Cys Thr Thr
   2285 2290 2295
   Gly Gly Thr Gly Gly Ala Cys Gly Ala Cys Cys Cys Gly Cys Gly
   2300 2305 2310
   Cys Thr Cys Gly Ala Thr Gly Cys Ala Gly Thr Cys Ala Ala Ala
   2315 2320 2325
   Ala Ala Gly Gly Gly Thr Cys Thr Gly Cys Cys Thr Cys Ala Thr
   2330 2335 2340
   Gly Cys Thr Cys Cys Cys Gly Cys Ala Thr Thr Gly Ala Thr Cys
   2345 2350 2355
   Ala Ala Ala Ala Gly Ala Ala Cys Cys Ala Ala Cys Cys Gly Gly
   2360 2365 2370
   Cys Gly Gly Ala Thr Thr Cys Cys Cys Gly Ala Gly Ala Gly Ala
   2375 2380 2385
   Ala Cys Thr Thr Cys Cys Cys Ala Thr Cys Gly Ala Gly Thr Cys
   2390 2395 2400
   Gly Cys Gly Gly Gly Ala Thr Cys Cys Gly Ala Thr Cys Cys Ala
   2405 2410 2415
   Ala Ala Ala Ala Ala Gly Ala Ala Gly Ala Gly Ala Ala Ala Gly
   2420 2425 2430
   Gly Thr Ala Cys Cys Ala Ala Ala Ala Ala Ala Gly Ala Ala Gly
   2435 2440 2445
   Ala Gly Ala Ala Ala Gly Gly Thr Cys Gly Ala Cys Gly Gly Cys
   2450 2455 2460
   Gly Gly Thr Gly Gly Thr Gly Cys Thr Thr Thr Gly Thr Cys Thr
   2465 2470 2475
   Cys Cys Thr Cys Ala Gly Cys Ala Cys Thr Cys Thr Gly Cys Thr
   2480 2485 2490
   Gly Thr Cys Ala Cys Thr Cys Ala Ala Gly Gly Ala Ala Gly Thr
   2495 2500 2505
   Ala Thr Cys Ala Thr Cys Ala Ala Gly Ala Ala Cys Ala Ala Gly
   2510 2515 2520
   Gly Ala Gly Gly Gly Cys Ala Thr Gly Gly Ala Thr Gly Cys Thr
   2525 2530 2535
   Ala Ala Gly Thr Cys Ala Cys Thr Ala Ala Cys Thr Gly Cys Cys
   2540 2545 2550
   Thr Gly  Gly Thr Cys Cys Cys  Gly Gly Ala Cys Ala  Cys Thr Gly
       2555                 2560                 2565
   Gly Thr  Gly Ala Cys Cys Thr  Thr Cys Ala Ala Gly  Gly Ala Thr
       2570                 2575                 2580
   Gly Thr  Ala Thr Thr Thr Gly  Thr Gly Gly Ala Cys  Thr Thr Cys
       2585                 2590                 2595
   Ala Cys  Cys Ala Gly Gly Gly  Ala Gly Gly Ala Gly  Thr Gly Gly
       2600                 2605                 2610
   Ala Ala  Gly Cys Thr Gly Cys  Thr Gly Gly Ala Cys  Ala Cys Thr
       2615                 2620                 2625
   Gly Cys  Thr Cys Ala Gly Cys  Ala Gly Ala Thr Cys  Gly Thr Gly
       2630                 2635                 2640
   Thr Ala  Cys Ala Gly Ala Ala  Ala Thr Gly Thr Gly  Ala Thr Gly
       2645                 2650                 2655
   Cys Thr  Gly Gly Ala Gly Ala  Ala Cys Thr Ala Thr  Ala Ala Gly
       2660                 2665                 2670
   Ala Ala  Cys Cys Thr Gly Gly  Thr Thr Thr Cys Cys  Thr Thr Gly
       2675                 2680                 2685
   Gly Gly  Thr Thr Ala Thr Cys  Ala Gly Cys Thr Thr  Ala Cys Thr
       2690                 2695                 2700
   Ala Ala  Gly Cys Cys Ala Gly  Ala Thr Gly Thr Gly  Ala Thr Cys
       2705                 2710                 2715
   Cys Thr  Cys Cys Gly Gly Thr  Thr Gly Gly Ala Gly  Ala Ala Gly
       2720                 2725                 2730
   Gly Gly  Ala Gly Ala Ala Gly  Ala Gly Cys Cys Cys  Thr Gly Gly
       2735                 2740                 2745
   Cys Thr  Gly Gly Thr Gly Gly  Ala Gly Ala Gly Ala  Gly Ala Ala
       2750                 2755                 2760
   Ala Thr  Thr Cys Ala Cys Cys  Ala Ala Gly Ala Gly  Ala Cys Cys
       2765                 2770                 2775
   Cys Ala  Thr Cys Cys Thr Gly  Ala Thr Thr Cys Ala  Gly Ala Gly
       2780                 2785                 2790
   Ala Cys  Thr Gly Cys Ala Thr  Thr Thr Gly Ala Ala  Ala Thr Cys
       2795                 2800                 2805
   Ala Ala  Ala Thr Cys Ala Thr  Cys Ala Gly Thr Thr  Ala Gly Ala
       2810                 2815                 2820
   Gly Cys  Cys Gly Ala Gly Gly  Gly Gly Ala Gly Ala  Gly Gly Ala
       2825                 2830                 2835
   Ala Gly  Thr Cys Thr Thr Cys  Thr Gly Ala Cys Cys  Thr Gly Cys
       2840                 2845                 2850
   Gly Gly  Ala Gly Ala Cys Gly  Thr Cys Gly Ala Ala  Gly Ala Gly
       2855                 2860                 2865
   Ala Ala  Thr Cys Cys Thr Gly  Gly Ala Cys Cys Cys  Gly Ala Cys
       2870                 2875                 2880
   Thr Ala  Cys Ala Ala Ala Gly  Ala Cys Cys Ala Thr  Gly Ala Cys
       2885                 2890                 2895
   Gly Gly  Thr Gly Ala Thr Thr  Ala Thr Ala Ala Ala  Gly Ala Thr
       2900                 2905                 2910
   Cys Ala  Thr Gly Ala Cys Ala  Thr Cys Gly Ala Thr  Thr Ala Cys
       2915                 2920                 2925
   Ala Ala  Gly Gly Ala Thr Gly  Ala Cys Gly Ala Thr  Gly Ala Cys
       2930                 2935                 2940
   Ala Ala  Gly Ala Thr Gly Gly  Cys Cys Cys Cys Cys  Ala Ala Gly
       2945                 2950                 2955
   Ala Ala  Gly Ala Ala Gly Ala  Gly Gly Ala Ala Gly  Gly Thr Gly
       2960                 2965                 2970
   Gly Gly  Cys Ala Thr Thr Cys  Ala Cys Cys Gly Cys  Gly Gly Gly
       2975                 2980                 2985
   Gly Thr  Ala Cys Cys Thr Ala  Thr Gly Gly Thr Gly  Gly Ala Cys
       2990                 2995                 3000
   Thr Thr  Gly Ala Gly Gly Ala  Cys Ala Cys Thr Cys  Gly Gly Thr
       3005                 3010                 3015
   Thr Ala  Thr Thr Cys Gly Cys  Ala Ala Cys Ala Gly  Cys Ala Ala
       3020                 3025                 3030
   Cys Ala  Gly Gly Ala Gly Ala  Ala Ala Ala Thr Cys  Ala Ala Gly
       3035                 3040                 3045
   Cys Cys  Thr Ala Ala Gly Gly  Thr Cys Ala Gly Gly  Ala Gly Cys
       3050                 3055                 3060
   Ala Cys  Cys Gly Thr Cys Gly  Cys Gly Cys Ala Ala  Cys Ala Cys
       3065                 3070                 3075
   Cys Ala  Cys Gly Ala Gly Gly  Cys Gly Cys Thr Thr  Gly Thr Gly
       3080                 3085                 3090
   Gly Gly  Gly Cys Ala Thr Gly  Gly Cys Thr Thr Cys  Ala Cys Thr
       3095                 3100                 3105
   Cys Ala  Thr Gly Cys Gly Cys  Ala Thr Ala Thr Thr  Gly Thr Cys
       3110                 3115                 3120
   Gly Cys  Gly Cys Thr Thr Thr  Cys Ala Cys Ala Gly  Cys Ala Cys
       3125                 3130                 3135
   Cys Cys  Thr Gly Cys Gly Gly  Cys Gly Cys Thr Thr  Gly Gly Gly
       3140                 3145                 3150
   Ala Cys  Gly Gly Thr Gly Gly  Cys Thr Gly Thr Cys  Ala Ala Ala
       3155                 3160                 3165
   Thr Ala  Cys Cys Ala Ala Gly  Ala Thr Ala Thr Gly  Ala Thr Thr
       3170                 3175                 3180
   Gly Cys  Gly Gly Cys Cys Cys  Thr Gly Cys Cys Cys  Gly Ala Ala
       3185                 3190                 3195
   Gly Cys  Cys Ala Cys Gly Cys  Ala Cys Gly Ala Gly  Gly Cys Ala
       3200                 3205                 3210
   Ala Thr  Thr Gly Thr Ala Gly  Gly Gly Gly Thr Cys  Gly Gly Thr
       3215                 3220                 3225
   Ala Ala  Ala Cys Ala Gly Thr  Gly Gly Thr Cys Gly  Gly Gly Ala
       3230                 3235                 3240
   Gly Cys  Gly Cys Gly Ala Gly  Cys Ala Cys Thr Thr  Gly Ala Gly
       3245                 3250                 3255
   Gly Cys  Gly Cys Thr Gly Cys  Thr Gly Ala Cys Thr  Gly Thr Gly
       3260                 3265                 3270
   Gly Cys  Gly Gly Gly Thr Gly  Ala Gly Cys Thr Thr  Ala Gly Gly
       3275                 3280                 3285
   Gly Gly  Gly Cys Cys Thr Cys  Cys Gly Cys Thr Cys  Cys Ala Gly
       3290                 3295                 3300
   Cys Thr  Cys Gly Ala Cys Ala  Cys Cys Gly Gly Gly  Cys Ala Gly
       3305                 3310                 3315
   Cys Thr  Gly Cys Thr Gly Ala  Ala Gly Ala Thr Cys  Gly Cys Gly
       3320                 3325                 3330
   Ala Ala  Gly Ala Gly Ala Gly  Gly Gly Gly Gly Ala  Gly Thr Ala
       3335                 3340                 3345
   Ala Cys  Ala Gly Cys Gly Gly  Thr Ala Gly Ala Gly  Gly Cys Ala
       3350                 3355                 3360
   Gly Thr  Gly Cys Ala Cys Gly  Cys Cys Thr Gly Gly  Cys Gly Cys
       3365                 3370                 3375
   Ala Ala  Thr Gly Cys Gly Cys  Thr Cys Ala Cys Cys  Gly Gly Gly
       3380                 3385                 3390
   Gly Cys  Cys Cys Cys Cys Thr  Thr Gly Ala Ala Cys  Cys Thr Gly
       3395                 3400                 3405
   Ala Cys  Cys Cys Cys Ala Gly  Ala Cys Cys Ala Gly  Gly Thr Ala
       3410                 3415                 3420
   Gly Thr  Cys Gly Cys Ala Ala  Thr Cys Gly Cys Gly  Thr Cys Ala
       3425                 3430                 3435
   Ala Ala  Cys Gly Gly Ala Gly  Gly Gly Gly Gly Ala  Ala Ala Gly
       3440                 3445                 3450
   Cys Ala  Ala Gly Cys Cys Cys  Thr Gly Gly Ala Ala  Ala Cys Cys
       3455                 3460                 3465
   Gly Thr  Gly Cys Ala Ala Ala  Gly Gly Thr Thr Gly  Thr Thr Gly
       3470                 3475                 3480
   Cys Cys  Gly Gly Thr Cys Cys  Thr Thr Thr Gly Thr  Cys Ala Ala
       3485                 3490                 3495
   Gly Ala  Cys Cys Ala Cys Gly  Gly Cys Cys Thr Thr  Ala Cys Ala
       3500                 3505                 3510
   Cys Cys  Gly Gly Ala Gly Cys  Ala Ala Gly Thr Cys  Gly Thr Gly
       3515                 3520                 3525
   Gly Cys  Cys Ala Thr Thr Gly  Cys Ala Ala Gly Cys  Ala Ala Thr
       3530                 3535                 3540
   Gly Gly  Gly Gly Gly Thr Gly  Gly Cys Ala Ala Ala  Cys Ala Gly
       3545                 3550                 3555
   Gly Cys  Thr Cys Thr Thr Gly  Ala Gly Ala Cys Gly  Gly Thr Thr
       3560                 3565                 3570
   Cys Ala Gly Ala Gly Ala Cys Thr Thr Cys Thr Cys Cys Cys Ala
   3575 3580 3585
   Gly Thr Thr Cys Thr Cys Thr Gly Thr Cys Ala Ala Gly Cys Cys
   3590 3595 3600
   Cys Ala Cys Gly Gly Gly Cys Thr Gly Ala Cys Thr Cys Cys Cys
   3605 3610 3615
   Gly Ala Thr Cys Ala Ala Gly Thr Thr Gly Thr Ala Gly Cys Gly
   3620 3625 3630
   Ala Thr Thr Gly Cys Gly Thr Cys Gly Ala Ala Cys Ala Thr Thr
   3635 3640 3645
   Gly Gly Ala Gly Gly Gly Ala Ala Ala Cys Ala Ala Gly Cys Ala
   3650 3655 3660
   Thr Thr Gly Gly Ala Gly Ala Cys Thr Gly Thr Cys Cys Ala Ala
   3665 3670 3675
   Cys Gly Gly Cys Thr Cys Cys Thr Thr Cys Cys Cys Gly Thr Gly
   3680 3685 3690
   Thr Thr Gly Thr Gly Thr Cys Ala Ala Gly Cys Cys Cys Ala Cys
   3695 3700 3705
   Gly Gly Thr Thr Thr Gly Ala Cys Gly Cys Cys Thr Gly Cys Ala
   3710 3715 3720
   Cys Ala Ala Gly Thr Gly Gly Thr Cys Gly Cys Cys Ala Thr Cys
   3725 3730 3735
   Gly Cys Cys Ala Gly Cys Cys Ala Thr Gly Ala Thr Gly Gly Cys
   3740 3745 3750
   Gly Gly Thr Ala Ala Gly Cys Ala Gly Gly Cys Gly Cys Thr Gly
   3755 3760 3765
   Gly Ala Ala Ala Cys Ala Gly Thr Ala Cys Ala Gly Cys Gly Cys
   3770 3775 3780
   Cys Thr Gly Cys Thr Gly Cys Cys Thr Gly Thr Ala Cys Thr Gly
   3785 3790 3795
   Thr Gly Cys Cys Ala Gly Gly Ala Thr Cys Ala Thr Gly Gly Ala
   3800 3805 3810
   Cys Thr Gly Ala Cys Cys Cys Cys Ala Gly Ala Cys Cys Ala Gly
   3815 3820 3825
   Gly Thr Ala Gly Thr Cys Gly Cys Ala Ala Thr Cys Gly Cys Gly
   3830 3835 3840
   Thr Cys Ala Ala Ala Cys Gly Gly Ala Gly Gly Gly Gly Gly Ala
   3845 3850 3855
   Ala Ala Gly Cys Ala Ala Gly Cys Cys Cys Thr Gly Gly Ala Ala
   3860 3865 3870
   Ala Cys Cys Gly Thr Gly Cys Ala Ala Ala Gly Gly Thr Thr Gly
   3875 3880 3885
   Thr Thr Gly Cys Cys Gly Gly Thr Cys Cys Thr Thr Thr Gly Thr
   3890 3895 3900
   Cys Ala Ala Gly Ala Cys Cys Ala Cys Gly Gly Cys Cys Thr Thr
   3905 3910 3915
   Ala Cys Ala Cys Cys Gly Gly Ala Gly Cys Ala Ala Gly Thr Cys
   3920 3925 3930
   Gly Thr Gly Gly Cys Cys Ala Thr Thr Gly Cys Ala Ala Ala Thr
   3935 3940 3945
   Ala Ala Thr Ala Ala Cys Gly Gly Thr Gly Gly Cys Ala Ala Ala
   3950 3955 3960
   Cys Ala Gly Gly Cys Thr Cys Thr Thr Gly Ala Gly Ala Cys Gly
   3965 3970 3975
   Gly Thr Thr Cys Ala Gly Ala Gly Ala Cys Thr Thr Cys Thr Cys
   3980 3985 3990
   Cys Cys Ala Gly Thr Thr Cys Thr Cys Thr Gly Thr Cys Ala Ala
   3995 4000 4005
   Gly Cys Cys Cys Ala Cys Gly Gly Gly Cys Thr Gly Ala Cys Thr
   4010 4015 4020
   Cys Cys Cys Gly Ala Thr Cys Ala Ala Gly Thr Thr Gly Thr Ala
   4025 4030 4035
   Gly Cys Gly Ala Thr Thr Gly Cys Gly Thr Cys Gly Cys Ala Thr
   4040 4045 4050
   Gly Ala Cys Gly Gly Ala Gly Gly Gly Ala Ala Ala Cys Ala Ala
   4055 4060 4065
   Gly Cys Ala Thr Thr Gly Gly Ala Gly Ala Cys Thr Gly Thr Cys
   4070 4075 4080
   Cys Ala  Ala Cys Gly Gly Cys  Thr Cys Cys Thr Thr  Cys Cys Cys
       4085                4090                4095
   Gly Thr  Gly Thr Thr Gly Thr  Gly Thr Cys Ala Ala  Gly Cys Cys
       4100                4105                4110
   Cys Ala  Cys Gly Gly Thr Thr  Thr Gly Ala Cys Gly  Cys Cys Thr
       4115                4120                4125
   Gly Cys  Ala Cys Ala Ala Gly  Thr Gly Gly Thr Cys  Gly Cys Cys
       4130                4135                4140
   Ala Thr  Cys Gly Cys Cys Thr  Cys Gly Ala Ala Thr  Gly Gly Cys
       4145                4150                4155
   Gly Gly  Cys Gly Gly Thr Ala  Ala Gly Cys Ala Gly  Gly Cys Gly
       4160                4165                4170
   Cys Thr  Gly Gly Ala Ala Ala  Cys Ala Gly Thr Ala  Cys Ala Gly
       4175                4180                4185
   Cys Gly  Cys Cys Thr Gly Cys  Thr Gly Cys Cys Thr  Gly Thr Ala
       4190                4195                4200
   Cys Thr  Gly Thr Gly Cys Cys  Ala Gly Gly Ala Thr  Cys Ala Thr
       4205                4210                4215
   Gly Gly  Ala Cys Thr Gly Ala  Cys Cys Cys Cys Ala  Gly Ala Cys
       4220                4225                4230
   Cys Ala  Gly Gly Thr Ala Gly  Thr Cys Gly Cys Ala  Ala Thr Cys
       4235                4240                4245
   Gly Cys  Gly Ala Ala Cys Ala  Ala Thr Ala Ala Thr  Gly Gly Gly
       4250                4255                4260
   Gly Gly  Ala Ala Ala Gly Cys  Ala Ala Gly Cys Cys  Cys Thr Gly
       4265                4270                4275
   Gly Ala  Ala Ala Cys Cys Gly  Thr Gly Cys Ala Ala  Ala Gly Gly
       4280                4285                4290
   Thr Thr  Gly Thr Thr Gly Cys  Cys Gly Gly Thr Cys  Cys Thr Thr
       4295                4300                4305
   Thr Gly  Thr Cys Ala Ala Gly  Ala Cys Cys Ala Cys  Gly Gly Cys
       4310                4315                4320
   Cys Thr  Thr Ala Cys Ala Cys  Cys Gly Gly Ala Gly  Cys Ala Ala
       4325                4330                4335
   Gly Thr  Cys Gly Thr Gly Gly  Cys Cys Ala Thr Thr  Gly Cys Ala
       4340                4345                4350
   Thr Cys  Cys Cys Ala Cys Gly  Ala Cys Gly Gly Thr  Gly Gly Cys
       4355                4360                4365
   Ala Ala  Ala Cys Ala Gly Gly  Cys Thr Cys Thr Thr  Gly Ala Gly
       4370                4375                4380
   Ala Cys  Gly Gly Thr Thr Cys  Ala Gly Ala Gly Ala  Cys Thr Thr
       4385                4390                4395
   Cys Thr  Cys Cys Cys Ala Gly  Thr Thr Cys Thr Cys  Thr Gly Thr
       4400                4405                4410
   Cys Ala  Ala Gly Cys Cys Cys  Ala Cys Gly Gly Gly  Cys Thr Gly
       4415                4420                4425
   Ala Cys  Thr Cys Cys Cys Gly  Ala Thr Cys Ala Ala  Gly Thr Thr
       4430                4435                4440
   Gly Thr  Ala Gly Cys Gly Ala  Thr Thr Gly Cys Gly  Thr Cys Cys
       4445                4450                4455
   Ala Ala  Cys Gly Gly Thr Gly  Gly Ala Gly Gly Gly  Ala Ala Ala
       4460                4465                4470
   Cys Ala  Ala Gly Cys Ala Thr  Thr Gly Gly Ala Gly  Ala Cys Thr
       4475                4480                4485
   Gly Thr  Cys Cys Ala Ala Cys  Gly Gly Cys Thr Cys  Cys Thr Thr
       4490                4495                4500
   Cys Cys  Cys Gly Thr Gly Thr  Thr Gly Thr Gly Thr  Cys Ala Ala
       4505                4510                4515
   Gly Cys  Cys Cys Ala Cys Gly  Gly Thr Thr Thr Gly  Ala Cys Gly
       4520                4525                4530
   Cys Cys  Thr Gly Cys Ala Cys  Ala Ala Gly Thr Gly  Gly Thr Cys
       4535                4540                4545
   Gly Cys  Cys Ala Thr Cys Gly  Cys Cys Ala Gly Cys  Cys Ala Thr
       4550                4555                4560
   Gly Ala  Thr Gly Gly Cys Gly  Gly Thr Ala Ala Gly  Cys Ala Gly
       4565                4570                4575
   Gly Cys  Gly Cys Thr Gly Gly  Ala Ala Ala Cys Ala  Gly Thr Ala
       4580                4585                4590
   Cys Ala Gly Cys Gly Cys Cys Thr Gly Cys Thr Gly Cys Cys Thr
   4595 4600 4605
   Gly Thr Ala Cys Thr Gly Thr Gly Cys Cys Ala Gly Gly Ala Thr
   4610 4615 4620
   Cys Ala Thr Gly Gly Ala Cys Thr Gly Ala Cys Cys Cys Cys Ala
   4625 4630 4635
   Gly Ala Cys Cys Ala Gly Gly Thr Ala Gly Thr Cys Gly Cys Ala
   4640 4645 4650
   Ala Thr Cys Gly Cys Gly Thr Cys Ala Cys Ala Thr Gly Ala Cys
   4655 4660 4665
   Gly Gly Gly Gly Gly Ala Ala Ala Gly Cys Ala Ala Gly Cys Cys
   4670 4675 4680
   Cys Thr Gly Gly Ala Ala Ala Cys Cys Gly Thr Gly Cys Ala Ala
   4685 4690 4695
   Ala Gly Gly Thr Thr Gly Thr Thr Gly Cys Cys Gly Gly Thr Cys
   4700 4705 4710
   Cys Thr Thr Thr Gly Thr Cys Ala Ala Gly Ala Cys Cys Ala Cys
   4715 4720 4725
   Gly Gly Cys Cys Thr Thr Ala Cys Ala Cys Cys Gly Gly Ala Gly
   4730 4735 4740
   Cys Ala Ala Gly Thr Cys Gly Thr Gly Gly Cys Cys Ala Thr Thr
   4745 4750 4755
   Gly Cys Ala Thr Cys Cys Cys Ala Cys Gly Ala Cys Gly Gly Thr
   4760 4765 4770
   Gly Gly Cys Ala Ala Ala Cys Ala Gly Gly Cys Thr Cys Thr Thr
   4775 4780 4785
   Gly Ala Gly Ala Cys Gly Gly Thr Thr Cys Ala Gly Ala Gly Ala
   4790 4795 4800
   Cys Thr Thr Cys Thr Cys Cys Cys Ala Gly Thr Thr Cys Thr Cys
   4805 4810 4815
   Thr Gly Thr Cys Ala Ala Gly Cys Cys Cys Ala Cys Gly Gly Gly
   4820 4825 4830
   Cys Thr Gly Ala Cys Thr Cys Cys Cys Gly Ala Thr Cys Ala Ala
   4835 4840 4845
   Gly Thr Thr Gly Thr Ala Gly Cys Gly Ala Thr Thr Gly Cys Gly
   4850 4855 4860
   Ala Ala Thr Ala Ala Cys Ala Ala Thr Gly Gly Ala Gly Gly Gly
   4865 4870 4875
   Ala Ala Ala Cys Ala Ala Gly Cys Ala Thr Thr Gly Gly Ala Gly
   4880 4885 4890
   Ala Cys Thr Gly Thr Cys Cys Ala Ala Cys Gly Gly Cys Thr Cys
   4895 4900 4905
   Cys Thr Thr Cys Cys Cys Gly Thr Gly Thr Thr Gly Thr Gly Thr
   4910 4915 4920
   Cys Ala Ala Gly Cys Cys Cys Ala Cys Gly Gly Thr Thr Thr Gly
   4925 4930 4935
   Ala Cys Gly Cys Cys Thr Gly Cys Ala Cys Ala Ala Gly Thr Gly
   4940 4945 4950
   Gly Thr Cys Gly Cys Cys Ala Thr Cys Gly Cys Cys Ala Gly Cys
   4955 4960 4965
   Cys Ala Thr Gly Ala Thr Gly Gly Cys Gly Gly Thr Ala Ala Gly
   4970 4975 4980
   Cys Ala Gly Gly Cys Gly Cys Thr Gly Gly Ala Ala Ala Cys Ala
   4985 4990 4995
   Gly Thr Ala Cys Ala Gly Cys Gly Cys Cys Thr Gly Cys Thr Gly
   5000 5005 5010
   Cys Cys Thr Gly Thr Ala Cys Thr Gly Thr Gly Cys Cys Ala Gly
   5015 5020 5025
   Gly Ala Thr Cys Ala Thr Gly Gly Ala Cys Thr Gly Ala Cys Ala
   5030 5035 5040
   Cys Cys Cys Gly Ala Ala Cys Ala Gly Gly Thr Gly Gly Thr Cys
   5045 5050 5055
   Gly Cys Cys Ala Thr Thr Gly Cys Thr Thr Cys Thr Ala Ala Thr
   5060 5065 5070
   Gly Gly Gly Gly Gly Ala Gly Gly Ala Cys Gly Gly Cys Cys Ala
   5075 5080 5085
   Gly Cys Cys Thr Thr Gly Gly Ala Gly Thr Cys Cys Ala Thr Cys
   5090 5095 5100
   Gly Thr Ala Gly Cys Cys Cys Ala Ala Thr Thr Gly Thr Cys Cys
   5105 5110 5115
   Ala Gly Gly Cys Cys Cys Gly Ala Thr Cys Cys Cys Gly Cys Gly
   5120 5125 5130
   Thr Thr Gly Gly Cys Thr Gly Cys Gly Thr Thr Ala Ala Cys Gly
   5135 5140 5145
   Ala Ala Thr Gly Ala Cys Cys Ala Thr Cys Thr Gly Gly Thr Gly
   5150 5155 5160
   Gly Cys Gly Thr Thr Gly Gly Cys Ala Thr Gly Thr Cys Thr Thr
   5165 5170 5175
   Gly Gly Thr Gly Gly Ala Cys Gly Ala Cys Cys Cys Gly Cys Gly
   5180 5185 5190
   Cys Thr Cys Gly Ala Thr Gly Cys Ala Gly Thr Cys Ala Ala Ala
   5195 5200 5205
   Ala Ala Gly Gly Gly Thr Cys Thr Gly Cys Cys Thr Cys Ala Thr
   5210 5215 5220
   Gly Cys Thr Cys Cys Cys Gly Cys Ala Thr Thr Gly Ala Thr Cys
   5225 5230 5235
   Ala Ala Ala Ala Gly Ala Ala Cys Cys Ala Ala Cys Cys Gly Gly
   5240 5245 5250
   Cys Gly Gly Ala Thr Thr Cys Cys Cys Gly Ala Gly Ala Gly Ala
   5255 5260 5265
   Ala Cys Thr Thr Cys Cys Cys Ala Thr Cys Gly Ala Gly Thr Cys
   5270 5275 5280
   Gly Cys Gly Gly Gly Ala Thr Cys Cys Gly Ala Thr Cys Cys Ala
   5285 5290 5295
   Ala Ala Ala Ala Ala Gly Ala Ala Gly Ala Gly Ala Ala Ala Gly
   5300 5305 5310
   Gly Thr Ala Gly Cys Cys Cys Cys Cys Cys Cys Gly Ala Cys Cys
   5315 5320 5325
   Gly Ala Thr Gly Thr Cys Ala Gly Cys Cys Thr Gly Gly Gly Gly
   5330 5335 5340
   Gly Ala Cys Gly Ala Gly Cys Thr Cys Cys Ala Cys Thr Thr Ala
   5345 5350 5355
   Gly Ala Cys Gly Gly Cys Gly Ala Gly Gly Ala Cys Gly Thr Gly
   5360 5365 5370
   Gly Cys Gly Ala Thr Gly Gly Cys Gly Cys Ala Thr Gly Cys Cys
   5375 5380 5385
   Gly Ala Cys Gly Cys Gly Cys Thr Ala Gly Ala Cys Gly Ala Thr
   5390 5395 5400
   Thr Thr Cys Gly Ala Thr Cys Thr Gly Gly Ala Cys Ala Thr Gly
   5405 5410 5415
   Thr Thr Gly Gly Gly Gly Gly Ala Cys Gly Gly Gly Gly Ala Thr
   5420 5425 5430
   Thr Cys Cys Cys Cys Gly Gly Gly Thr Cys Cys Gly Gly Gly Ala
   5435 5440 5445
   Thr Thr Thr Ala Cys Cys Cys Cys Cys Cys Ala Cys Gly Ala Cys
   5450 5455 5460
   Thr Cys Cys Gly Cys Cys Cys Cys Cys Thr Ala Cys Gly Gly Cys
   5465 5470 5475
   Gly Cys Thr Cys Thr Gly Gly Ala Thr Ala Thr Gly Gly Cys Cys
   5480 5485 5490
   Gly Ala Cys Thr Thr Cys Gly Ala Gly Thr Thr Thr Gly Ala Gly
   5495 5500 5505
   Cys Ala Gly Ala Thr Gly Thr Thr Thr Ala Cys Cys Gly Ala Thr
   5510 5515 5520
   Gly Cys Cys Cys Thr Thr Gly Gly Ala Ala Thr Thr Gly Ala Cys
   5525 5530 5535
   Gly Ala Gly Thr Ala Cys Gly Gly Thr Gly Gly Gly Ala Gly Ala
   5540 5545 5550
   Gly Cys Cys Gly Ala Gly Gly Gly Gly Ala Gly Ala Gly Gly Ala
   5555 5560 5565
   Ala Gly Thr Cys Thr Thr Cys Thr Gly Ala Cys Cys Thr Gly Cys
   5570 5575 5580
   Gly Gly Ala Gly Ala Cys Gly Thr Cys Gly Ala Ala Gly Ala Gly
   5585 5590 5595
   Ala Ala Thr Cys Cys Thr Gly Gly Ala Cys Cys Cys Ala Thr Gly
   5600 5605 5610
   Ala Gly  Cys Gly Ala Gly Cys  Thr Gly Ala Thr Thr  Ala Ala Gly
       5615                 5620                 5625
   Gly Ala  Gly Ala Ala Cys Ala  Thr Gly Cys Ala Cys  Ala Thr Gly
       5630                 5635                 5640
   Ala Ala  Gly Cys Thr Gly Thr  Ala Cys Ala Thr Gly  Gly Ala Gly
       5645                 5650                 5655
   Gly Gly  Cys Ala Cys Cys Gly  Thr Gly Gly Ala Cys  Ala Ala Cys
       5660                 5665                 5670
   Cys Ala  Thr Cys Ala Cys Thr  Thr Cys Ala Ala Gly  Thr Gly Cys
       5675                 5680                 5685
   Ala Cys  Ala Thr Cys Cys Gly  Ala Gly Gly Gly Cys  Gly Ala Ala
       5690                 5695                 5700
   Gly Gly  Cys Ala Ala Gly Cys  Cys Cys Thr Ala Cys  Gly Ala Gly
       5705                 5710                 5715
   Gly Gly  Cys Ala Cys Cys Cys  Ala Gly Ala Cys Cys  Ala Thr Gly
       5720                 5725                 5730
   Ala Gly  Ala Ala Thr Cys Ala  Ala Gly Gly Thr Gly  Gly Thr Cys
       5735                 5740                 5745
   Gly Ala  Gly Gly Gly Cys Gly  Gly Cys Cys Cys Thr  Cys Thr Cys
       5750                 5755                 5760
   Cys Cys  Cys Thr Thr Cys Gly  Cys Cys Thr Thr Cys  Gly Ala Cys
       5765                 5770                 5775
   Ala Thr  Cys Cys Thr Gly Gly  Cys Thr Ala Cys Thr  Ala Gly Cys
       5780                 5785                 5790
   Thr Thr  Cys Cys Thr Cys Thr  Ala Cys Gly Gly Cys  Ala Gly Cys
       5795                 5800                 5805
   Ala Ala  Gly Ala Cys Cys Thr  Thr Cys Ala Thr Cys  Ala Ala Cys
       5810                 5815                 5820
   Cys Ala  Cys Ala Cys Cys Cys  Ala Gly Gly Gly Cys  Ala Thr Cys
       5825                 5830                 5835
   Cys Cys  Cys Gly Ala Cys Thr  Thr Cys Thr Thr Cys  Ala Ala Gly
       5840                 5845                 5850
   Cys Ala  Gly Thr Cys Cys Thr  Thr Cys Cys Cys Thr  Gly Ala Gly
       5855                 5860                 5865
   Gly Gly  Cys Thr Thr Cys Ala  Cys Ala Thr Gly Gly  Gly Ala Gly
       5870                 5875                 5880
   Ala Gly  Ala Gly Thr Cys Ala  Cys Cys Ala Cys Ala  Thr Ala Cys
       5885                 5890                 5895
   Gly Ala  Ala Gly Ala Cys Gly  Gly Gly Gly Gly Cys  Gly Thr Gly
       5900                 5905                 5910
   Cys Thr  Gly Ala Cys Cys Gly  Cys Thr Ala Cys Cys  Cys Ala Gly
       5915                 5920                 5925
   Gly Ala  Cys Ala Cys Cys Ala  Gly Cys Cys Thr Cys  Cys Ala Gly
       5930                 5935                 5940
   Gly Ala  Cys Gly Gly Cys Thr  Gly Cys Cys Thr Cys  Ala Thr Cys
       5945                 5950                 5955
   Thr Ala  Cys Ala Ala Cys Gly  Thr Cys Ala Ala Gly  Ala Thr Cys
       5960                 5965                 5970
   Ala Gly  Ala Gly Gly Gly Gly  Thr Gly Ala Ala Cys  Thr Thr Cys
       5975                 5980                 5985
   Ala Cys  Ala Thr Cys Cys Ala  Ala Cys Gly Gly Cys  Cys Cys Thr
       5990                 5995                 6000
   Gly Thr  Gly Ala Thr Gly Cys  Ala Gly Ala Ala Gly  Ala Ala Ala
       6005                 6010                 6015
   Ala Cys  Ala Cys Thr Cys Gly  Gly Cys Thr Gly Gly  Gly Ala Gly
       6020                 6025                 6030
   Gly Cys  Cys Thr Thr Cys Ala  Cys Cys Gly Ala Gly  Ala Cys Gly
       6035                 6040                 6045
   Cys Thr  Gly Thr Ala Cys Cys  Cys Cys Gly Cys Thr  Gly Ala Cys
       6050                 6055                 6060
   Gly Gly  Cys Gly Gly Cys Cys  Thr Gly Gly Ala Ala  Gly Gly Cys
       6065                 6070                 6075
   Ala Gly  Ala Ala Ala Cys Gly  Ala Cys Ala Thr Gly  Gly Cys Cys
       6080                 6085                 6090
   Cys Thr  Gly Ala Ala Gly Cys  Thr Cys Gly Thr Gly  Gly Gly Cys
       6095                 6100                 6105
   Gly Gly  Gly Ala Gly Cys Cys  Ala Thr Cys Thr Gly  Ala Thr Cys
       6110                 6115                 6120
   Gly Cys  Ala Ala Ala Cys Ala  Thr Cys Ala Ala Gly  Ala Cys Cys
       6125                6130                6135
   Ala Cys  Ala Thr Ala Thr Ala  Gly Ala Thr Cys Cys  Ala Ala Gly
       6140                6145                6150
   Ala Ala  Ala Cys Cys Cys Gly  Cys Thr Ala Ala Gly  Ala Ala Cys
       6155                6160                6165
   Cys Thr  Cys Ala Ala Gly Ala  Thr Gly Cys Cys Thr  Gly Gly Cys
       6170                6175                6180
   Gly Thr  Cys Thr Ala Cys Thr  Ala Thr Gly Thr Gly  Gly Ala Cys
       6185                6190                6195
   Thr Ala  Cys Ala Gly Ala Cys  Thr Gly Gly Ala Ala  Ala Gly Ala
       6200                6205                6210
   Ala Thr  Cys Ala Ala Gly Gly  Ala Gly Gly Cys Cys  Ala Ala Cys
       6215                6220                6225
   Ala Ala  Cys Gly Ala Gly Ala  Cys Cys Thr Ala Cys  Gly Thr Cys
       6230                6235                6240
   Gly Ala  Gly Cys Ala Gly Cys  Ala Cys Gly Ala Gly  Gly Thr Gly
       6245                6250                6255
   Gly Cys  Ala Gly Thr Gly Gly  Cys Cys Ala Gly Ala  Thr Ala Cys
       6260                6265                6270
   Thr Gly  Cys Gly Ala Cys Cys  Thr Cys Cys Cys Thr  Ala Gly Cys
       6275                6280                6285
   Ala Ala  Ala Cys Thr Gly Gly  Gly Gly Cys Ala Cys  Ala Ala Gly
       6290                6295                6300
   Cys Thr  Cys Ala Ala Thr
       6305
<210> 18
   <211> 6330
   <212> PRT
   <213> Unknown
<220>
   <223> Synthetic genetic construct
<400> 18
<210> 19
   <211> 17
   <212> DNA
   <213> Unknown
<220>
   <223> DNA-binding domain
<400> 19
   taaagagcag aaactca 17
<210> 20
   <211> 18
   <212> DNA
   <213> Unknown
<220>
   <223> DNA-binding domain
<400> 20
   tttactgctg ctcccgct 18
<210> 21
   <211> 1762
   <212> DNA
   <213> Unknown
<220>
   <223> pCMV-tTR:KRAB
<400> 21
   gagcttggcc cattgcatac gttgtatcca tatcataata tgtacattta tattggctca     60
   tgtccaacat taccgccatg ttgacattga ttattgacta gttattaata gtaatcaatt    120
   acggggtcat tagttcatag cccatatatg gagttccgcg ttacataact tacggtaaat    180
   ggcccgcctg gctgaccgcc caacgacccc cgcccattga cgtcaataat gacgtatgtt    240
   cccatagtaa cgccaatagg gactttccat tgacgtcaat gggtggagta tttacgctaa    300
   actgcccact tggcagtaca tcaagtgtat catatgccaa gtacgccccc tattgacgtc    360
   aatgacggta aatggcccgc ctggcattat gcccagtaca tgaccttatg ggactttcct    420
   acttggcagt acatctacgt attagtcatc gctattacca tggtgatgcg gttttggcag    480
   tacatcaatg ggcgtggata gcggtttgac tcacggggat ttccaagtct ccaccccatt    540
   gacgtcaatg ggagtttgtt ttggcaccaa aatcaacggg actttccaaa atgtcgtaac    600
   aactccgccc cattgacgca aatgggcggt aggcgtgtac ggtgggaggt ctatataagc    660
   agagctcgtt tagtgaaccg tcagatcgcc tggagacgcc atccacgctg ttttgacctc    720
   catagaagac accgggaccg atccagcctc cgcggaattc gccaccatgg ctagattaga    780
   taaaagtaaa gtgattaaca gcgcattaga gctgcttaat gaggtcggaa tcgaaggttt    840
   aacaacccgt aaactcgccc agaagctagg tgtagagcag cctacattgt attggcatgt    900
   aaaaaataag cgggctttgc tcgacgcctt agccattgag atgttagata ggcaccatac    960
   tcacttttgc cctttagaag gggaaagctg gcaagatttt ttacgtaata acgctaaaag   1020
   ttttagatgt gctttactaa gtcatcgcga tggagcaaaa gtacatttag gtacacggcc   1080
   tacagaaaaa cagtatgaaa ctctcgaaaa tcaattagcc tttttatgcc aacaaggttt   1140
   ttcactagag aatgcattat atgcactcag cgctgtgggg cattttactt taggttgcgt   1200
   attggaagat caagagcatc aagtcgctaa agaagaaagg gaaacaccta ctactgatag   1260
   tatgccgcca ttattacgac aagctatcga attatttgat caccaaggtg cagagccagc   1320
   cttcttattc ggccttgaat tgatcatatg cggattagaa aaacaactta aatgtgaaag   1380
   tgggtcccgc gcgccagatc caaaaaagaa gagaaaggta gatccaaaaa agaagagaaa   1440
   ggtagatcca aaaaagaaga gaaaggtaat ggatgctaag tcactaactg cctggtcccg   1500
   gacactggtg accttcaagg atgtatttgt ggacttcacc agggaggagt ggaagctgct   1560
   ggacactgct cagcagatcg tgtacagaaa tgtgatgctg gagaactata agaacctggt   1620
   ttccttgggt tatcagctta ctaagccaga tgtgatcctc cggttggaga agggagaaga   1680
   gccctggctg gtggagagag aaattcacca agagacccat cctgattcag agactgcatt   1740
   tgaaatcaaa tcatcagttt aa                                            1762
<210> 22
   <211> 8363
   <212> DNA
   <213> Unknown
<220>
   <223> plasmid ekdison induktor
<400> 22
   cacctgacgc gccctgtagc ggcgcattaa gcgcggcggg tgtggtggtt acgcgcagcg      60
   tgaccgctac acttgccagc gccctagcgc ccgctccttt cgctttcttc ccttcctttc     120
   tcgccacgtt cgccggcttt ccccgtcaag ctctaaatcg ggggctccct ttagggttcc     180
   gatttagtgc tttacggcac ctcgacccca aaaaacttga ttagggtgat ggttcacgta     240
   gtgggccatc gccctgatag acggtttttc gccctttgac gttggagtcc acgttcttta     300
   atagtggact cttgttccaa actggaacaa cactcaaccc tatctcggtc tattcttttg     360
   atttataagg gattttgccg atttcggcct attggttaaa aaatgagctg atttaacaaa     420
   aatttaacgc gaattttaac aaaatattaa cgcttacaat ttacgcgtcc agacatgata     480
   agatacattg atgagtttgg acaaaccaca actagaatgc agtgaaaaaa atgctttatt     540
   tgtgaaattt gtgatgctat tgctttattt gtaaccatta taagctgcaa taaacaagtt     600
   aacaacaaca attgcattca ttttatgttt caggttcagg gggaggtgtg ggaggttttt     660
   taaagcaagt aaaacctcta caaatgtggt atggctgatt atgatcatga acagactgtg     720
   aggactgagg ggcctgaaat gagccttggg actgtgatct aaaatacaca aacaattaga     780
   atcagtagtt taacacatta tacacttaaa aattttatat ttaccttaga gctttaaatc 840
   tctgtaggta gtttgtccaa ttatgtcaca ccacagaagt aaggttcctt cacaaagatc 900
   gcggccgcct aagtcatttg gtgcggcgcc tccagcatct ccataaggaa ggtgtcaatg 960
   ggtgtgtccc cgatgagctt gaagaagaag agatgttcca ggcatttgag cccgatggag 1020
   cgcagagccg gcaggcggag caagagctta gcgaaccttc ccggctgctc tgggtacttg 1080
   tgcttgcagt aggcctccaa ggacgcatag accttctccc tcagcgcctc cacctcggcc 1140
   gggttcgaga gccccttgga gtcagggtta aagaggacga tggcgcgcag gcagcccagc 1200
   tccgtcttgt ccatctgcat gtcccgcatc ttggacacaa gctccgtcag caccctgtca 1260
   aagatggcgc ccacccctgc gctgtgggcg ctgttccggt ggacgtgcag cccggtggcc 1320
   aggaggatcc cgtccttcac ggcgatggag cggtgggaga aggaggcgat gagcagctca 1380
   ttccagcctg cccgcagcag gatgacctgg tcgtccaggg gcagctctga gaagtgtggg 1440
   atccgcttgg cccactccac cagggtgaaa agctgtttgt cggctgcttg gcaaatgttg 1500
   gtgacagggt cgttcggcga gctggggttc agccccatgt ttgcctccac gtaggtctcg 1560
   gtcttgggct ccacggccag ctcagcctcc aggatcctct ccaccggcat gtcctcgttg 1620
   gcgctgctgg tcgactccac ctcattctcg ttccggtcct tgccacgctg ccgctcctcc 1680
   tgcacggctt cccgcttcat gcccatggcc aggcacttct ggtagcggca gtactggcac 1740
   cggttccgct gccgcttgtc aatcaggcag tccttgttgt cgcggcaggt gtaggtcagg 1800
   tccttgcgca ccgtccgctt gaagaagccc ttgcacccct cgcagctgta cactccatag 1860
   tgcttgcctg aggagcggtc cccgcagatg gcgcagatgt gcttggtgaa ggaagccatg 1920
   tttcctgagg ggtgggcggg gaccttgagg acgccattga ggcccagggg gggcttgatg 1980
   tcctcgctgc tgctgacggg gttcataggt gagctgagct gggggctgcc agtgctgaag 2040
   cccagggtgg gtgtggtggg caccgacatg gagtgggggc ccatggggga gctgatgacc 2100
   gagaaaggcg ggcccatgcc gttgatgggg gagctcaggg tgctgatggg agaatgcagc 2160
   tgtcccgggg agccgatgcc aggccccagg gacgggtgca gcgagggggc agccatggag 2220
   cctcgccccg tcggggaggt gagggaggag ttcacctggg tggagaaatc gagcggcagg 2280
   aaatgtttgg tgtccaagtc ttcttcagaa ataagttttt gttccatatt atcgatcgtg 2340
   tttttcaaag gaaaaccacg tccccgtggt tcggggggcc tagacgtttt ttaacctcga 2400
   ctaaacacat gtaaagcatg tgcaccgagg ccccagatca gatcccatac aatggggtac 2460
   cttctgggca tccttcagcc ccttgttgaa tacgcttgag gagagccatt tgactctttc 2520
   cacaactatc caactcacaa cgtggcactg gggttgtgcc gcctttgcag gtgtatctta 2580
   tacacgtggc ttttggccgc agaggcacct gtcgccaggt ggggggttcc gctgcctgca 2640
   aagggtcgct acagacgttg tttgtcttca agaagcttcc agaggaactg cttccttcac 2700
   gacattcaac agaccttgca ttcctttggc gagaggggaa agacccctag gaatgctcgt 2760
   caagaagaca gggccaggtt tccgggccct cacattgcca aaagacggca atatggtgga 2820
   aaataacata tagacaaacg cacaccggcc ttattccaag cggcttcggc cagtaacgtt 2880
   aggggggggg gagggagagg ggggtaaccc tatgcagtcg tcgagtgctc cgacttaacc 2940
   gccactcccc cgataagctg ctcttggtgc gaatgcaggg ctacacccat caaggccatc 3000
   gccgtctggg cgttcgcata catgctgacg ttgccgccca ccccaacacc gactccaact 3060
   ccgttgccca tcggtaccgc tgatgtggtg gagctagcgg taacggcagc cgtgatactg 3120
   ctggtggttg ccggcgtgat gggtcctatg gccgcacttc cgcccatgta ttcgctgctc 3180
   gtactgaccg cggacaagga accaggtgcg gttacggagg cgggcacggg agcggagacg 3240
   ggaaggagct gtggctgtgg ttgaatctgt ggctggagtt gcgtctgaag ctgtgggtgg 3300
   agctggggtt gcagttgacc ttgcagctga ggtggtagct gaggttgtag ctgcggctgt 3360
   gtctggtgct gggaatcgtt ctgggtcagg gaggagggtt ggggctgggg ctgaggctga 3420
   ggctgatgct gggccgcggc tgccgccgcc gaagtggagg cagagtcgca atcaatgccg 3480
   gcggtaatgg cgcccccaac cgatgcccgc atacgctcag cccgctcgag acgctcgttc 3540
   tcctcctggg taatctgaag gtgcgactgg accgatggcg ggatggcatg aacgtcccag 3600
   atctcctcga ggaacttggg cagtttgcgg tttttgagct ttagtgagaa acacatctcg 3660
   gcgttctggt tgcccagcgt acgcagctcg gtgaggatcg agagcagctt tgcgtagaag 3720
   acgaggctca ttgagtcgcc gcagtggcgg ttgagtatat aaatgcgtag cgtgtcgatg 3780
   tagtagctct ggatcgcttc gactagctgg gccttctcca ggcccggccg gtccgagaag 3840
   atcacaatgg cagtgagaag cgcgtattcg acgttgtcca ccttcatcga gaacatttgg 3900
   cggcagaaat gcagcaggtc ttcaatgtta tcagccattc cggccatttt gtaagaatcc 3960
   cgcgtatatg atctattatt cgcgaagaat attgagtccg agctgtggtc atagcgtcgt 4020
   gccatacgca gcatcatcac ctccgacgag caggccttta gtaacgtgat ctggtcctcc 4080
   tggggtatct ttgtaaacgc tggtagacct ttagcaaact caacaatcaa ctggaccgtg 4140
   agtatggtta tctcggttat atgccgaaag ctgacgtccg tttggctctc gttctcatcg 4200
   ggttgactca ttatacgcct gagatcctct tcagatggct gctcatagcc atcctggtac 4260
   caaattaact tgtatataac ggccaactga ttgtacgtta aggaaggtat attgcgcgct 4320
   tgacacttgg ccaatatttc atcaggtagt agcggaatag tggcatgctg gggcggctcg 4380
   catgtcataa ggtcaagaat ctccttctta acaaagtctt ggccgccacc agaggccaag 4440
   ctgccattgc cgccatgctg agagctcggc gaagtggtca ttttgtcctt ctccttctgg 4500
   gccttctttt cgcgccgctt catcgcacat tggttctccg ggacgacgca ttccggccgc 4560
   atacccacgg ccaggcactt tttcaggcgg cactcctgac actttcgcct catgtacatg 4620
   tccatttcgc aggcgcgccc gaacttgcag cagtagacgg cgctcttcgt aacgctgcgt 4680
   cgaaagaaca ccttgcagga tccacaggtg agggcgttgt agtggtagcc ggaggccctg 4740
   tcctttcgtt cgccgagtat ccgttcaagc tgctcgaagg cgagagatca tcgcgacctg 4800
   aagatataga atttgatatt cttctagatg tacctagaag cttcccaccg tactcgtcaa 4860
   ttccaagggc atcggtaaac atctgctcaa actcgaagtc ggccatatcc agagcgccgt     4920
   agggggcgga gtcgtggggg gtaaatcccg gacctgggga atccccgtcc cccaacatgt     4980
   ccagatcgaa atcgtctagc gcgtcggcat gcgccatcgc cacgtcctcg ccgtctaagt     5040
   ggagttcgtc ccccaggctg acatcggtcg ggggggccaa gtcttcttca gaaataagtt     5100
   tttgttccat ggtggcggcc ggccactagc ggatctgacg gttcactaaa ccagctctgc     5160
   ttatatagac ctcccaccgt acacgcctac cgcccatttg cgtcaatggg gcggagttgt     5220
   tacgacattt tggaaagtcc cgttgatttt ggtgccaaaa caaactccca ttgacgtcaa     5280
   tggggtggag acttggaaat ccccgtgagt caaaccgcta tccacgccca ttgatgtact     5340
   gccaaaaccg catcaccatg gtaatagcga tgactaatac gtagatgtac tgccaagtag     5400
   gaaagtccca taaggtcatg tactgggcat aatgccaggc gggccattta ccgtcattga     5460
   cgtcaatagg gggcgtactt ggcatatgat acacttgatg tactgccaag tgggcagttt     5520
   accgtaaata ctccacccat tgacgtcaat ggaaagtccc tattggcgtt actatgggaa     5580
   catacgtcat tattgacgtc aatgggcggg ggtcgttggg cggtcagcca ggcgggccat     5640
   ttaccgtaag ttatgtaacg cggaactcca tatatgggct atgaactaat gaccccgtaa     5700
   ttgattacta ttaataacta atgcaacggc gctgcagcca ctgcatggcg gtaatacggt     5760
   tatccacaga atcaggggat aacgcaggaa agaacatgtg agcaaaaggc cagcaaaagg     5820
   ccaggaaccg taaaaaggcc gcgttgctgg cgtttttcca taggctccgc ccccctgacg     5880
   agcatcacaa aaatcgacgc tcaagtcaga ggtggcgaaa cccgacagga ctataaagat     5940
   accaggcgtt tccccctgga agctccctcg tgcgctctcc tgttccgacc ctgccgctta     6000
   ccggatacct gtccgccttt ctcccttcgg gaagcgtggc gctttctcat agctcacgct     6060
   gtaggtatct cagttcggtg taggtcgttc gctccaagct gggctgtgtg cacgaacccc     6120
   ccgttcagcc cgaccgctgc gccttatccg gtaactatcg tcttgagtcc aacccggtaa     6180
   gacacgactt atcgccactg gcagcagcca ctggtaacag gattagcaga gcgaggtatg     6240
   taggcggtgc tacagagttc ttgaagtggt ggcctaacta cggctacact agaaggacag     6300
   tatttggtat ctgcgctctg ctgaagccag ttaccttcgg aaaaagagtt ggtagctctt     6360
   gatccggcaa acaaaccacc gctggtagcg gtggtttttt tgtttgcaag cagcagatta     6420
   cgcgcagaaa aaaaggatct caagaagatc ctttgatctt ttctacgggg tctgacgctc     6480
   agtggaacga aaactcacgt taagggattt tggtcatgag attatcaaaa aggatcttca     6540
   cctagatcct tttaaattaa aaatgaagtt ttaaatcaat ctaaagtata tatgagtaac     6600
   ctgaggctat ggcagggcct gccgccccga cgttggctgc gagccctggg ccttcacccg     6660
   aacttggggg gtggggtggg gaaaaggaag aaacgcgggc gtattggccc caatggggtc     6720
   tcggtggggt atcgacagag tgccagccct gggaccgaac cccgcgttta tgaacaaacg     6780
   acccaacacc gtgcgtttta ttctgtcttt ttattgccgt catagcgcgg gttccttccg     6840
   gtattgtctc cttccgtgtt tcagttagcc tccccctagg gtgggcgaag aactccagca     6900
   tgagatcccc gcgctggagg atcatccagc cggcgtcccg gaaaacgatt ccgaagccca     6960
   acctttcata gaaggcggcg gtggaatcga aatctcgtga tggcaggttg ggcgtcgctt     7020
   ggtcggtcat ttcgaacccc agagtcccgc tcagaagaac tcgtcaagaa ggcgatagaa     7080
   ggcgatgcgc tgcgaatcgg gagcggcgat accgtaaagc acgaggaagc ggtcagccca     7140
   ttcgccgcca agctcttcag caatatcacg ggtagccaac gctatgtcct gatagcggtc     7200
   cgccacaccc agccggccac agtcgatgaa tccagaaaag cggccatttt ccaccatgat     7260
   attcggcaag caggcatcgc catgggtcac gacgagatcc tcgccgtcgg gcatgctcgc     7320
   cttgagcctg gcgaacagtt cggctggcgc gagcccctga tgctcttcgt ccagatcatc     7380
   ctgatcgaca agaccggctt ccatccgagt acgtgctcgc tcgatgcgat gtttcgcttg     7440
   gtggtcgaat gggcaggtag ccggatcaag cgtatgcagc cgccgcattg catcagccat     7500
   gatggatact ttctcggcag gagcaaggtg agatgacagg agatcctgcc ccggcacttc     7560
   gcccaatagc agccagtccc ttcccgcttc agtgacaacg tcgagcacag ctgcgcaagg     7620
   aacgcccgtc gtggccagcc acgatagccg cgctgcctcg tcttgcagtt cattcagggc     7680
   accggacagg tcggtcttga caaaaagaac cgggcgcccc tgcgctgaca gccggaacac     7740
   ggcggcatca gagcagccga ttgtctgttg tgcccagtca tagccgaata gcctctccac     7800
   ccaagcggcc ggagaacctg cgtgcaatcc atcttgttca atcatgcgaa acgatcctca     7860
   tcctgtctct tgatcgatct ttgcaaaagc ctaggcctcc aaaaaagcct cctcactact     7920
   tctggaatag ctcagaggcc gaggcggcct cggcctctgc ataaataaaa aaaattagtc     7980
   agccatgggg cggagaatgg gcggaactgg gcggagttag gggcgggatg ggcggagtta     8040
   ggggcgggac tatggttgct gactaattga gatgcatgct ttgcatactt ctgcctgctg     8100
   gggagcctgg ggactttcca cacctggttg ctgactaatt gagatgcatg ctttgcatac     8160
   ttctgcctgc tggggagcct ggggactttc cacaccctaa ctgacacaca ttccacagct     8220
   ggttctttcc gcctcaggac tcttcctttt tcaatattat tgaagcattt atcagggtta     8280
   ttgtctcatg agcggataca tatttgaatg tatttagaaa aataaacaaa taggggttcc     8340
   gcgcacattt ccccgaaaag tgc                                               8363